# EUROPEAN PATENT APPLICATION

(11) **EP 4 837 908 A1**
(43) Date of publication of application: **11.11.2026**
(21) Application number: 25747762.0
(22) Date of filing: 23.01.2025
(51) Int. Cl.: C07D 405/12, C07D 405/14, A61K 31/443, A61P 29/00

(54) **NOVEL SODIUM CHANNEL MODULATOR COMPOUND AND USE THEREOF**

(30) Priority: 31.01.2024 CN 202410137514; 04.11.2024 CN 202411563504
(71) Applicant: Shanghai Huilun Pharmaceutical Co., Ltd., Shanghai 200241 (CN)
(72) Inventor: DUAN, Shudong, Shanghai 200241 (CN); DU, Shuqi, Shanghai 200241 (CN); QIN, Jihong, Shanghai 200241 (CN)
(74) Representative: Maiwald GmbH
(86) International application number: PCT/CN2025/074442
(87) International publication number: WO 2025/162194

(57) **Abstract**

Provided are a novel sodium channel modulator compound and use thereof. The sodium channel modulator has a completely new structure as shown in formula I, and exhibits excellent in vitro inhibitory effects against Naᵥ1.8. The compound or a pharmaceutically acceptable salt thereof can be used for treating sodium channel-related diseases, including diseases such as pain, multiple sclerosis, Charcot-Marie-Tooth disease, incontinence, pathological cough, or arrhythmia.

## Description

This application claims priorities to Chinese Patent Application No. 2024101375145 filed on January 31, 2024 and Chinese Patent Application No. 2024115635044 filed on November 4, 2024, both of which are incorporated herein by reference in their entireties.

### TECHNICAL FIELD

The present invention relates to the field of medicine, and specifically, to a novel sodium channel modulator compound, a preparation method thereof, and therapeutic use thereof in treatment of disease.

### BACKGROUND

Pain is a complex sensation, usually uncomfortable, caused by physical injury, illness, or undesirable external stimuli. For clinical research purposes, the International Association for the Study of Pain (IASP) defines pain as "an unpleasant sensory and emotional experience associated with actual or potential tissue damage". Pain serves as a warning signal that alerts the body to potential danger, which is indispensable for the protection of normal life activities. Pain is also a common clinical symptom. After the disappearance of an external stimulus that causes pain, the intense or persistent pain can cause physiological dysfunctions, which seriously affect the quality of life of living organisms. Some data shows that about one in five people worldwide suffer from moderate or severe chronic pain.

Pain originates in nociceptors of the peripheral nervous system. The nociceptors are free nerve endings that are widely distributed throughout the body in the skin, muscles, joints, and visceral tissues, and that can convert perceived thermal, mechanical, or chemical stimuli into nerve impulses (action potentials) and transmit the nerve impulses via afferent nerve fibers to their cell bodies located in dorsal root ganglia (DRG), and ultimately to higher nervous centers to elicit pain perception. The generation and conduction of action potentials in neurons depend on voltage-gated sodium channels (VGSCs) on cell membranes. When the cell membrane is depolarized, the sodium channels are activated and open, allowing sodium ions to flow inward, which further depolarizes the cell membrane, leading to the generation of action potentials. Therefore, the inhibition of abnormal activity of sodium channels contributes to the treatment and relief of pain.

Voltage-gated sodium channels are widely found on cell membranes of excitable cells such as neurons and skeletal muscle cells, and are a class of transmembrane glycoprotein complexes composed of one α subunit and several β subunits. The α subunit is a functional carrier of the sodium channels and consists of 1700-2000 amino acids. The β subunit primarily plays an auxiliary role in the modification of kinetics and voltage gating dependence of the ion channels. Sodium channels may be categorized based on different α subunits. Currently, nine sodium channel isoforms, Naᵥ1 (Naᵥ1.1 to Naᵥ1.9), have been identified in mammals. Different isoforms exhibit different tissue distributions, and different electrophysiological and pharmacological characteristics. Based on whether they can be effectively inhibited by nanomolar tetrodotoxin (TTX), sodium channels are categorized into TTX-sensitive (TTX-S) sodium channels and TTX-resistant (TTX-R) sodium channels. Naᵥ1.1, Naᵥ1.2, Naᵥ1.3, and Naᵥ1.7 are TTX-S, with encoding genes located on human chromosome 2q23-24, and abundantly expressed in neurons. Naᵥ1.5, Naᵥ1.8, and Naᵥ1.9 are TTX-R, with encoding genes located on human chromosome 3p21-24. Naᵥ1.5 is found primarily in cardiomyocytes. Naᵥ1.8 and Naᵥ1.9 are found in the peripheral nervous system (PNS).

Naᵥ1.8 is an important ion channel involved in chronic pain, atrial fibrillation, and Budd-Chiari syndrome, and is a highly selective target for the treatment of pain. It has been proved that Naᵥ1.8 as a carrier of sodium currents maintains action potential firing of small dorsal root ganglion neurons, and is also involved in spontaneous electrical signaling of damaged neurons, for example, driving the production of neuropathic pain. Currently reported small-molecule Naᵥ1.8 inhibitors include PF-01247324, A-803467, PF-06305591, VX-150, HRS-4800, JMKX-000623, HBW-004, VX-548, and the like. PF-01247324, A-803467, and PF-06305591 have the disadvantages of poor selectivity, poor pharmacokinetic data, and low bioavailability.

### SUMMARY

According to a first aspect of the present invention, provided is a compound shown in formula I 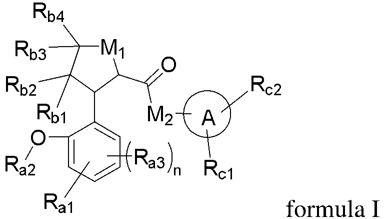 or a pharmaceutically acceptable salt thereof, where the compound further includes a stereoisomer and a racemate thereof when a chiral center is present.

In some implementations of the present invention, the compound is further shown in formula I-A or I-B:

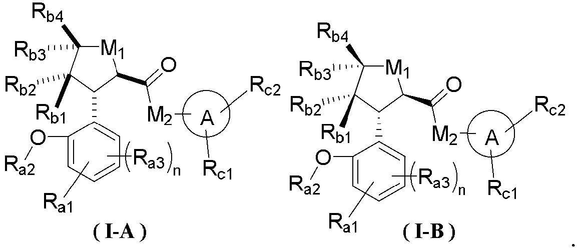

In the compound, Rₐ₁ and Rₐ₃ are each independently selected from hydrogen, halogen, hydroxyl, C₁-C₅ alkyl, C₁-C₅ alkoxy, C₃-C₆ cycloalkyl, C₃-C₆ cycloalkoxy, or -N(R_{c3}R_{c4})ₘ, and the alkyl, alkoxy, cycloalkyl, or cycloalkoxy is optionally substituted with one or more halogen, hydroxyl, cyano, C₁-C₅ alkyl, C₁-C₅ alkoxy, or amino; and Rₐ₂ is selected from C₁-C₆ alkyl substituted with one or more deuterium or fluorine.

In some implementations of the present invention, any adjacent two of Rₐ₁ and Rₐ₃ are linked together to form a 5- to 7-membered ring, the 5- to 7-membered ring may be a saturated or unsaturated carbocyclic or heterocyclic ring; the heterocyclic ring optionally contains one or more N, O, or S(=O)ₘ heteroatoms; and the 5- to 7-membered ring may be optionally substituted with one or more halogen, hydroxyl, cyano, C₁-C₅ alkyl, C₁-C₅ alkoxy, or amino.

In some specific implementations of the present invention, Rₐ₁ and Rₐ₃ are each independently selected from fluorine, chlorine, methyl, ethyl, methoxy, ethoxy, propoxy, trifluoromethoxy, difluoromethoxy, or amino.

In some specific implementations, Rₐ₂ is optionally selected from methyl substituted with one, two, or three deuterium or fluorine.

In some specific implementations, any adjacent two of Rₐ₁ and Rₐ₃ are linked together to form a 5- or 6-membered carbocyclic or heterocyclic ring.

In some specific implementations, the 5- or 6-membered heterocyclic ring contains one or two N, O, or S(=O)ₘ heteroatoms.

In some specific implementations, the 5- or 6-membered carbocyclic or heterocyclic ring is optionally substituted with one or more halogen, hydroxyl, cyano, C₁-C₅ alkyl, C₁-C₅ alkoxy, or amino.

In the compound, R_{b1}, R_{b2}, R_{b3}, and R_{b4} are each independently selected from hydrogen, halogen, hydroxyl, C₁-C₅ alkyl, or C₁-C₅ alkoxy, and the alkyl or alkoxy is optionally substituted with one or more halogen, hydroxyl, cyano, C₁-C₅ alkyl, C₁-C₅ alkoxy, or amino.

In a specific implementation of the present invention, R_{b1}, R_{b2}, R_{b3}, and R_{b4} are each independently selected from hydrogen, methyl, trifluoromethyl, ethyl, methoxy, or ethoxy.

In some specific implementations, R_{b1} and R_{b2} are each independently selected from hydrogen, methyl, or trifluoromethyl.

In some specific implementations, R_{b3} and R_{b4} are each independently selected from hydrogen, methyl, or trifluoromethyl.

In some specific implementations, any two of R_{b1}, R_{b2}, R_{b3}, and R_{b4} are linked together to form a 3- to 7-membered ring, the 3- to 7-membered ring may be a saturated or unsaturated carbocyclic or heterocyclic ring; the heterocyclic ring optionally contains one or more N, O, or S(=O)ₘ heteroatoms; and the 3- to 7-membered ring may be further optionally substituted with one or more halogen, hydroxyl, cyano, C₁-C₅ alkyl, C₁-C₅ alkoxy, or amino.

In some specific implementations, R_{b1} and R_{b2} are linked together to form a 3- to 7-membered saturated carbocyclic ring, and the 3- to 7-membered ring may be optionally substituted with one or more halogen, hydroxyl, cyano, C₁-C₅ alkyl, C₁-C₅ alkoxy, or amino.

In some specific implementations, R_{b1} and R_{b2} are linked together to form a 3-membered saturated carbocyclic ring, and the 3-membered ring may be optionally substituted with one or more halogen, hydroxyl, cyano, C₁-C₅ alkyl, C₁-C₅ alkoxy, or amino.

In some specific implementations, R_{b3} and R_{b4} are linked together to form a 3- to 7-membered saturated carbocyclic ring, and the 3- to 7-membered ring may be optionally substituted with one or more halogen, hydroxyl, cyano, C₁-C₅ alkyl, C₁-C₅ alkoxy, or amino.

In some specific implementations, any one of R_{b1} and R_{b2} and any one of R_{b3} and R_{b4} are linked together to form a 3- to 7-membered ring, and the 3- to 7-membered ring may be optionally substituted with one or more halogen, hydroxyl, cyano, C₁-C₅ alkyl, C₁-C₅ alkoxy, or amino.

In some specific implementations, any one of R_{b1} and R_{b2} and any one of R_{b3} and R_{b4} are linked together to form a 3- to 7-membered saturated carbocyclic ring, and the 3- to 7-membered saturated carbocyclic ring may be optionally substituted with one or more halogen, hydroxyl, cyano, C₁-C₅ alkyl, C₁-C₅ alkoxy, or amino.

In some specific implementations, any one of R_{b1} and R_{b2} and any one of R_{b3} and R_{b4} are linked together to form a 3- to 6-membered saturated carbocyclic ring, and the 3- to 6-membered saturated carbocyclic ring may be optionally substituted with one or more halogen, hydroxyl, cyano, C₁-C₅ alkyl, C₁-C₅ alkoxy, or amino.

In the present invention, a linkage between any one of R_{b1} and R_{b2} and any one of R_{b3} and R_{b4} may be a linkage between R_{b1} and R_{b3}, a linkage between R_{b1} and R_{b4}, a linkage between R_{b2} and R_{b3}, or a linkage between R_{b2} and R_{b4}.

M₁ and M₂ are each independently selected from C, O, S(=O)ₘ, or N-Rₙ, where Rₐ is optionally selected from hydrogen or C₁-C₅ alkyl. In a specific implementation, Rₙ is optionally selected from hydrogen, methyl, or ethyl.

In the compound of the present invention, a ring A has a structure shown in A 1 : 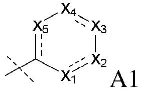
=-=-= optionally represents a single bond or a double bond, without violating the chemical bonding principle; and
X₁, X₂, X₃, X₄, and X₅ are each independently selected from a bond, C, N, O, C=O, or S(=O)ₘ.

That X₁, X₂, X₃, X₄, and X₅ are each selected from a "bond" indicates a direct linkage in the form of a bond, without violating the chemical bonding principle. For example, when X₂ is selected from a bond, X₁ is directly linked to X₃ by the bond, and then the ring A is a 5-membered ring.

In some specific implementations of the present invention, X₁, X₂, X₃, X₄, and X₅ are each independently selected from a bond, C, N, C=O, or S(=O)₂.

In some specific implementations of the present invention, X₁, X₂, X₃, X₄, and X₅ are all selected from C atoms.

In some specific implementations of the present invention, X₁, X₂, X₃, X₄, and X₅ optionally include one or two N atoms.

In some specific implementations of the present invention, X₁, X₂, X₃, X₄, and X₅ optionally include one C=O.

In some specific implementations of the present invention, X₁, X₂, X₃, X₄, and X₅ optionally include one S(=O)₂.

In some specific implementations of the present invention, X₁, X₂, X₃, X₄, and X₅ are optionally selected from a bond, C, S, and O.

In some specific implementations of the present invention, the ring A has the following structures:

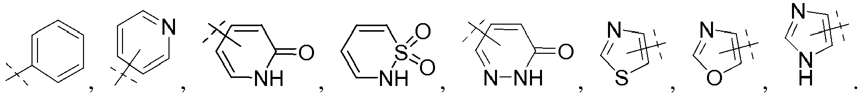

In the compound of the present invention, the ring A has a structure shown in A2: 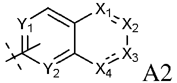 - - - optionally represents a single bond or a double bond, without violating the chemical bonding principle; and X₁, X₂, X₃, and X₄ are defined as in A1. That X₁, X₂, X₃, and X₄ are each selected from a "bond" indicates a direct linkage in the form of a bond. For example, when X₃ is selected from a bond, X₂ is directly linked to X₄ by the bond to form a 5-membered ring. Y₁ and Y₂ may be the same or different, and may be each optionally selected from a C atom or a N atom.

In some specific implementations of the present invention, X₁, X₂, X₃, and X₄ in A2 are each independently selected from a bond, C, N, or C=O.

In some specific implementations of the present invention, X₁, X₂, X₃, and X₄ in A2 are each independently selected from a bond, C, N, or O.

In some specific implementations of the present invention, X₁, X₂, X₃, and X₄ in A2 are each independently selected from a bond, C, N, or S(=O)ₘ.

In some specific implementations of the present invention, X₁, X₂, X₃, and X₄ in A2 are each independently selected from C, N, or C=O.

In some specific implementations of the present invention, the ring A has the following structures:

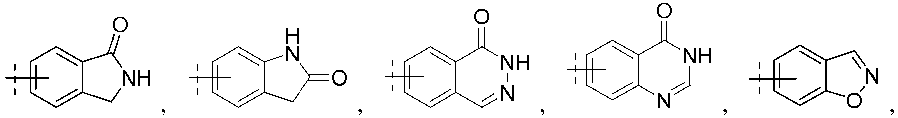

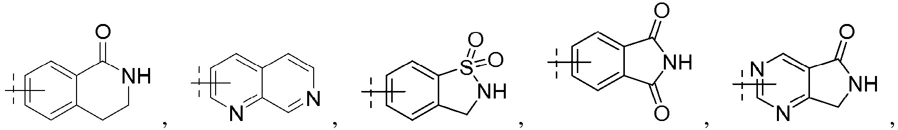

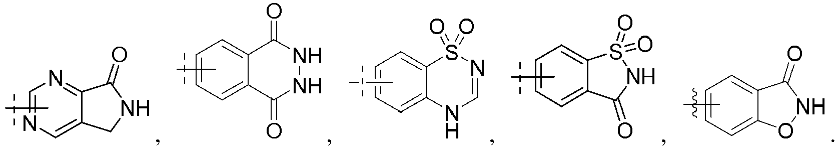

R_{c1} and R_{c2} are each independently selected from hydrogen, halogen, cyano, hydroxyl, amino, C₁-C₅ alkyl, C₁-C₅ alkoxy, -S(=O)ₘ-R_{c3}R_{c4}, -C(=O)-NR_{c3}R_{c4}, -C(=S)-NR_{c3}R_{c4}, - N(R_{c3}R_{c4})ₘ, -P(=O)ₘ-R_{c3}R_{c4}, -C(=N)-NR_{c3}R_{c4}, -S(=O)₂NR_{c3}R_{c4}, -CH₂NR_{c3}R_{c4}, or - S(=O)(=NR_{c3})R_{c4}; and the C₁-C₅ alkyl or C₁-C₅ alkoxy may be further substituted with one or more optionally selected from the following substituents: halogen, hydroxyl, cyano, amino, C₁-C₅ alkyl, and C₁-C₅ alkoxy.

R_{c1} and R_{c2} are substituted at any substitutable positions on the ring A, including at X₁, X₂, X₃, X₄, Y₁, and Y₂. In some specific implementations of the present invention, R_{c1} and R_{c2} are substituted at carbon atoms. In some specific implementations of the present invention, R_{c1} and R_{c2} are substituted at heteroatoms.

R_{c3} and R_{c4} are each independently selected from hydrogen, hydroxyl, amino, C₁-C₅ alkyl, C₁-C₅ alkoxy, or a 3- to 7-membered ring, or R_{c3} and R_{c4} are linked together to form a 3- to 7-membered ring; the 3- to 7-membered ring may be a saturated or unsaturated carbocyclic or heterocyclic ring; the heterocyclic ring optionally contains one or more N, O, or S(=O)ₘ heteroatoms; and the 3- to 7-membered ring may be further optionally substituted with one or more halogen, hydroxyl, cyano, C₁-C₅ alkyl, C₁-C₅ alkoxy, or amino.

In the substituent -S(=O)ₘ-R_{c3}R_{c4}, when m = 2, R_{c4} is absent.

In some specific implementations of the present invention, the compound shown in formula I is further shown in general formula II:

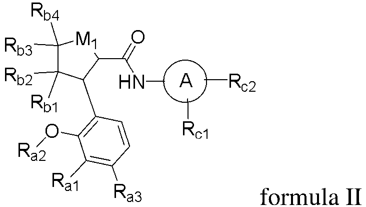

M₁, a ring A, Rₐ₁, Rₐ₂, Rₐ₃, R_{b1}, R_{b2}, R_{b3}, R_{b4}, R_{c1}, and R_{c2} are defined as above.

In some specific implementations of the present invention, R_{c1} and R_{c2} are each independently selected from hydrogen, hydroxyl, amino, fluorine, cyano, oxo, methyl, - C(=O)NH₂, -C(=O)NHCH₃, -C(=O)NHC₂H₅, -S(O)₂CH₃, -S(O)₂NH₂, -S(O)₂NHCH₃, - S(O)₂NHCD₃, -P(O)(CH₃)₂,

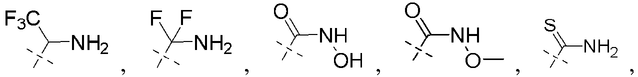

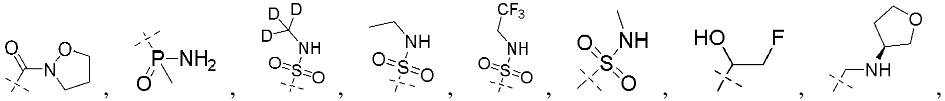

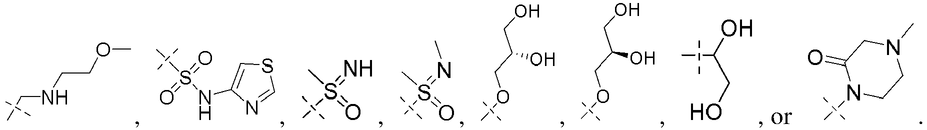

In some specific implementations of the present invention, R_{c1} and R_{c2} are each independently selected from hydrogen, fluorine, chlorine, amino, cyano, or -C(=O)NH₂. In the compound of the present invention, m and n are each optionally 0, 1, or 2.

In a specific implementation of the present invention, a specific compound of the present invention has the following structures:

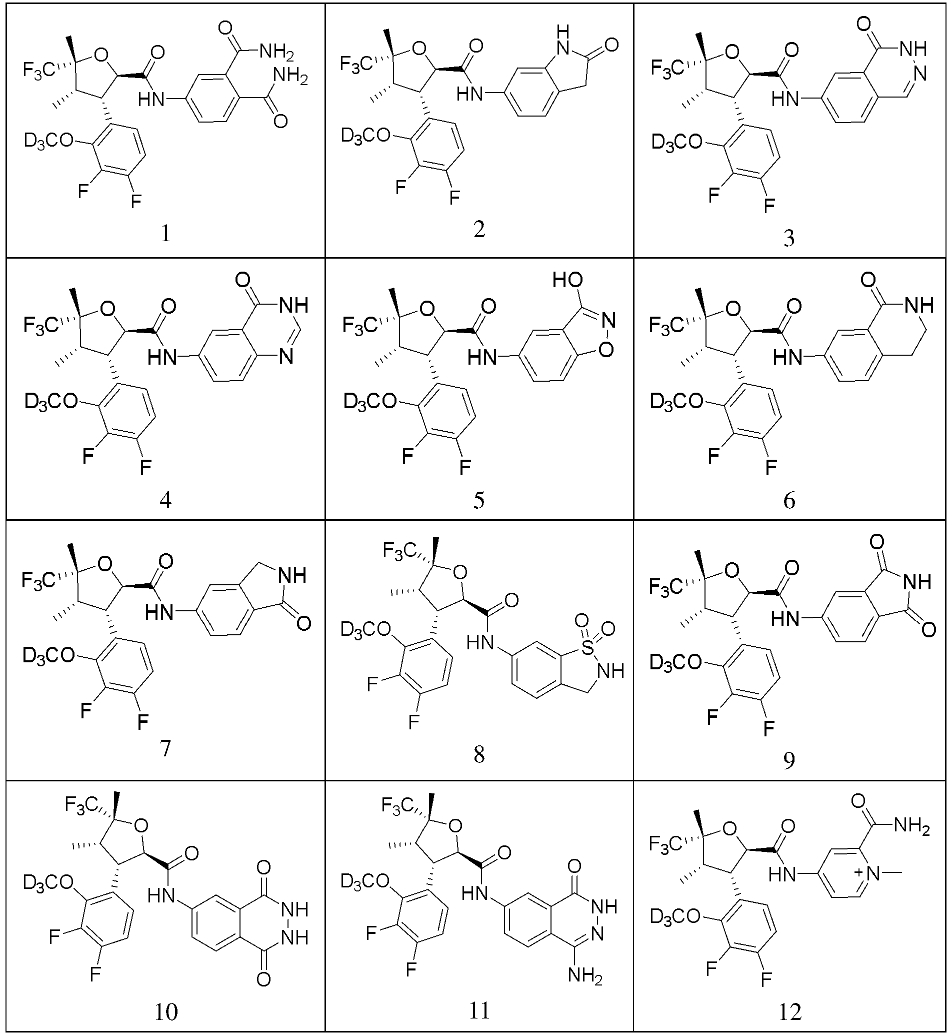

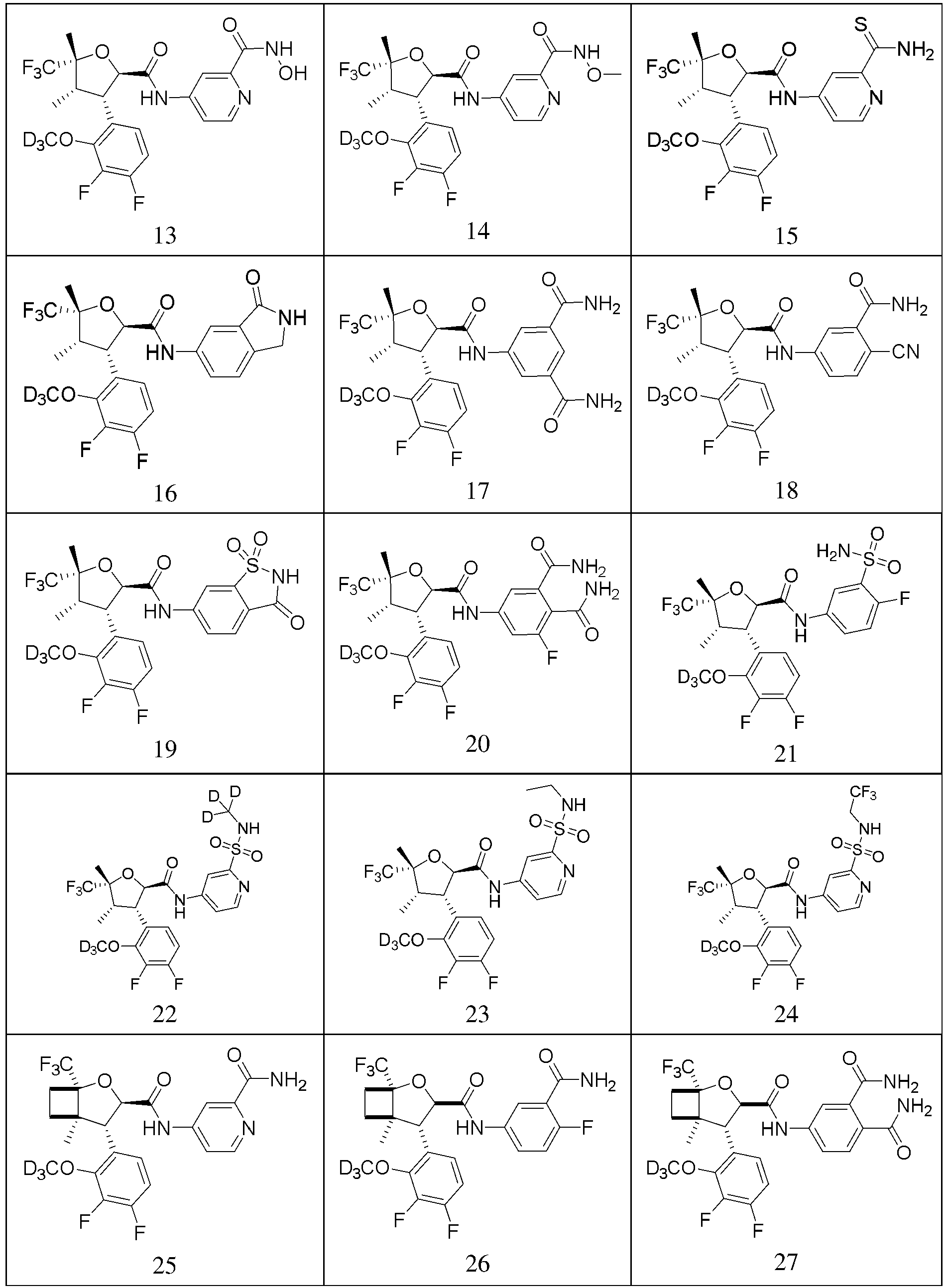

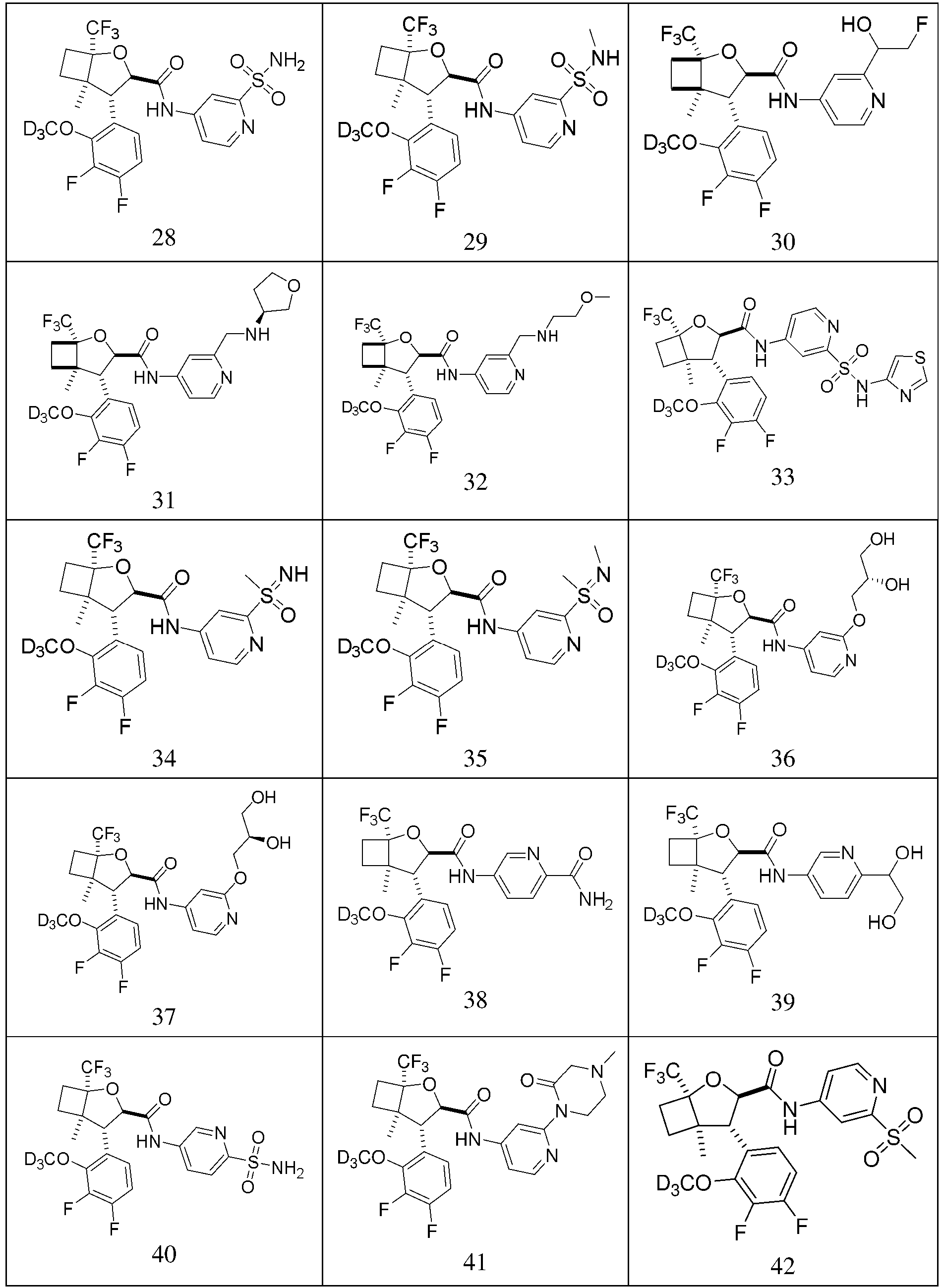

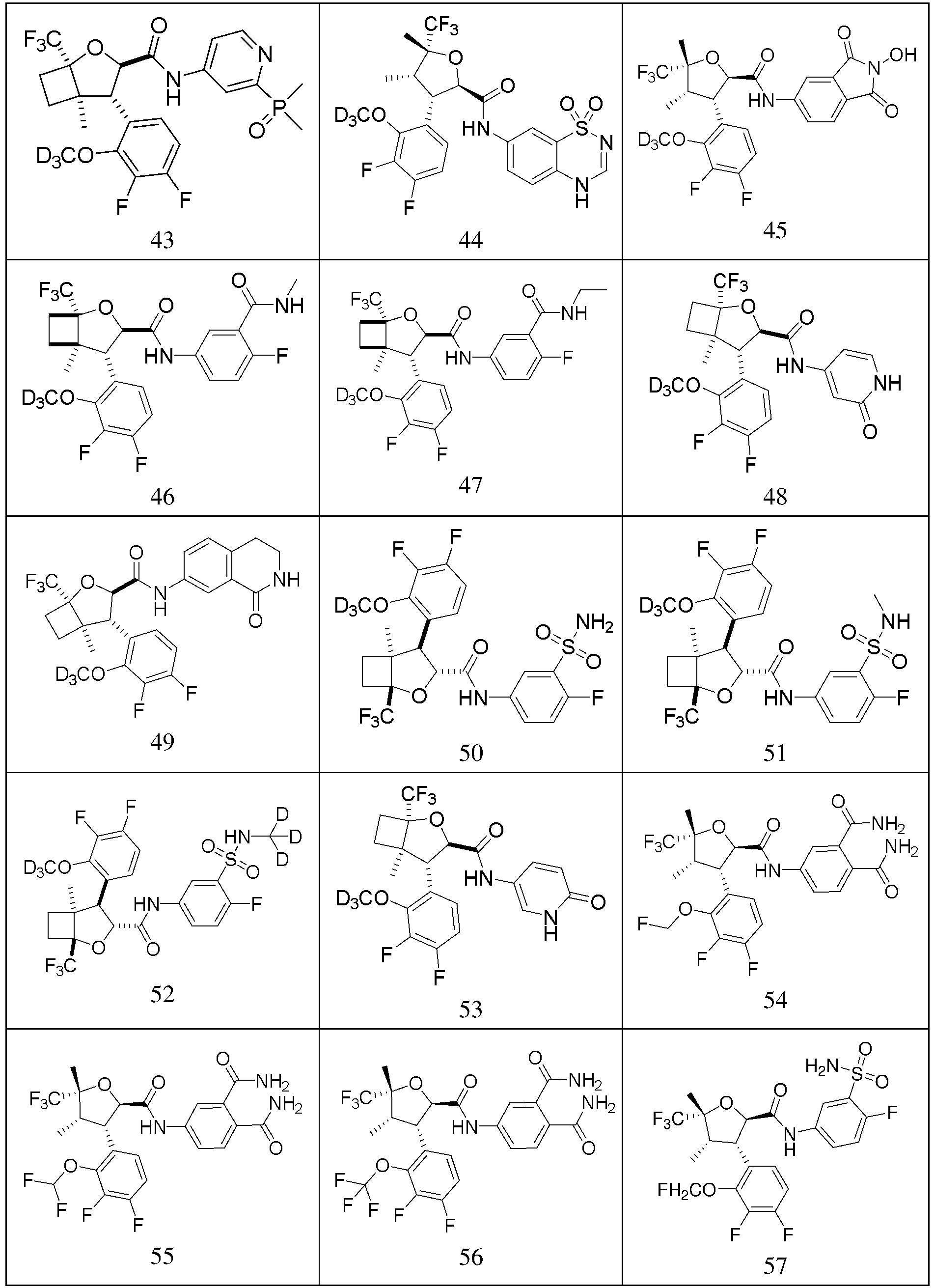

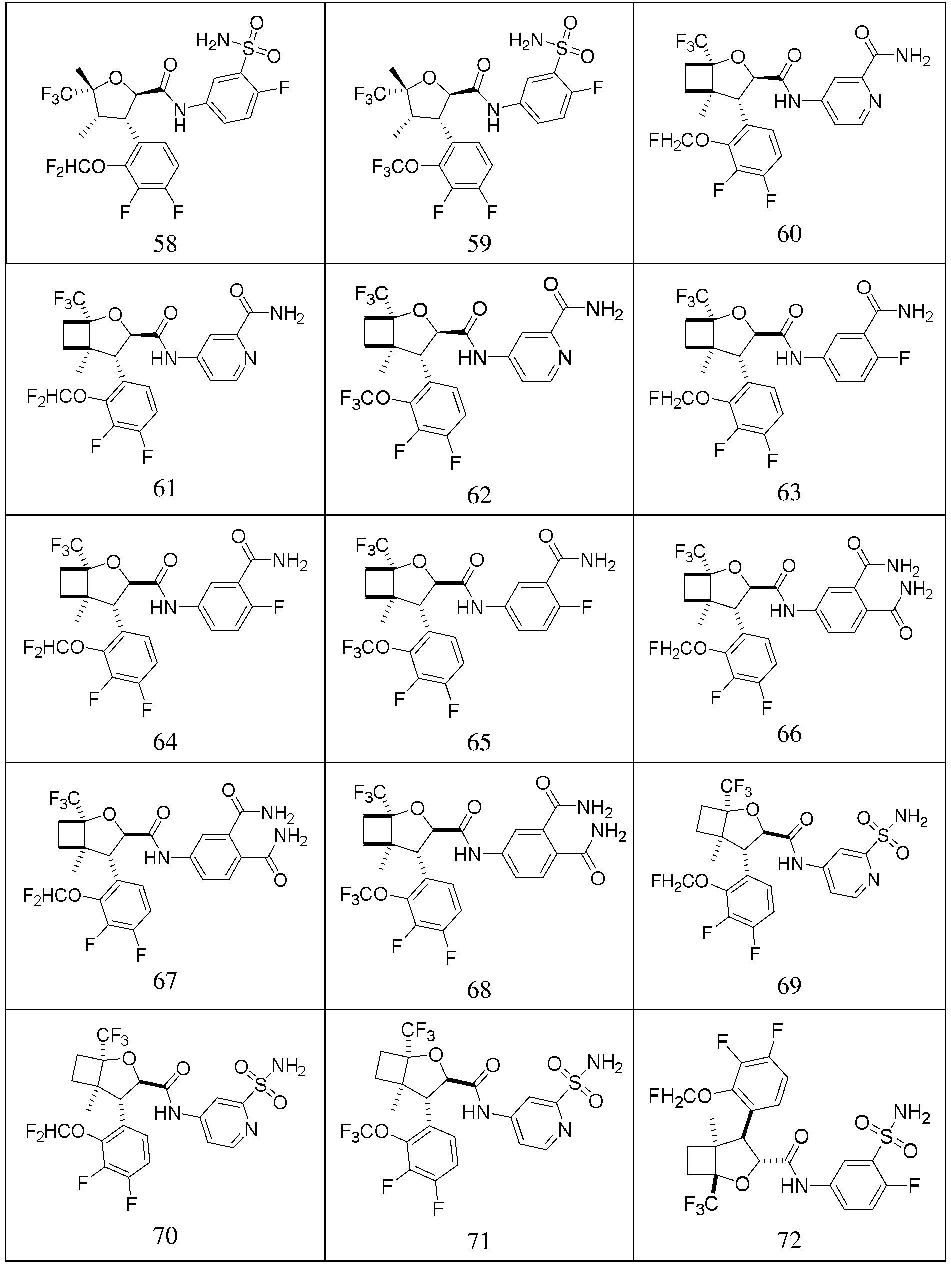

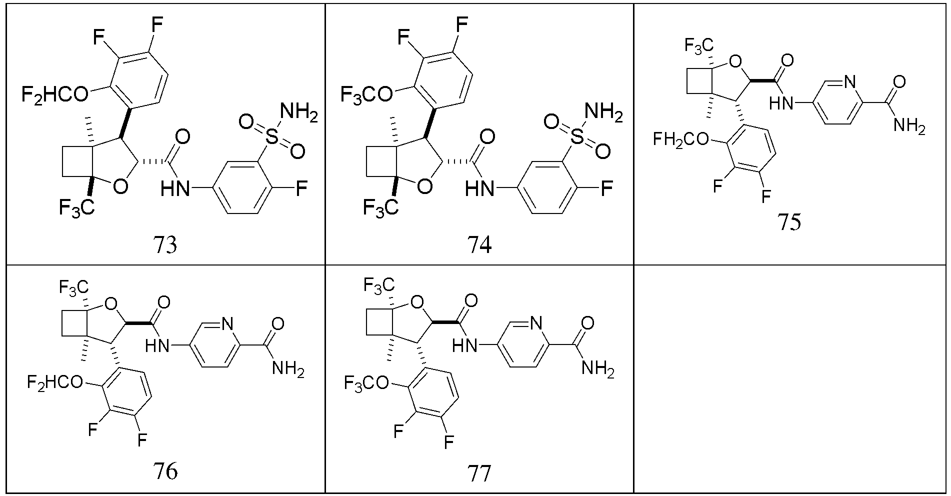

According to a second aspect of the present invention, provided is use of the compound shown in formula I or a pharmaceutically acceptable salt thereof in preparation of a drug for inhibition against voltage-gated sodium channels. The voltage-gated sodium channels are Naᵥ1.8.

According to a third aspect of the present invention, provided is use of the compound shown in formula I or a pharmaceutically acceptable salt thereof in preparation of a drug for treatment, prevention, or relief of a disease related to voltage-gated sodium channels, where the related disease includes, but is not limited to: pain, multiple sclerosis, Charcot-Marie-Tooth disease, incontinence, pathologic cough, or arrhythmia.

The pain includes: acute pain, chronic pain, intestinal pain, neuropathic pain, musculoskeletal pain, inflammatory pain, cancer pain, idiopathic pain, postoperative pain, and visceral pain. Therefore, the present invention further provides use of the compound shown in formula I or a pharmaceutically acceptable salt thereof in preparation of a drug for treatment, prevention, or relief of pain.

According to a fourth aspect of the present invention, provided is a pharmaceutical composition, where the pharmaceutical composition contains the compound shown in formula I or a pharmaceutically acceptable salt thereof, and a pharmaceutically acceptable excipient. The pharmaceutically acceptable excipient includes a filler, a disintegrant, a surfactant, and a solubilizer.

In the present invention, the pharmaceutical composition containing the compound shown in formula I or the pharmaceutically acceptable salt thereof may be administered in various known ways, such as orally, topically, rectally, parenterally, by inhalation, or by implantation. The pharmaceutical composition may be prepared in the form of tablets, capsules, powder in packets, sugar-coated pills, dispersions, granules, lozenges, powder for injection, liquid formulations, suppositories, or other formulations.

According to a fifth aspect of the present invention, provided is use of the pharmaceutical composition containing the compound shown in formula I or a pharmaceutically acceptable salt thereof in preparation of a drug for treatment, prevention, or relief of a disease related to voltage-gated sodium channels, where the disease related to voltage-gated sodium channels includes, but is not limited to: pain, multiple sclerosis, Charcot-Marie-Tooth disease, incontinence, pathologic cough, or arrhythmia.

The pain includes: acute pain, chronic pain, intestinal pain, neuropathic pain, musculoskeletal pain, inflammatory pain, cancer pain, idiopathic pain, postoperative pain, and visceral pain. Therefore, the present invention further provides use of the pharmaceutical composition containing the compound shown in formula I or a pharmaceutically acceptable salt thereof in preparation of a drug for treatment, prevention, or relief of pain.

### DETAILED DESCRIPTION

### Term Description

"Halogen" or "halogen atom" includes fluorine, chlorine, bromine, and iodine. In the present invention, the halogen is preferably fluorine or chlorine.

"C₁-C₅ alkyl" is straight- or branched-chain alkyl containing 1-5 carbon atoms, such as methyl, ethyl, propyl, isopropyl, n-butyl, isobutyl, tert-butyl, or n-pentyl. Preferably, C₁-C₅ alkyl is C₁-C₅ alkyl, including methyl, ethyl, propyl, or isopropyl.

"C₁-C₅ alkoxy" is straight- or branched-chain alkoxy containing 1-5 carbon atoms, such as methoxy, ethoxy, propoxy, isopropoxy, or tert-butoxy. Preferably, C₁-C₅ alkoxy is C₁-C₅ alkoxy, including methoxy, ethoxy, propoxy, or isopropoxy. "C₃-C₆ cycloalkyl" is cycloalkyl containing 3-6 carbon atoms, including cyclopropyl, cyclobutyl, cyclopentyl, or cyclohexyl. On the cycloalkyl, there may be further substitutions with the following substituents: one or more halogen, hydroxyl, cyano, C₁-C₅ alkyl, C₁-C₅ alkoxy, and amino.

"C₃-C₆ cycloalkoxy" is cycloalkoxy containing 3-6 carbon atoms, including cyclopropoxy, cyclobutoxy, cyclopentyloxy, or cyclohexyloxy. On the cycloalkoxy, there may be further substitutions with the following substituents: one or more halogen, hydroxyl, cyano, C₁-C₅ alkyl, C₁-C₅ alkoxy, and amino.

"3- to 7-membered ring" is a saturated or unsaturated ring containing 3-7 carbon atoms, such as a 3-membered ring, a 4-membered ring, a 5-membered ring, a 6-membered ring, or a 7-membered ring. In the present invention, a carbon atom on the ring may be optionally substituted with one or more C(=O), N, O, or S(=O)ₘ to form a 3- to 7-membered heterocyclic ring, and an atom on the 3- to 7-membered ring may be further substituted optionally with one or more halogen, hydroxyl, cyano, C₁-C₅ alkyl, C₁-C₅ alkoxy, or amino, where m is 0, 1, or 2.

"5- to 7-membered ring" is a saturated or unsaturated ring containing 5-7 carbon atoms, such as a 5-membered ring, a 6-membered ring, or a 7-membered ring. In the present invention, a carbon atom on the ring may be optionally substituted with one or more N, O, or S(=O)ₘ to form a 5- to 7-membered heterocyclic ring, and an atom on the 5- to 7-membered ring may be further substituted optionally with one or more halogen, hydroxyl, cyano, C₁-C₅ alkyl, C₁-C₅ alkoxy, or amino, where m is 0, 1, or 2.

"More" may indicate two, three, four, or more. In the present invention, a substitution with more halogen may form, for example, trifluoromethyl or difluoromethyl.

In the present invention, the alkyl, alkoxy, cycloalkyl, cycloalkoxy, 5- to 7-membered ring, or 3- to 7-membered ring may be optionally substituted with one or more deuterium, halogen, hydroxyl, cyano, oxo, C₁-C₅ alkyl, C₁-C₅ alkoxy, or amino.

As used herein, in any chemical structure or formula, a bold or hashed straight bond (respectively 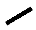 or 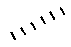 ) linked to a stereoisomeric center of a compound, for example, in 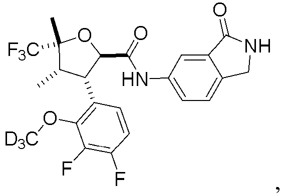 indicates the relative stereochemistry of the stereoisomeric center relative to another stereoisomeric center to which the bold or hashed straight bond is linked.

As used herein, in any chemical structure or formula, a bold or hashed wedge bond (respectively 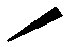 or 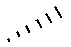 ) linked to a stereoisomeric center of a compound, for example, in 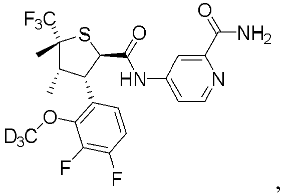 indicates the absolute stereochemistry of the stereoisomeric center and the relative stereochemistry of the stereoisomeric center relative to another stereoisomeric center to which the bold or hashed wedge bond is linked.

As used herein, the prefix *"rel-"* indicates a single enantiomer with an unknown absolute configuration when used in combination with a chiral compound. In a compound with the prefix *"rel-",* the (R)- and (S)- notations in the chemical name reflect the relative stereochemistry of the compound, but not necessarily the absolute stereochemistry of the compound.

"Pharmaceutically acceptable salt" includes, but is not limited to: an acid addition salt formed from the compound shown in formula I and an inorganic acid, such as hydrochloride, hydrobromide, phosphate, sulfate, or nitrate; and an acid addition salt formed from the compound shown in formula I and an organic acid, such as formate, acetate, malate, maleate, fumarate, tartrate, succinate, citrate, lactate, methanesulfonate, p-toluenesulfonate, 2-hydroxyethylsulfonate, or benzoate.

In the present invention, the compound 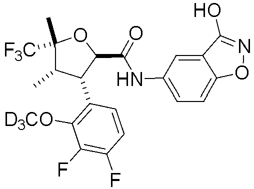 is an enol structure of the compound 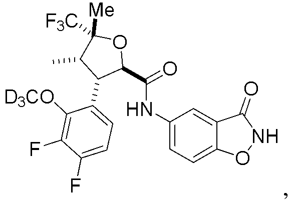 and they belong to the same compound.

A common method for preparing the compound in the present invention is as follows:

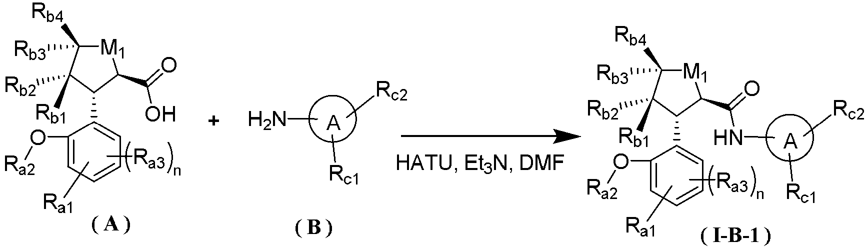

The compound shown in formula (A) reacts with the compound shown in formula (B) to obtain the compound in the present invention shown in formula (I-B-1), where the ring A, M₁, Rₐ₁, Rₐ₂, Rₐ₃, R_{b1}, R_{b2}, R_{b3}, R_{b4}, R_{c1}, R_{c2}, and n are defined as the foregoing in the present invention.

In the present invention, specific compounds are further prepared by the following methods. Unless otherwise specified, compounds, reagents, and the like used in examples of the present invention are purchased from qualified suppliers or synthesized according to methods disclosed in the related art. Some intermediate compounds may be synthesized according to the methods described in WO2022256660A1. For example, an intermediate **1a** is prepared according to a method in the related art.

### Synthesis of intermediate 1d:

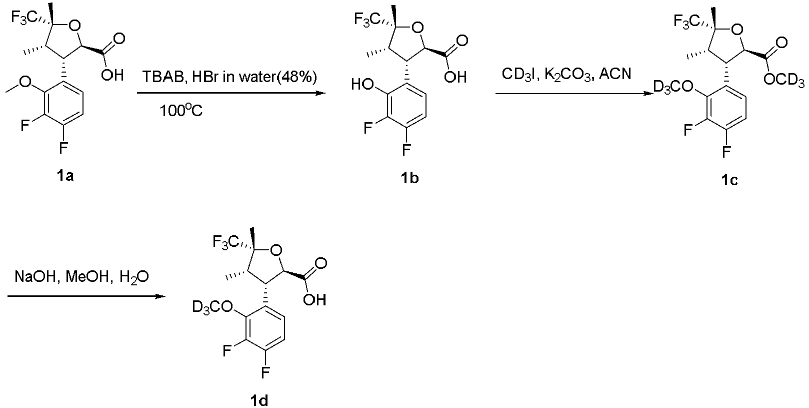

### Step 1: (2R,3S,4S,5R)-3-(3,4-difluoro-2-hydroxyphenyl)-4,5-dimethyl-5-(trifluoromethyl)tetrahydrofuran-2-carboxylic acid (1b)

(*2R,3S,4S,5R*)-3-(3,4-difluoro-2-methoxyphenyl)-4,5-dimethyl-5-(trifluoromethyl)tetrahydrofuran-2-carboxylic acid (**1a**, 1.5 g, 4.23 mmol, 1.0 eq), TBAB (1.5 g, 4.7 mmol, 1.1 eq), and 48% aqueous hydrobromic acid solution (25 mL) were added into a single-necked reaction flask. The reaction solution reacted at 100°C under nitrogen protection for 18 h. The reaction was monitored by LCMS until most of the raw materials were converted. Then, the reaction solution was subjected to extraction with water and dichloromethane. A dichloromethane layer was collected, concentrated, and then subjected to column chromatography (PE/EA = 0-100%), to obtain the compound **1b** (1.2 g, 83.3% yield), which was a yellow liquid. LCMS: m/z (254 nm): 339.3 [M-H]⁻.

### Step 2: (2R,3S,4S,5R)-3-(3,4-difluoro-2-methoxyphenyl-d3)-4,5-dimethyl-5-(trifluoromethyl)tetrahydrofuran-2-methyl formate-d3 (1c)

**1b** (0.5 g, 1.5 mmol, 1.0 eq), iodomethane-d3 (1.0 g, 7.4 mmol, 5.0 eq), potassium carbonate (1.9 g, 14.7 mmol, 10.0 eq), and acetonitrile (25 mL) were added into a single-necked reaction flask to react at room temperature for 18 h. The reaction was monitored by LCMS until the reaction was completed. Then, extraction was carried out with water and ethyl acetate. An ethyl acetate layer was collected and concentrated, to obtain the compound **1c** (0.7 g, crude), which was a yellow liquid. LCMS: m/z (254 nm): 375.3 [M+H]⁺.

### Step 3: (2R,3S,4S,5R)-3-(3,4-difluoro-2-methoxyphenyl-d3)-4,5-dimethyl-5-(trifluoromethyl)tetrahydrofuran-2-carboxylic acid (1d)

The compound **1c** (0.7 g, 1.5 mmol, 1.0 eq), sodium hydroxide (0.3 g, 7.4 mmol, 5.0 eq), water (20 mL), and methanol (20 mL) were added into a single-necked reaction flask to react at room temperature for 16 h. The reaction was monitored by LCMS until the reaction was completed. Then, the reaction solution was concentrated to remove methanol, adjusted with hydrochloric acid to pH = 1, and subjected to extraction with water and ethyl acetate. An ethyl acetate layer was collected, concentrated, and subjected to column chromatography (PE/EA = 0-100%), to obtain the compound **1d** (0.46 g, 86% yield obtained through the two-step reaction), which was a yellow liquid. LCMS: m/z (254 nm): 356.3 [M-H]⁻.

### Synthesis of intermediate 3d:

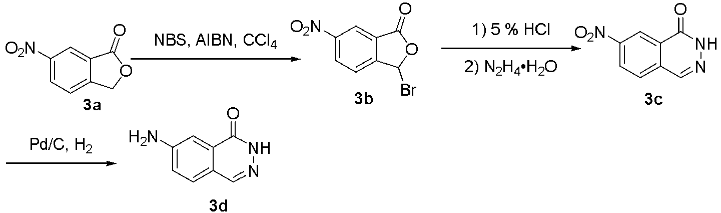

### Step 1: 3-bromo-6-nitrobenzofuran-1(3H)-one (3b)

**3a** (3.0 g) and CCl₄ (30 mL) were added in a 100 mL reaction flask, and NBS (3.28 g) and AIBN (275 mg) were added sequentially while stirring. Then, the reaction solution was heated to 78°C and stirred for 12 h. The reaction was monitored by TLC until the raw materials were fully converted. The reaction solution was cooled down to room temperature and filtered with diatomaceous earth. The filtrate was concentrated and separated by column chromatography (EA/PE, 0%-30%), to obtain 3.3 g of yellow solid with a yield of 77%. LCMS: m/z (254 nm): 257.9 [M+H]⁺

### Step 2: 7-nitrophthalazin-1(2H)-one (3c)

**3b** (1.0 g) and 5% HCl (5 mL/mmol) were added in a reaction flask and stirred at 80°C for 3 h. The reaction was monitored by TLC until the raw materials were fully converted. The reaction solution was cooled down to room temperature. N₂H₄·H₂O (274 mg) was added. Then, the reaction solution was stirred at room temperature for 3 h. The reaction was monitored by TLC until the raw materials were fully converted. The reaction solution was filtered and washed with water. The solid residue was separated by column chromatography to obtain 237 mg of white solid with a yield of 32%. LCMS: m/z (254 nm): 192.0 [M+H]⁺

### Step 3: 7-aminophthalazin-1(2H)-one (3d)

**3c** (200 mg), MeOH (10 mL), and THF (10 mL) were added in a reaction flask and stirred uniformly. Then, palladium on carbon (20 mg) was added. The mixture was purged with hydrogen for three times, and stirred at room temperature for 4 h. The reaction was monitored by TLC until the raw materials were fully converted. The reaction solution was filtered with diatomaceous earth. The filtrate was concentrated to obtain 120 mg of white solid with a yield of 71%. LCMS: m/z (254 nm): 162.0 [M+H]⁺

### Synthesis of intermediate 5e:

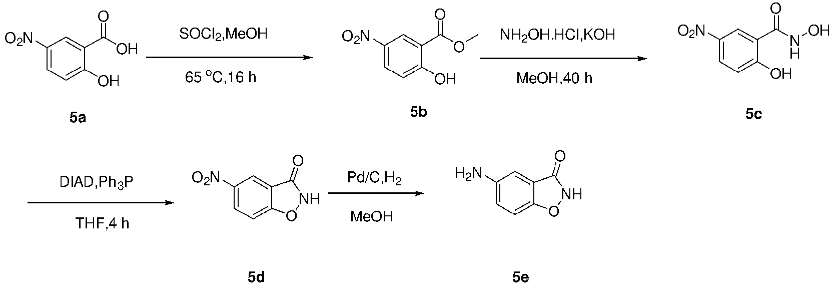

### Step 1: methyl 2-hydroxy-5-nitrobenzoate (5b)

Methanol (50 mL) and **5a** (5.0 g) were added in a three-necked flask, and sulfoxide chloride (10 mL) was slowly added dropwise to react at 65°C for 16 h. Then, the mixture was concentrated until dried. Water and ethyl acetate were added to carry out extraction. An ethyl acetate layer was collected and concentrated to obtain 6.0 g of white solid with a yield of 111 %. ESI-MS m/z: 198.1 (M+1)⁺.

### Step 2: (2-hydroxy-5-nitrophenyl)methane hydroxamic acid (5c)

Methanol (500 mL) and potassium hydroxide (21 g) were added in a reaction flask. Hydroxylamine hydrochloride (17.5 g) was added under an ice bath and stirred for 1.5 h. Then, the mixture was filtered to obtain a filtrate. **5b** (5 g) was added to the filtrate to react for 16 h. The reaction was monitored by LCMS until the reaction was completed. The reaction solution was concentrated to remove methanol, adjusted with hydrochloric acid to pH 2, and subjected to extraction with water and ethyl acetate. An ethyl acetate layer was collected, dried, and concentrated to obtain 4.8 g of light yellow solid with a yield of 96%. ESI-MS m/z: 198.9 (M+1)⁺.

### Step 3: 5-nitro-2,3-dihydrobenzo[d]isoxazol-3-one (5d)

THF (100 mL) and **5c** (2.76 g) were added in a reaction flask. Triphenylphosphine (4.8 g) was added under nitrogen protection. The mixture was stirred under an ice bath for 20 min. Then, DIAD (3.7 g) was added to react for 4 h. The reaction was monitored by LCMS until the reaction was completed. The reaction solution was concentrated to remove the solvent, and then water was added. Then, the reaction solution was adjusted with sodium hydroxide until pH 13, and subjected to extraction with DCM. An aqueous layer was collected, adjusted with hydrochloric acid until pH 1, subjected to extraction with DCM, washed with saturated saline, and concentrated to obtain 1.6 g of white solid with a yield of 64%. ESI-MS m/z: 181.0 (M+1)⁺.

### Step 4: 5-amino-2,3-dihydrobenzo[d]isoxazol-3-one (5e)

Methanol (50 mL), **5d** (800 mg), and 5% palladium on carbon (200 mg) were added in a reaction flask to react under hydrogenation conditions for 36 h. The reaction was monitored by LCMS until the reaction was completed. The reaction solution was filtered. The filtrate was collected and concentrated to obtain 400 mg of white solid with a yield of 60.6%. ESI-MS m/z: 151.0 (M+1)⁺.

### Synthesis of intermediate 8b: 6-aminophenylpropyl[d]isothiazol-3(2H)-one-1,2-dioxygen

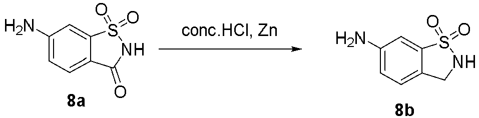

**8a** (650 mg) and concentrated hydrochloric acid (9 mL) were added in a reaction flask under an ice bath, and zinc powder (1.1 g) was added in batches within about 30 min. Then, the mixture was stirred at room temperature for 2 h. The reaction was monitored by LCMS until the raw materials were fully converted. The reaction solution was adjusted with a saturated sodium bicarbonate solution under an ice bath to be weakly basic, and subjected to extraction with ethyl acetate (50 mL × 3). The organic phases were combined, dried over anhydrous sodium sulfate, and filtered, to obtain 320 mg of brown solid (about 70% purity) with a yield of 37.0%. LCMS: MS m/z (254 nm): 185.0 [M+H]⁺.

### Synthesis of intermediate 11c:

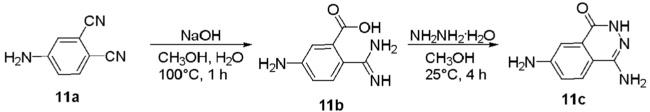

### Step 1: 5-amino-2-carbamimidoylbenzoic acid (11b)

The compound 4-aminophthalonitrile **11a** (500 mg, 3.49 mmol, 1.0 eq) was dissolved in methanol and water in 3:2 (5 mL), and sodium hydroxide (139 mg, 3.49 mmol, 1.0 eq) was added. The reaction solution reacted at 100°C under nitrogen protection for 1 h. The reaction was monitored by LCMS until the raw materials were fully converted. Then, the reaction solution was cooled down to room temperature and concentrated. The residue was separated and purified by prep-HPLC (0.01% FA) to obtain the compound 5-amino-2-carbamimidoylbenzoic acid **11b** (733 mg, 82% yield), which was a yellow solid. LCMS: [M+H]⁺ = 180.1.

### Step 2: 4,7-diamino-1,2-dihydrobenzo[2,1-d][1,2]dioxine-1-one (11c)

The compound 5-amino-2-carbamimidoylbenzoic acid **11b** (680 mg, 3.79 mmol, 1.0 eq) was dissolved in methanol (10 mL), and hydrazine hydrate (7 mL) was added. The reaction solution reacted at 25°C for 4 h. The reaction was monitored by LCMS until the reaction was completed. The reaction mixture was concentrated. The residue was subjected to extraction with water (10 mL) and ethyl acetate (10 mL × 5), washed with water and saturated saline sequentially, dried over anhydrous sodium sulfate, and then concentrated. The residue was filtered. The filter residue was dried under vacuum to obtain the compound 4,7-diaminophthalazin-1-ol **11c** (100 mg, 13% yield), which was a yellow solid. LCMS: [M+H]⁺ = 177.1.

### Synthesis of intermediate 13c:

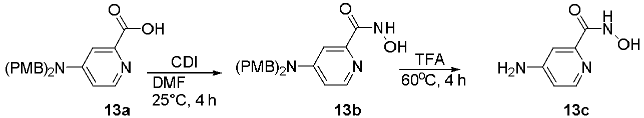

### Step 1: 4-(bis(4-methoxybenzyl)amino)-N-hydroxypicolinamide (13b)

The compound 4-(bis(4-methoxybenzyl)amino)picolinic acid **13a** (800 mg, 2.1 mmol, 1.0 eq) was dissolved in N,N-dimethylformamide (10 mL), and N,N'-carbonyldiimidazole (411.32 mg, 2.5 mmol, 1.2 eq) was added. The mixture was stirred at 25°C for 2 h. Then, hydroxylamine hydrochloride (882.81 mg, 10.6 mmol, 5.0 eq) was added. The reaction solution was stirred at 25°C to react for 2 h. The reaction was monitored by LCMS until the reaction was completed. The reaction solution was poured into water and subjected to extraction with ethyl acetate. The organic phase was washed with saturated saline. The organic phase was dried and concentrated. The residue was separated and purified by silica gel column chromatography (dichloromethane/methanol = 10/1) to obtain the compound **13b** (500 mg, 60% yield), which was a brown solid. LCMS: [M+H]⁺ = 394.2.

### Step 2: 4-amino-N-hydroxypicolinamide (13c)

The compound **13b** (500 mg, 1.3 mmol, 1.0 eq) was dissolved in trifluoroacetic acid (3 mL). The reaction solution was stirred at 60°C to react for 4 h. The reaction was monitored by LCMS until the reaction was completed. The reaction solution was concentrated. The residue was dissolved in dichloromethane, and a hydrogen chloride ether solution (2 mL) was added. Then, the mixture was concentrated to obtain a hydrochloride salt of the compound **13c** (260 mg, 93% yield), which was a brown solid. LCMS: [M+H]⁺ = 154.2.

### Synthesis of intermediate 14d:

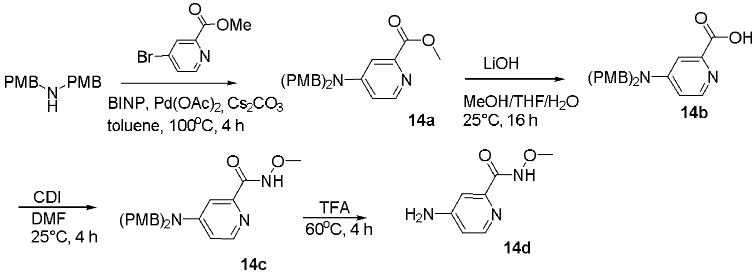

### Step 1: 4-(bis(4-methoxybenzyl)amino)methyl picolinate (14a)

Bis(4-methoxybenzyl)amine (4 g, 1.8 mmol, 1.0 eq), methyl 4-bromopicolinate (6.2 g, 2.4 mmol, 1.3 eq), 1,1'-[1,1'-binaphthyl]-2,2'-diylbis[1,1-diphenyl-(ACI)phosphine (580 mg, 0.09 mmol, 0.05 eq), palladium acetate (210 mg, 0.09 mmol, 0.05 eq), cesium carbonate (12.06 g, 3.7 mmol, 2.0 eq), and toluene (40 mL) were added sequentially in a microwave tube and stirred to react at 100°C for 4 h. The reaction was monitored by LCMS until the reaction was completed. The reaction solution was filtered, and the filtrate was concentrated. The residue was separated and purified by silica gel column chromatography (petroleum ether/ethyl acetate = 1/1) to obtain **14a** (3.2 g, 44% yield), which was a yellow solid. LCMS: [M+H]⁺ = 393.2.

### Step 2: 4-(bis(4-methoxybenzyl)amino)picolinic acid (14b)

The compound 4-(bis(4-methoxybenzyl)amino)methyl picolinate **14a** (3.2 g, 8.2 mmol, 1.0 eq) was dissolved in methanol, tetrahydrofuran, and water in 3/3/1 (30 mL), and lithium hydroxide monohydrate (1 g, 24.6 mmol, 3.0 eq) was added. Then, the mixture was stirred to react at 25°C for 16 h. The reaction was monitored by LCMS until the reaction was completed. The reaction solution was poured into water, adjusted with 1 N dilute hydrochloric acid to pH 5, and subjected to extraction with ethyl acetate. The organic phase was washed with saline. The organic phase was dried and concentrated to obtain the compound 4-(bis(4-methoxybenzyl)amino)picolinic acid **14b** (2 g, 65% yield), which was a yellow solid. LCMS: [M+H]⁺ = 379.1.

### Step 3: 4-(bis(4-methoxybenzyl)amino)-N-methoxypicolinamide (14c)

The compound 4-(bis(4-methoxybenzyl)amino)picolinic acid **14b** (200 mg, 0.53 mmol, 1.0 eq) was dissolved in N,N-dimethylformamide (2 mL), and N,N'-carbonyldiimidazole (102.8 mg, 0.63 mmol, 1.2 eq) was added. The mixture was stirred at 25°C for 2 h. Then, methoxyamine hydrochloride (221 mg, 10.6 mmol, 5.0 eq) was added. The reaction solution was stirred at room temperature to react for 2 h. The reaction was monitored by LCMS until the reaction was completed. The reaction solution was poured into water and subjected to extraction with ethyl acetate. The organic phase was washed with saline. The organic phase was dried and concentrated. The residue was separated and purified by silica gel column chromatography (dichloromethane/methanol = 20/1) to obtain the compound 4-(bis(4-methoxybenzyl)amino)-N-methoxypicolinamide **14c** (140 mg, 65% yield), which was a brown solid. LCMS: [M+H]⁺ = 408.1.

### Step 4: 4-amino-N-methoxypicolinamide (14d)

The compound 4-(bis(4-methoxybenzyl)amino)-N-methoxypicolinamide **14c** (100 mg, 0.24 mmol, 1.0 eq) was dissolved in trifluoroacetic acid (2 mL). The reaction solution was stirred at 60°C to react for 4 h. The reaction was monitored by LCMS until the reaction was completed. The reaction solution was concentrated. The residue was dissolved in dichloromethane, and hydrogen chloride ether (2 mL) was added. Then, the mixture was concentrated to obtain a hydrochloride salt of the compound 4-amino-N-methoxypicolinamide **14d** (80 mg, 78% yield), which was a brown solid. LCMS: [M+H]⁺ = 168.1.

### Synthesis of intermediate 18d:

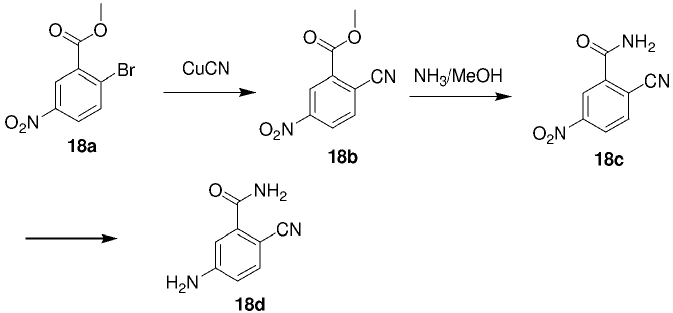

### Step 1: methyl 2-cyano-5-nitrobenzoate (18b)

**18a** (3 g), cuprous cyanide (2 g), and DMF (30 mL) were added in a reaction flask and stirred uniformly, and Pd₂(dba)₃ (527 mg) and dppf (638 mg) were added sequentially. The mixture was purged with nitrogen for three times, and stirred at 115°C for 16 h. The reaction solution was cooled down to room temperature, and then water (70 mL) was added. Then, the reaction solution was subjected to extraction with ethyl acetate (40 mL × 3). The organic phases were combined, concentrated, and separated by column chromatography (EA/PE, 0%-15%), to obtain 2 g of white solid with a yield of 84.4%. LCMS: m/z (254 nm): 207.0 [M+H]⁺

### Step 2: 2-cyano-5-nitrobenzamide (18c)

**18b** (1 g) and an ammonia in methanol solution (7 M, 10 mL) were added in a reaction flask and stirred at room temperature for 16 h. The reaction solution was filtered and washed with methanol to obtain 720 mg of light yellow solid with a yield of 77.6%. LCMS: m/z (254 nm): 192.0 [M+H]⁺

### Step 3: 2-cyano-5-anthranilamide (18d)

**18c** (300 mg), MeOH (10 mL), and THF (10 mL) were added in a reaction flask and stirred uniformly. Then, palladium on carbon (30 mg) was added. The mixture was purged with hydrogen for three times, and stirred at room temperature for 6 h. The reaction was monitored by TLC until the raw materials were fully converted. The reaction solution was filtered with diatomaceous earth. The filtrate was concentrated to obtain 179 mg of yellow solid with a yield of 71%. LCMS: m/z (254 nm): 162.0 [M+H]⁺. ¹H NMR (400 MHz, DMSO-*d₆*) *δ* 9.00 (s, 2H), 7.58 (d, 1H), 6.77 (d, 1H), 6.73 (dd, 1H), 5.99 (s, 2H).

### Synthesis of intermediate 20f:

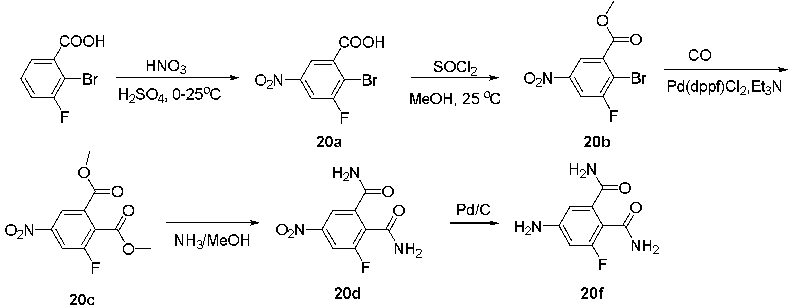

### Step 1: 2-bromo-3-fluoro-5-nitrobenzoic acid (20a)

The compound 2-bromo-3-fluorobenzoic acid (5 g, 22.8 mmol, 1.0 eq) was dissolved in concentrated sulfuric acid (15 mL), and fuming nitric acid (1 mL) was added at 0°C. The reaction mixture was stirred at 25°C to react for 4 h. The reaction was monitored by LCMS until the reaction was completed. The reaction solution was poured slowly into ice water and subjected to extraction with dichloromethane (50 mL × 3). The organic phase was collected. The organic phase was washed with saturated saline (50 mL × 3), dried over anhydrous sodium sulfate, filtered, and concentrated, to obtain a residue. The residue was purified by C18 column chromatography (0.1% FA/acetonitrile = 1/4) to obtain the compound 2-bromo-3-fluoro-5-nitrobenzoic acid **20a** (800 mg, 13%), which was a yellow solid. LCMS: [M+H]⁺ = 263.9.

### Step 2: methyl 2-bromo-3-fluoro-5-nitrobenzoate (20b)

The compound 2-bromo-3-fluoro-5-nitrobenzoic acid **20a** (800 mg, 3.03 mmol, 1.0 eq) was dissolved in methanol (5 mL), and thionyl chloride (1 mL) was added. The reaction mixture was stirred at 80°C to react for 6 h. The reaction was monitored by LCMS until the reaction was completed. The reaction solution was filtered to obtain a filtrate. The filtrate was concentrated to obtain the compound methyl 2-bromo-3-fluoro-5-nitrobenzoate **20b** (800 mg, 95%), which was a yellow solid. LCMS: [M+H]⁺ = 277.9.

### Step 3: dimethyl 3-fluoro-5-nitrobenzoate (20c)

The compound methyl 2-bromo-3-fluoro-5-nitrobenzoate **20b** (400 mg, 1.43 mmol, 1.0 eq) was dissolved in methanol (10 mL), and Pd(dppf)Cl₂ (32 mg, 0.143 mmol, 0.1 eq) and Et₃N (437 mg, 4.32 mmol, 3.0 eq) were added. The reaction solution was stirred in the presence of carbon monoxide (0.1 MPa) at 80°C for 16 h. The reaction solution was concentrated to obtain a residue. The residue was purified by silica gel column chromatography (petroleum ether/ethyl acetate = 10/1) to obtain the compound dimethyl 3-fluoro-5-nitrobenzoate **20c** (40 mg, 11%), which was a brown oily substance. LCMS: [M+H]⁺ = 258.1.

### Step 4: ammonium 3-fluoro-5-nitrophthalate (20d)

The compound dimethyl 3-fluoro-5-nitrobenzoate **20c** (40 mg, 0.155 mmol, 1.0 eq) was dissolved in ammonia in methanol (5 mL). The reaction solution was stirred at 25°C for 16 h. The reaction solution was concentrated to obtain a residue. The residue was washed with dichloromethane, and filtered. The solid was collected to obtain the compound ammonium 3-fluoro-5-nitrophthalate **20d** (20 mg, 57%), which was a white solid. LCMS: [M+H]⁺ = 228.1.

### Step 5: ammonium 3-fluoro-5-aminophthalate (20f)

The compound ammonium 3-fluoro-5-nitrophthalate **20d** (20 mg, 0.088 mmol, 1.0 eq) was dissolved in methanol (5 mL). The reaction solution was stirred in the presence of hydrogen at 25°C for 1 h. The reaction solution was filtered, and the filtrate was collected and concentrated to obtain the compound ammonium 3-fluoro-5-aminophthalate **20f** (13 mg, 75%), which was a yellow solid. LCMS: [M+H]⁺ = 198.1.

### Synthesis of intermediate 21c:

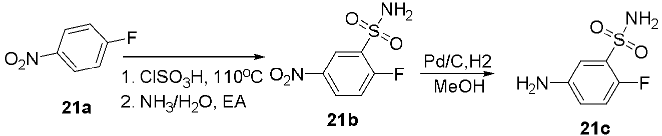

### Step 1: 2-fluoro-5-nitrobenzenesulfonamide (21b)

The compound p-fluoronitrobenzene **21a** (2.0 g, 14.2 mmol, 1.0 eq) was dissolved in chlorosulfonic acid (10 mL). The reaction mixture was stirred at 110°C to react for 24 h. The reaction solution was cooled down to room temperature. The reaction solution was poured slowly into an ice bath, and subjected to extraction with ethyl acetate (50 mL × 3) when all the ice melted. The organic phase was collected. The organic phase was washed with saturated saline (50 mL × 3), dried over anhydrous sodium sulfate, filtered, and concentrated, to obtain a residue. The residue was dissolved in ethyl acetate (60 mL), and aqueous ammonia (60 mL) was added slowly at 0°C. The reaction mixture was stirred at 25°C for 16 h. The reaction was monitored by TLC until the reaction was completed. The reaction solution was poured into water and subjected to extraction with ethyl acetate (50 mL × 3). The organic phase was collected. The organic phase was washed with saturated saline (50 mL × 3), dried over anhydrous sodium sulfate, filtered, and concentrated, to obtain a residue. The residue was purified by silica gel column chromatography (petroleum ether/ethyl acetate = 2/3) to obtain the compound 2-fluoro-5-nitrobenzenesulfonamide **21b** (400 mg, 13% yield), which was a yellow solid. ¹H NMR (400 MHz, DMSO) *δ* 8.56-8.53 (m, 2H), 8.06 (s, 2H), 7.86-7.65 (m, 1H).

### Step 2: 5-amino-2-fluorobenzenesulfonamide (21c)

The compound 2-fluoro-5-nitrobenzenesulfonamide **21b** (200 mg, 0.90 mmol, 1.0 eq) was dissolved in methanol (5 mL), and wet palladium on carbon (193 mg, 1.80 mmol, 2.0 eq) was added. The reaction mixture was stirred in the presence of hydrogen at 25°C to react for 1 h. The reaction was monitored by LCMS until the reaction was completed. The reaction solution was filtered to obtain a filtrate. The filtrate was concentrated to obtain the compound 5-amino-2-fluorobenzenesulfonamide **21c** (160 mg, 92% yield), which was a black solid. LCMS: [M+H]⁺ = 191.1

### Preparation of intermediate 25g:

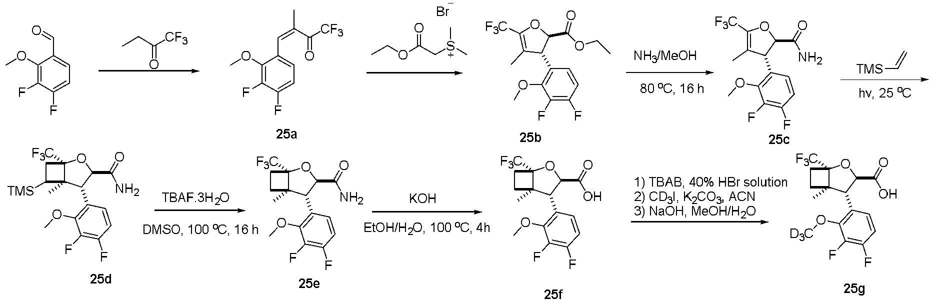

### Step 1: (3Z)-4-(3,4-difluoro-2-methoxyphenyl)-1,1,1-trifluoro-3-methyl-3-buten-2-one (25a)

Toluene (25 mL), 3,4-difluoro-2-methoxybenzaldehyde (5.0 g, 29.0 mmol, 1.0 eq), 1,1,1-trifluorobutan-2-one (11 g, 87.2 mmol, 3.0 eq), piperidinium acetate (4.2 g, 29.0 mmol, 1.0 eq), and acetic acid (0.9 g, 14.5 mmol, 0.5 eq) were added sequentially in a reaction flask to react with the reaction flask sealed at room temperature for 2 h, and heated to 75°C to react for 16 h. The reaction was monitored by TLC until the reaction was completed. Extraction was carried out with water and ethyl acetate. The ethyl acetate layer was collected, concentrated, and then separated by column chromatography (PE:EA = 20:1), to obtain **25a** (7.5 g, 92% yield), which was a yellow liquid.

### Step 2: rac-(2R,3S)-3-(3,4-difluoro-2-methoxyphenyl)-4-methyl-5-(trifluoromethyl)-2,3-dihydrofuran-2-carboxylic acid ethyl ester (25b)

Acetonitrile (25 mL), the compound **25a** (2.5 g, 8.9 mmol, 1.0 eq), (ethoxycarbonylmethyl)dimethylsulfonium bromide (2.6 g, 11.6 mmol, 1.3 eq), and cesium carbonate (3.8 g, 11.6 mmol, 1.3 eq) were added sequentially in a reaction flask to react at room temperature for 16 h. The reaction was monitored by TLC (PE:EA = 10:1) until the compound **25a** was fully converted. The reaction solution was filtered. The filtrate was collected, concentrated, and then separated by column chromatography (PE:EA = 20:1), to obtain 2.3 g of yellow liquid (72% yield).

### Step 3: rac-(2R,3S)-3-(3,4-difluoro-2-methoxyphenyl)-4-methyl-5-(trifluoromethyl)-2,3-dihydrofuran-2-carboxamide (25c)

The compound **25b** (5 g, 13.6 mmol, 1.0 eq) was dissolved in ammonia/methanol (7 M, 15 mL). The reaction solution reacted at 80°C for 16 h. The reaction was monitored by LCMS until the raw materials were fully converted. The reaction solution was concentrated. The residue was purified by silica gel column chromatography (dichloromethane/methanol = 20/1) to obtain the compound **25c** (3.8 g, 83% yield), which was a white solid. LCMS: [M+H]⁺ = 338.0.

### Step 4: rac-(1R,3R,4R,5R)-4-(3,4-difluoro-2-methoxyphenyl)-5-methyl-1-(trifluoromethyl)-6-(trimethylsilyl)-2-oxabicyclo[3.2.0]heptane-3-carboxamide (25d)

The compound **25c** (2.0 g, 5.9 mmol, 1.0 eq) was dissolved in dichloroethane (50 mL), and the compound trimethyl(vinyl)silane (11.8 g, 11.8 mmol, 20.0 eq) was added. The reaction solution was irradiated under a mercury lamp to react at 25°C for 16 h. The reaction mixture was concentrated. The residue was purified by preparative separation (0.01% FA/acetonitrile = 1/1) to obtain the compound **25d** (600 mg, 23% yield), which was a yellow oily substance. LCMS: [M+H]⁺ = 438.2.

### Step 5: rac-(1R,3R,4S,5R)-4-(3,4-difluoro-2-methoxyphenyl)-5-methyl-1-trifluoromethyl-2-oxabicyclo[3.2.0]heptane-3-carboxamide (25e)

The compound **25d** (500 mg, 3.0 mmol, 1.0 eq) was dissolved in dimethyl sulfoxide (20 mL), and tetrabutylammonium fluoride trihydrate (6.4 g, 22.8 mmol, 20 eq) was added. The reaction solution reacted at 100°C for 16 h. The reaction mixture was concentrated. The residue was purified by preparative separation (0.01% FA/acetonitrile = 3/2) to obtain the compound **25e** (170 mg, 40% yield), which was a yellow oily substance. LCMS: [M+H]⁺ = 366.1.

### Step 6: rac-(1R,3R,4S,5R)-4-(3,4-difluoro-2-methoxyphenyl)-5-methyl-1-trifluoromethyl-2-oxabicyclo[3.2.0]heptane-3-carboxylic acid (25f)

The compound **25d** (170 mg, 0.387 mmol, 1.0 eq) was dissolved in ethanol/water (3 mL + 1 mL), and potassium hydroxide (217 mg, 3.87 mmol, 10 eq) was added. The reaction solution reacted at 100°C for 4 h. The reaction was monitored by LCMS until the reaction was completed. The reaction solution was adjusted with 1 M dilute hydrochloric acid to pH 5-6, and subjected to extraction with ethyl acetate (10 mL × 3). The organic phase was collected. The organic phase was washed with saturated saline, dried over anhydrous sodium sulfate, and then concentrated, to obtain the compound **25f** (100 mg, 70% yield), which was a yellow oily substance. LCMS: [M+H]⁺ = 367.1.

### Step 7: rac-(1R,3R,4S,5R)-4-(3,4-difluoro-2-methoxyphenyl-d3)-5-methyl-1-trifluoromethyl-2-oxabicyclo[3.2.0]heptane-3-carboxylic acid (25g)

The intermediate **25g** was prepared according to a method similar to that for the intermediate **1d**. LCMS: m/z (254 nm): 368.3 [M-H]⁻.

### Preparation of intermediate 33b: 5-bromo-2-fluoro-N-(thiazol-4-yl)benzenesulfonamide

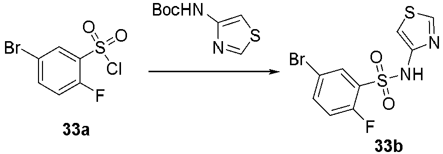

4-bromo-2-fluorobenzenesulfonyl chloride **33a** (200 mg, 1.0 eq) was dissolved in THF (5 mL) and cooled down to 0°C, and NaHMDS (0.9 mL, 1.0 M, 1.2 eq) was added dropwise under nitrogen protection. After 1 h, a thiazole-4-tert-butyl carbamate (161 mg, 1.1 eq) in THF (1 mL) solution was added. After the mixture was warmed slowly to room temperature, a saturated ammonium chloride solution was added. Extraction was carried out with ethyl acetate (20 mL ×3). The organic phases were combined, washed with saline, dried over sodium sulfate, filtered, spin-dried, and purified by silica gel column chromatography (petroleum ether/ethyl acetate = 3/1), to obtain 5-bromo-2-fluoro-N-(thiazol-4-yl)benzenesulfonamide **33b** (150 mg, 60% yield), which was a white solid. LCMS: [M+H]⁺ = 336.8, 338.8.

### Preparation of intermediate 34c:

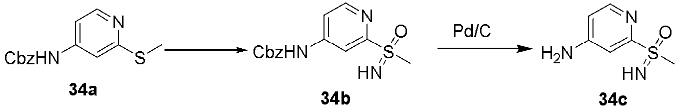

### Step 1: (2-(S-methylsulfoximine)pyridin-4-yl)benzyl carbamate (34b)

**34a** (2.2 g, 8.0 mmol, 1.0 eq) was weighed and added in a reaction flask, and (diacetoxyiodo)benzene (8.3 g, 25.7 mmol, 3.2 eq), aminocarboxamide (4.3 g, 56.2 mmol, 7.0 eq), dichloromethane (150 mL), and methanol (150 mL) were added. The mixture reacted at room temperature for 16 h. The reaction was monitored by LCMS until the reaction was completed. The reaction solution was concentrated and then purified by column chromatography (petroleum ether:ethyl acetate = 0-100%) to obtain **34b,** which was a yellow solid (2.3 g, 94% yield). LCMS: [M+H]⁺ = 306.4.

### Step 2: (4-aminopyridin-2-yl)(imino)(methyl)-1-6-sulfonic acid ketone (34c)

The compound **34b** (300 mg, 0.98 mmol, 1.0 eq) was dissolved in methanol (10 mL), and palladium on carbon (314 mg, 2.95 mmol, 3.0 eq) was added. The reaction solution reacted in hydrogen at 25°C for 16 h. The reaction was monitored by LCMS until the reaction was completed. The reaction solution was filtered. The filtrate was collected and concentrated to obtain a residue. The residue was purified by silica gel column chromatography (dichloromethane/methanol = 10/1) to obtain the compound **34c** (100 mg, 59% yield), which was a white solid. LCMS: [M+H]⁺ = 172.1.

### Preparation of intermediate 35b:

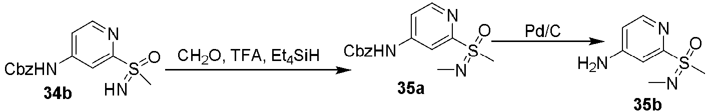

### Step 1: (2-(N,S-dimethylsulfoximine)pyridin-4-yl)benzyl carbamate (35a)

The compound **34b** (300 mg, 0.98 mmol, 1.0 eq) was dissolved in acetonitrile (20 mL), and tetraethylsilane (283 mg, 1.96 mmol, 2.0 eq), formaldehyde (294 mg, 4.91 mmol, 5.0 eq), and trifluoroacetic acid (233 mg, 2.94 mmol, 3.0 eq) were added. The reaction solution reacted at 25°C for 16 h. The reaction was monitored by LCMS until the reaction was completed. The reaction solution was concentrated to obtain a residue. The residue was purified by silica gel column chromatography (dichloromethane/methanol = 30/1) to obtain the compound **35a** (300 mg, 95% yield), which was a colorless oily substance. LCMS: [M+H]⁺ = 320.1.

### Step 2: (4-aminopyridin-2-yl)(methyl)(methylimino)-1-6-sulfonic acid ketone (35b)

The compound **35a** (300 mg, 0.94 mmol, 1.0 eq) was dissolved in methanol (10 mL), and palladium on carbon (300 mg, 2.82 mmol, 3.0 eq) was added. The reaction solution reacted in hydrogen at 25°C for 16 h. The reaction was monitored by LCMS until the reaction was completed. The reaction solution was filtered. The filtrate was collected and concentrated to obtain a residue. The residue was purified by silica gel column chromatography (dichloromethane/methanol = 10/1) to obtain the compound **35b** (150 mg, 86% yield), which was a white solid. LCMS: [M+H]⁺ = 186.1.

### Preparation of intermediate 42b:

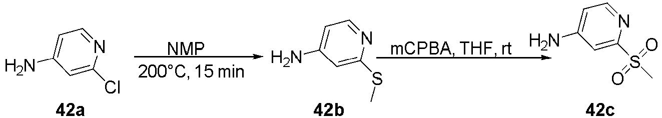

### Step 1: 2-(methylthio)pyridin-4-amine (42b)

Sodium thiomethoxide (1.9 g, 27.0 mmol, 1.7 eq) was dissolved in 1-methylpyrrolidone (10 mL), and 2-chloropyridin-4-amine **42a** (2.0 g, 16.0 mmol, 1.0 eq) was added. The mixture was sealed in a container and heated in a microwave oven to 200°C to react for 15 min, and then purified by rapid chromatography on silica gel through elution with 8% methanol/dichloromethane to obtain 2-(methylthio)pyridin-4-amine **42b** (1.5 g, 55% yield), which was a yellow solid. LCMS: [M+H]⁺ = 141.0.

### Step 2: 2-(methylsulfonyl)pyridin-4-amine (42c)

2-(methylthio)pyridin-4-amine (100 mg, 0.7 mmol, 1.0 eq) was dissolved in tetrahydrofuran (5 mL), and meta-chloroperbenzoic acid (369 mg, 2.1 mmol, 3.0 eq) was added. The mixture was stirred at 25°C for 16 h. After the reaction, the mixture was concentrated under reduced pressure to obtain a mixture. The mixture was purified by column chromatography (dichloromethane/methanol = 10/1) to obtain 2-(methylsulfonyl)pyridin-4-amine **42c** (30 mg, 22% yield), which was a yellow solid. LCMS: [M+H]⁺ = 173.0.

### Preparation of intermediate 43b: (4-aminopyridin-2-yl)dimethylphosphine oxide

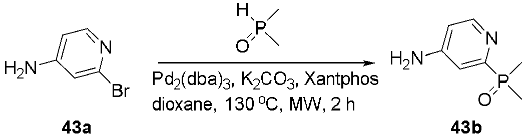

In a microwave tube, the compound 2-bromopyridin-4-amine **43a** (200 mg, 1.16 mmol, 1.0 eq) was dissolved in 1,4-dioxane (3 mL), and the compound dimethylphosphine oxide (180 mg, 2.31 mmol, 2.0 eq), 4,5-bis(diphenylphosphino)-9,9-dimethylxanthene (67 mg, 0.12 mmol, 0.1 eq), tris(dibenzylideneacetone)palladium (106 mg, 0.12 mmol, 0.1 eq), and potassium carbonate (320 mg, 2.3 mmol, 2.0 eq) were added. The reaction solution underwent a microwave reaction at 130°C in a nitrogen atmosphere for 2 h. The reaction was monitored by LCMS until the raw materials were fully converted. The reaction solution was concentrated. The residue was purified by silica gel column chromatography (petroleum ether/ethyl acetate = 8/1) to obtain the compound (4-aminopyridin-2-yl)dimethylphosphine oxide **43b** (150 mg, 76% yield), which was a yellow solid.

### Preparation of intermediate 44d:

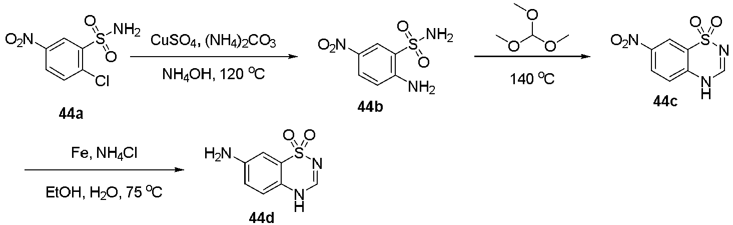

### Step 1: 2-amino-5-nitrobenzenesulfonamide (44b)

**44a** (2.0 g, 8.45 mmol, 1.0 eq), ammonium carbonate (1.62 g, 16.9 mmol, 2.0 eq), copper sulfate (405 mg, 2.54 mmol, 0.3 eq), and aqueous ammonia (10 mL) were added in a reaction flask and stirred at 120°C for 6 h. The reaction was monitored by TLC until the raw materials were fully converted. The reaction solution was cooled down to room temperature and poured into water to precipitate a solid. The mixture was filtered. The filter cake was washed with water and dried to obtain the compound **44b** (1.3 g, 71% yield), which was a light yellow solid. LCMS: 218.1 [M+H]⁺.

### Step 2: 7-nitro-4H-benzo[e][1,2,4]thiadiazine-1,1-dioxide (44c)

**44b** (600 mg, 2.76 mmol, 1.0 eq) and trimethyl orthoformate (10 mL) were added in a reaction flask and stirred at 140°C for 8 h. The reaction was monitored by TLC until the raw materials were fully converted. The reaction solution was cooled down to room temperature and poured into water to precipitate a solid. The mixture was filtered. The filter cake was washed with water and dried to obtain the compound **44c** (230 mg, 37% yield), which was a pale white solid. LCMS: 228.1 [M+H]⁺.

### Step 3: 7-amino-4H-benzo[e][1,2,4]thiadiazine-1,1-dioxide (44d)

**44c** (200 mg, 0.88 mmol, 1.0 eq), iron powder (246 mg, 4.4 mmol, 5.0 eq), ammonium chloride (141 mg, 2.64 mmol, 3.0 eq), ethanol (5 mL), and water (2 mL) were added in a reaction flask and stirred at 75°C for 2 h. The reaction was monitored by LCMS until the raw materials were fully converted. The reaction solution was filtered with diatomaceous earth while hot. The filtrate was concentrated and separated by column chromatography (MeOH/DCM, 0-10%), to obtain the compound **44d** (82 mg, 47% yield), which was a light yellow solid. LCMS: 198.2 [M+H]⁺.

### Preparation of intermediate 54b:

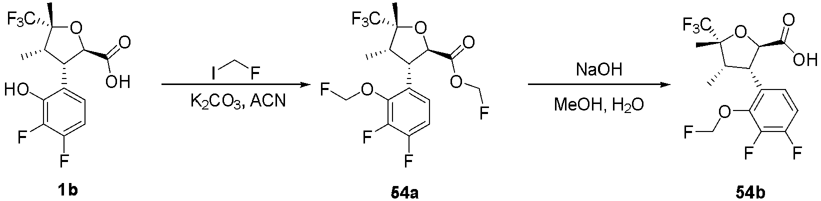

### Step 1: Synthesis of fluoromethyl (2R,3S,4S,5R)-3-{(3,4-difluoro-2-[(fluoromethyl)oxy]phenyl) }-4,5-dimethyl-5-(trifluoromethyl)tetrahydrofuran-2-carboxylate (54a)

**1b** (100 mg, 0.29 mmol, 1.0 eq), fluoroiodomethane (0.23 g, 1.5 mmol, 5.0 eq), potassium carbonate (0.38 g, 2.9 mmol, 10.0 eq), and acetonitrile (10 mL) were added into a reaction flask to react at room temperature for 18 h. The reaction was monitored by LCMS until the raw materials were fully converted (no MS response for the product). The reaction solution with water (10 mL) added was subjected to extraction with ethyl acetate (10 mL × 3). The organic phases were combined, dried over anhydrous sodium sulfate, filtered, and concentrated, to obtain the crude compound **54a** (0.145 g, crude), which was a yellow liquid.

### Step 2: Synthesis of compound (2R,3S,4S,SR)-3-{(3,4-difluoro-2-[(fluoromethyl)oxy]phenyl) }-4,5-dimethyl-5-(trifluoromethyl)tetrahydrofuran-2-carboxylic acid (54b)

The compound **54a** (0.145 g, 0.29 mmol, 1.0 eq), sodium hydroxide (0.06 g, 1.5 mmol, 5.0 eq), water (10 mL), and methanol (10 mL) were added into a reaction flask to react at room temperature for 16 h. The reaction was monitored by LCMS until the raw materials were fully converted. The reaction solution was concentrated and adjusted with hydrochloric acid (2 M) to pH = 1, and then water (10 mL) was added. Then, the reaction solution was subjected to extraction with ethyl acetate (10 mL × 3). The organic phases were combined, dried over anhydrous sodium sulfate, filtered, concentrated, and separated by column chromatography (EA/PE, 0-100%), to obtain the compound **54b** (110 mg, 100% yield), which was a yellow liquid. LCMS: 371.0 [M-H]⁻.

### Preparation of intermediate 55b:

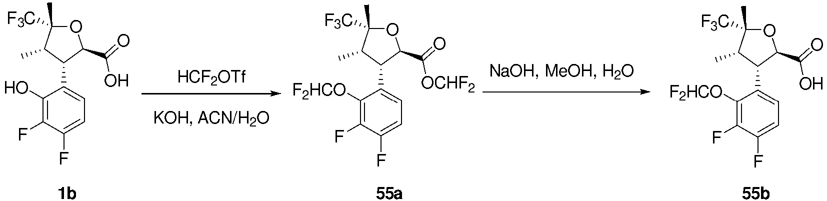

### Step 1: difluoromethyl (2R,3S,4S,5R)-3-(3,4-difluoro-2-difluoromethoxyphenyl)-4,5-dimethyl-5-(trifluoromethyl)tetrahydrofuran-2-carboxylate (55a)

**1b** (50 mg, 0.15 mmol, 1.0 eq) and acetonitrile (5 mL) were added into a single-necked reaction flask, a potassium hydroxide (99 mg, 1.8 mmol, 12.0 eq) in water (11 mL) solution was added, and difluoromethyl trifluoromethanesulfonate (88 mg, 0.44 mmol, 3.0 eq) was added, to react at room temperature for 30 min. The reaction was monitored by LCMS until most of the raw materials were fully converted, and then the reaction was terminated. The reaction system was adjusted with 1 N hydrochloric acid to pH = 5, and subjected to extraction with ethyl acetate. The ethyl acetate layer was collected, concentrated, and then subjected to column chromatography (PE:EA = 0-100%), to obtain the compound **55a** (30 mg). LCMS: m/z (254 nm): 441.0 [M+H]⁺.

### Step 2: (2R,3S,4S,5R)-3-(3,4-difluoro-2-difluoromethoxyphenyl)-4,5-dimethyl-5-(trifluoromethyl)tetrahydrofuran-2-carboxylic acid (55b)

The intermediate **55b** was prepared according to a synthesis method similar to that for the intermediate **1d**. LCMS: m/z (254 nm): 389.1 [M-H]⁻.

### Example 1: (2R,35,4S,5R)-3-(3,4-difluoro-2-methoxyphenyl-d3)-4,5-dimethyl-N-(3-oxoisoindolin-5-yl)-5-(trifluoromethyl)tetrahydrofuran-2-carboxamide

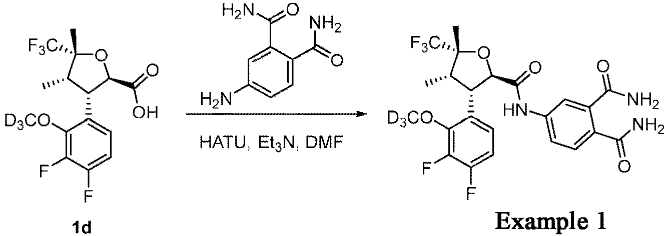

**1d** (0.16 g, 0.45 mmol, 1.0 eq), 4-aminobenzene-1,2-dicarboxamide (0.12 g, 0.67 mmol, 1.5 eq), HATU (0.255 g, 0.67 mmol, 1.5 eq), triethylamine (0.113 g, 1.12 mmol, 2.5 eq), and DMF (2 mL) were added into a single-necked reaction flask to react at 30°C for 16 h. The reaction was monitored by LCMS until the reaction was completed. Then, extraction was carried out with water and ethyl acetate. An ethyl acetate layer was collected, concentrated, and subjected to column chromatography (MeOH/DCM = 0-20%) through elution, to obtain **Example** 1 (0.22 g, 94% yield). ¹H NMR (400 MHz, CD₃OD) δ 7.82 (d, *J =* 2.0 Hz, 1H), 7.73 (dd, *J* = 8.4, 2.1 Hz, 1H), 7.59 (d, *J =* 8.4 Hz, 1H), 7.15-7.09 (m, 1H), 6.97 (dd, *J* = 17.0, 9.3 Hz,1H), 5.06 (d, *J =* 10.5 Hz, 1H), 4.34-4.27 (m, 1H), 2.78 (dd, *J =* 15.4, 7.8 Hz, 1H), 1.66 (s, 3H), 0.81 (d, *J* = 7.4 Hz, 3H).

### Example 54: 4-((2R,3S,4S,5R)-3-(3,4-difluoro-2-(fluoromethoxy)phenyl)-4,5-dimethyl-5-(trifluoromethyl)tetrahydrofuran-2-carboxamide)phthalamide

Example 54 was prepared according to a synthesis method similar to that in Example 1 with the intermediate **54b** that replaced **1d**. ESI-MS m/z = 534.1 (M+1)⁺. ¹H NMR (400 MHz, DMSO-d6) δ 10.43 (s, 1H), 7.74-7.66 (m, 4H), 7.49 (d, *J* = 8.3 Hz, 1H), 7.37-7.24 (m, 4H), 5.90 (dd, *J* = 5.9, 2.5 Hz, 1H), 5.76 (dd, *J =* 6.2, 2.5 Hz, 1H), 5.11 (d, *J =* 10.4 Hz, 1H), 4.32 (dd, *J =* 10.3, 7.7 Hz, 1H), 2.84-2.75 (m, 1H), 1.59 (s, 3H), 0.74 (d, *J =* 6.0 Hz, 3H).

### Example 55: 4-((2R,3S,4S,5R)-3-(3,4-difluoro-2-(difluoromethoxy)phenyl)-4,5-dimethyl-5-(trifluoromethyl)tetrahydrofuran-2-carboxamide)phthalamide

Example 55 was prepared according to a synthesis method similar to that in Example 1 with the intermediate **55b** that replaced **1d**. ESI-MS m/z = 552.1 (M+1)⁺. ¹H NMR (400 MHz, DMSO-d6) δ 10.43 (s, 1H), 7.73-7.66 (m, 4H), 7.53-7.08 (m, 6H), 5.13 (d, *J =* 10.3 Hz, 1H), 4.27 (dd, *J =* 10.1, 7.7 Hz, 1H), 2.79-2.72 (m, 1H), 1.59 (s, 3H), 0.76 (d, *J =* 6.1 Hz, 3H).

**Table 1: The following compounds were prepared with corresponding reagents as raw materials according to operations similar to the operations described in Example 1.**

| | | |
|---|---|---|
| 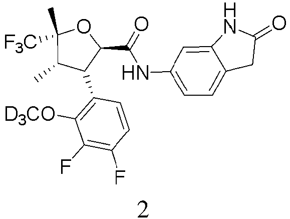 | 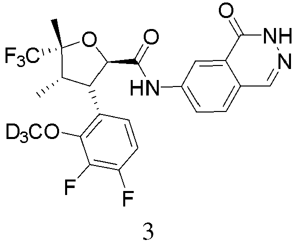 | 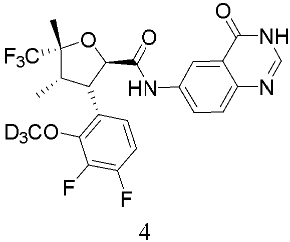 |
| ESI-MS m/z: 488.1 (M+1)⁺ | ESI-MS m/z: 501.1 (M+1)⁺ | ESI-MS m/z: 501.1 (M+1)⁺ |
| 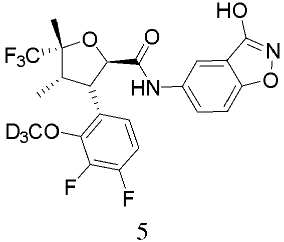 | 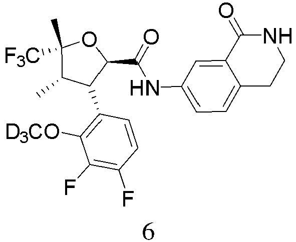 | 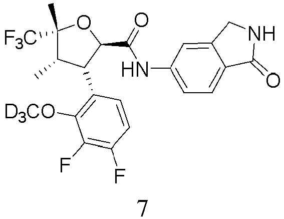 |
| ESI-MS m/z: 490.1 (M+1)⁺ | ESI-MS m/z: 502.1 (M+1)⁺ | ESI-MS m/z: 488.1 (M+1)⁺ |
| 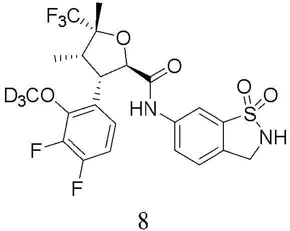 | 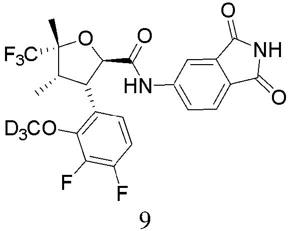 | 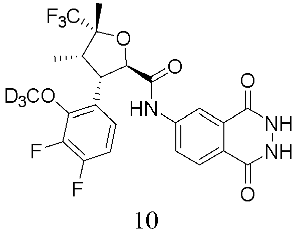 |
| ESI-MS m/z: 524.1 (M+1)⁺ | ESI-MS m/z: 502.1 (M+1)⁺ | ESI-MS m/z: 517.1 (M+1)⁺ |
| 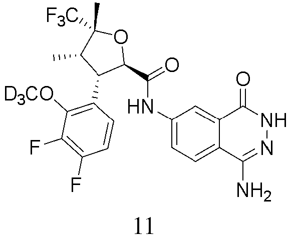 | 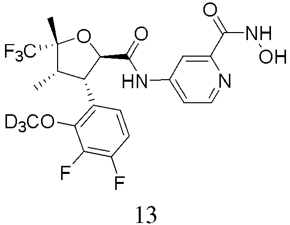 | 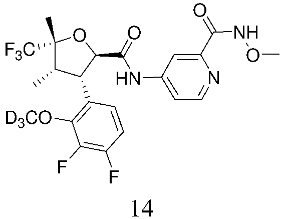 |
| ESI-MS m/z: 516.2 (M+1)⁺ | ESI-MS m/z: 493.2 (M+1)⁺ | ESI-MS m/z: 507.2 (M+1)⁺ |
| 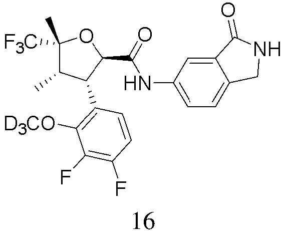 | 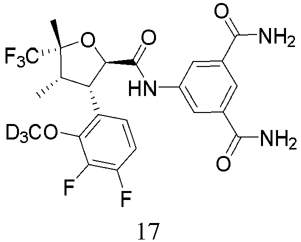 | 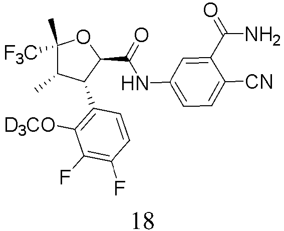 |
| ESI-MS m/z: 488.2 (M+1)⁺ | ESI-MS m/z: 519.2 (M+1)⁺ | ESI-MS m/z: 501.1 (M+1)⁺ |
| 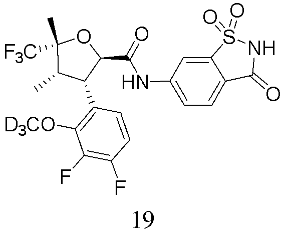 | 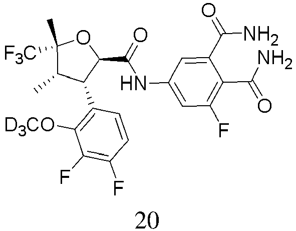 | 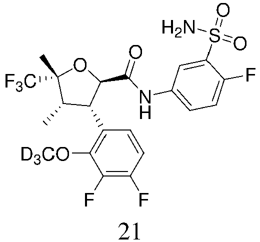 |
| ESI-MS m/z: 538.2 (M+1)⁺ | ESI-MS m/z: 537.1 (M+1)⁺ | ESI-MS m/z: 530.1 (M+1)⁺ |
| 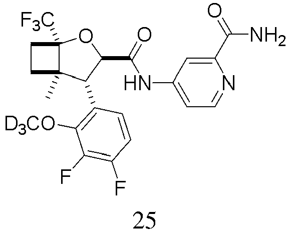 | 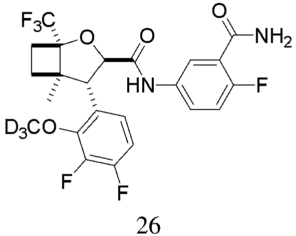 | 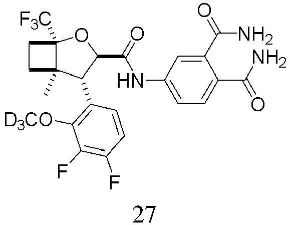 |
| ESI-MS m/z: 489.2 (M+1)⁺ | ESI-MS m/z: 506.2 (M+1)⁺ | ESI-MS m/z: 531.2 (M+1)⁺ |
| 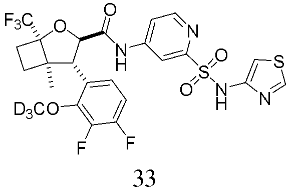 | 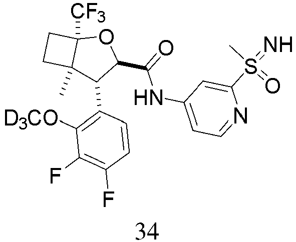 | 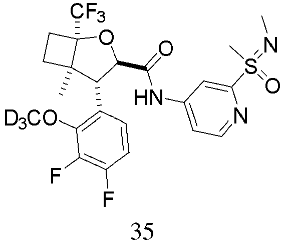 |
| ESI-MS m/z: 608.1 (M+1)⁺ | ESI-MS m/z: 523.1 (M+1)⁺ | ESI-MS m/z: 537.2 (M+1)⁺ |
| 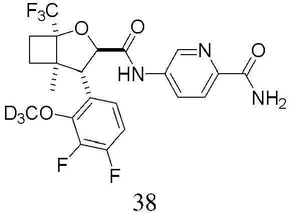 | 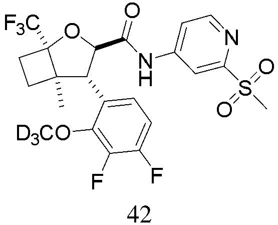 | 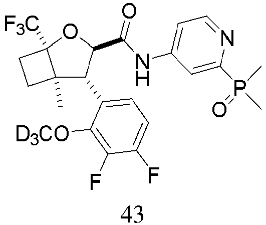 |
| ESI-MS m/z: 489.2 (M+1)⁺ | ESI-MS m/z: 524.1 (M+1)⁺ | ESI-MS m/z: 522.2 (M+1)⁺ |
| 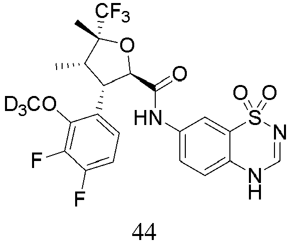 | 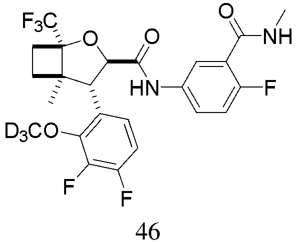 | 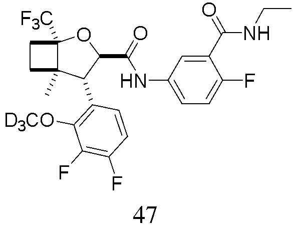 |
| ESI-MS m/z: 537.1 (M+1)⁺ | ESI-MS m/z: 520.2 (M+1)⁺ | ESI-MS m/z: 534.2 (M+1)⁺ |
| 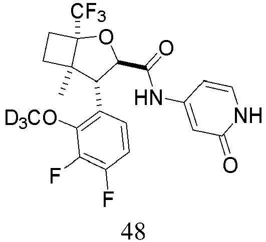 | 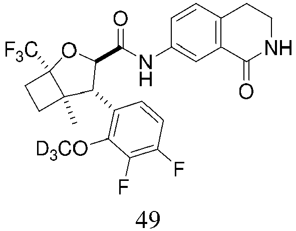 | 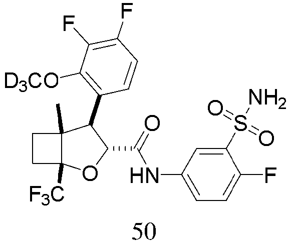 |
| ESI-MS m/z: 462.2 (M+1)⁺ | ESI-MS m/z: 514.2 (M+1)⁺ | ESI-MS m/z: 542.1 (M+1)⁺ |
| 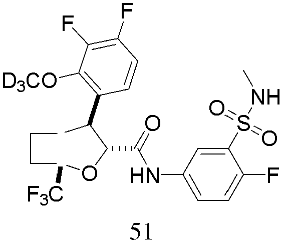 | 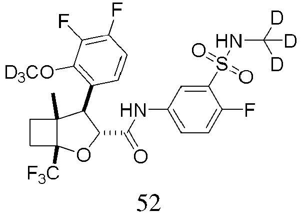 | 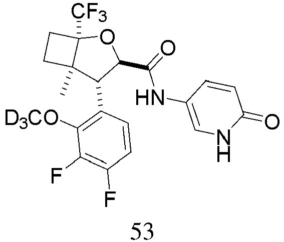 |
| ESI-MS m/z: 556.2 (M+1)⁺ | ESI-MS m/z: 559.2 (M+1)⁺ | ESI-MS m/z: 462.1 (M+1)⁺ |
| 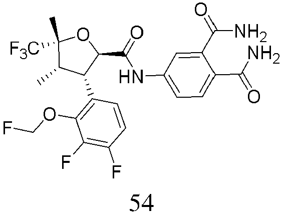 | 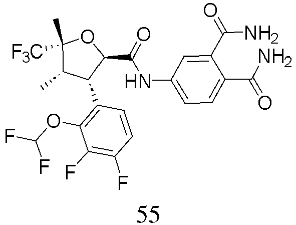 | 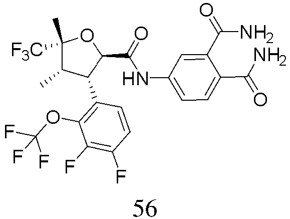 |
| ESI-MS m/z: 534.1 (M+1)⁺ | ESI-MS m/z: 552.1 (M+1)⁺ | ESI-MS m/z: 570.1 (M+1)⁺ |
| 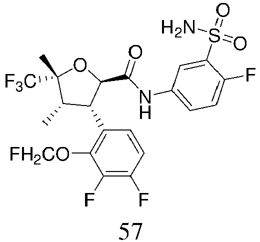 | 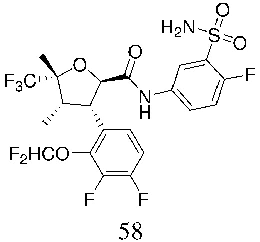 | 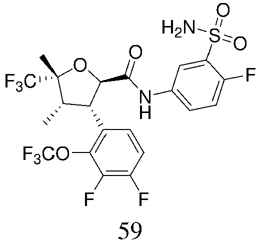 |
| ESI-MS m/z: 545.0 (M+1)⁺ | ESI-MS m/z: 563.0 (M+1)⁺ | ESI-MS m/z: 581.1 (M+1)⁺ |
| 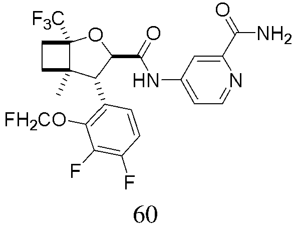 | 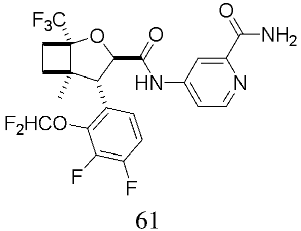 | 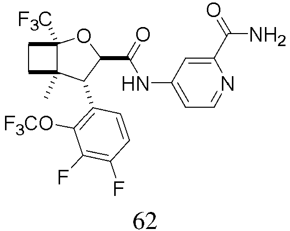 |
| ESI-MS m/z: 504.1 (M+1)⁺ | ESI-MS m/z: 522.1 (M+1)⁺ | ESI-MS m/z: 540.1 (M+1)⁺ |
| 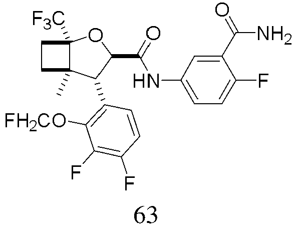 | 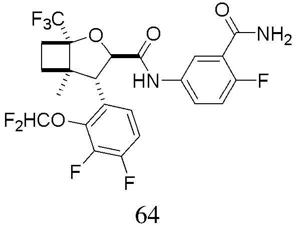 | 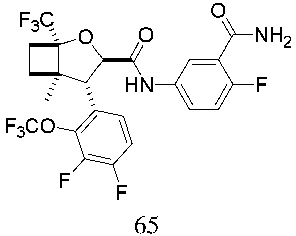 |
| ESI-MS m/z: 521.1 (M+1)⁺ | ESI-MS m/z: 539.1 (M+1)⁺ | ESI-MS m/z: 557.1 (M+1)⁺ |
| 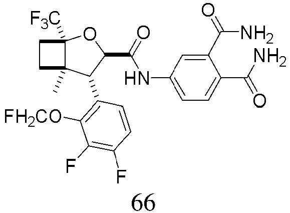 | 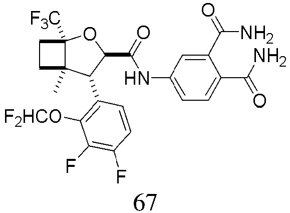 | 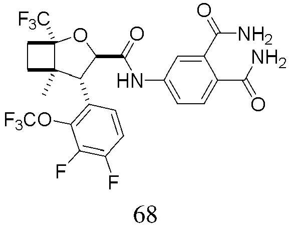 |
| ESI-MS m/z: 546.1 (M+1)⁺ | ESI-MS m/z: 564.1 (M+1)⁺ | ESI-MS m/z: 582.1 (M+1)⁺ |
| 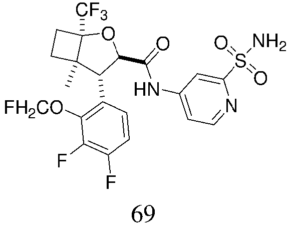 | 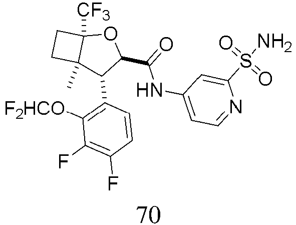 | 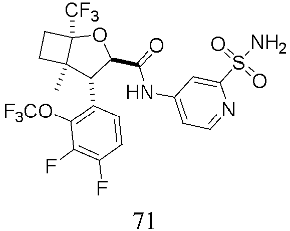 |
| ESI-MS m/z: 540.1 (M+1)⁺ | ESI-MS m/z: 558.1 (M+1)⁺ | ESI-MS m/z: 576.1 (M+1)⁺ |
| 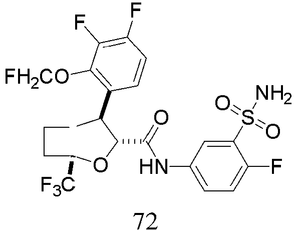 | 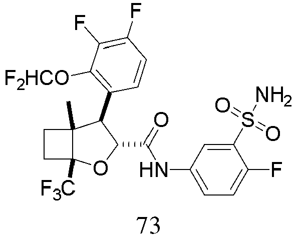 | 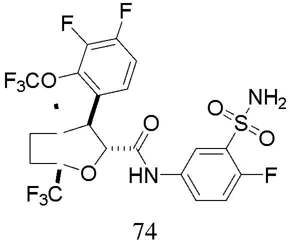 |
| ESI-MS m/z: 557.1 (M+1)⁺ | ESI-MS m/z: 575.1 (M+1)⁺ | ESI-MS m/z: 593.1 (M+1)⁺ |
| 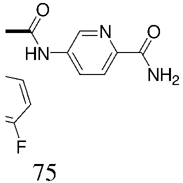 | 76 | 77 |
| ESI-MS m/z: 504.1 (M+1)⁺ | ESI-MS m/z: 522.1 (M+1)⁺ | ESI-MS m/z: 540.1 (M+1)⁺ |

### Example 12:

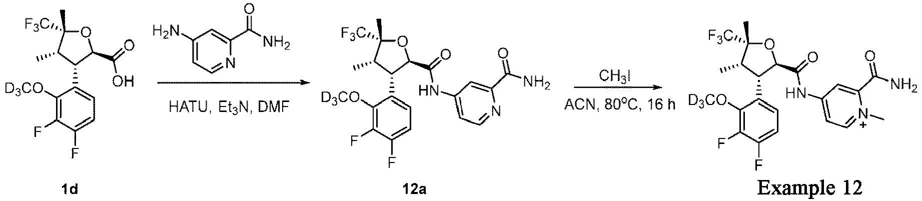

### Step 1: 4-({[(2R,3S,4S,5R)-3-{3,4-difluoro-2-[(methyl-d3)oxy]phenyl}-4,5-dimethyl-5-(trifluoromethyl)tetrahydrofuran-2-yl]carbonyl}amino)pyridine-2-carboxamide (12a)

The intermediate **12a** was prepared according to operations similar to the operations described in Example 1. LCMS: [M+H]⁺ = 477.2.

### Step 2: 2-formamido-4-({[(2R,3S,4S,5R)-3-{ 3,4-difluoro-2-[(methyl-d3)oxy]phenyl }-4,5-dimethyl-5-(trifluoromethyl)tetrahydrofuran-2-yl]carbonyl}amino-1-methylpyridine-1-cation (Example 12)

The compound **12a** (42 mg, 0.08 mmol, 1.0 eq), iodomethane (1 mL), and acetonitrile (2 mL) were added sequentially in a microwave tube. The reaction solution reacted at 80°C under nitrogen protection for 16 h. The reaction was monitored by LCMS until the reaction was completed. The reaction mixture was concentrated. The residue was purified by preparative separation (0.01% aqueous ammonia) to obtain the compound **12** (12 mg, 27% yield). LCMS: [M+H]⁺ = 492.2.

### Example 15: (2R,3S,4S,5R)-N-[2-(aminothiomethylidene)pyridin-4-yl]-3-{3,4-difluoro-2-[(methyl-d3)oxy]phenyl}-4,5-dimethyl-5-(trifluoromethyl)tetrahydrofuran-2-carboxamide

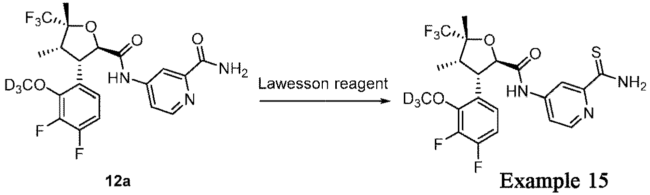

The compound **12a** (30 mg, 0.06 mmol, 1.0 eq) was dissolved in toluene (20 mL), and the Lawesson reagent (26 mg, 0.06 mmol, 1.0 eq) was added. The mixture was heated to 110°C to react for 24 h. After the reaction, the reaction solution was concentrated to remove the solvent, and prepared by thin-layer chromatography, to obtain **Example 15** (15 mg, 48% yield). LCMS: [M+H]⁺ = 493.2.

### Example 22:

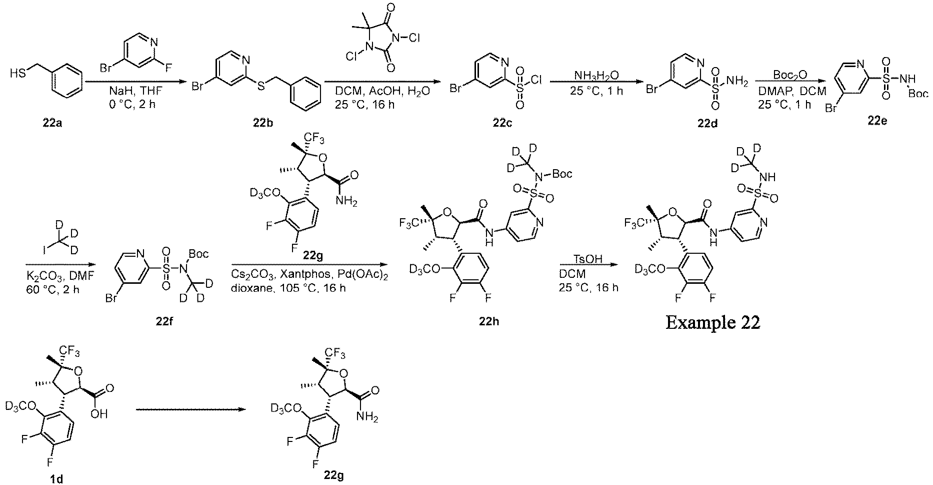

### Step 1: 2-(benzylthio)-4-bromopyridine (22b)

The compound benzyl mercaptan **22a** (2.0 g, 16.1 mmol, 1.0 eq) was dissolved in anhydrous tetrahydrofuran (30 mL), and sodium hydride (640 mg, 16.1 mmol, 1.0 eq) was added at 0°C under nitrogen protection. The reaction solution was stirred at 0°C for 30 min. A solution of the compound 4-bromo-2-fluoropyridine (2.8 g, 16.1 mmol, 1.0 eq) in anhydrous tetrahydrofuran (10 mL) was added dropwise at this temperature. Then, the mixture was stirred at 0°C for 1.5 h. The reaction was monitored by LCMS until the raw materials were fully converted. The reaction solution with water (50 mL) added was subjected to extraction with ethyl acetate (20 mL × 3), washed with saturated saline, dried over anhydrous sodium sulfate, and then concentrated. The residue was purified by silica gel column chromatography (petroleum ether/ethyl acetate = 50/1) to obtain the compound 2-(benzylthio)-4-bromopyridine **22b** (4.3 g, 95% yield), which was a pink oily substance. LCMS: [M+H]⁺ = 280.0.

### Step 2: 4-bromopyridine-2-sulfonyl chloride (22c)

The compound 2-(benzylthio)-4-bromopyridine **22b** (4.3 g, 15.3 mmol, 1.0 eq) was dissolved in dichloromethane, acetic acid, and water in 7:1:2 (80 mL), and 1,3-dichloro-5,5-dimethylimidazolidine-2,4-dione (1.1 g, 7.2 mmol, 0.7 eq) was added. The reaction solution reacted at 25°C under nitrogen protection for 16 h. The reaction was monitored by TLC until the raw materials were fully converted. The reaction solution with water (20 mL) added was subjected to extraction with dichloromethane (20 mL × 3), washed with saturated saline, dried over anhydrous sodium sulfate, concentrated, diluted with dichloromethane, and filtered. The filtrate was spin-dried to obtain the crude compound 4-bromopyridine-2-sulfonyl chloride **22c** (4.5 g, 92% yield), which was a yellow oily substance.

### Step 3: 4-bromopyridine-2-sulfonamide (22d)

Aqueous ammonia (10 mL) was added to the compound 4-bromopyridine-2-sulfonyl chloride **22c** (3.0 g, 9.4 mmol, 1.0 eq). The reaction solution reacted at 25°C for 1 h. The reaction was monitored by LCMS until the raw materials were fully converted. The reaction solution was concentrated. The residue was purified by silica gel column chromatography (petroleum ether/ethyl acetate = 1/1) to obtain the compound 4-bromopyridine-2-sulfonamide **22d** (1.7 g, 77% yield), which was a gray solid. LCMS: [M+H]⁺ = 238.9.

### Step 4: ((4-bromopyridin-2-yl)sulfonyl)tert-butyl carbamate (22e)

The compound 4-bromopyridine-2-sulfonamide **22d** (500 mg, 2.1 mmol, 1.0 eq) was dissolved in dichloromethane (10 mL), and triethylamine (213 mg, 2.1 mmol, 1.0 eq), 4-dimethylaminopyridine (13 mg, 0.10 mmol, 0.05 eq), and di-tert-butyl dicarbonate (483 mg, 2.2 mmol, 1.05 eq) were added. The reaction solution reacted at 25°C for 1 h. The reaction was monitored by LCMS until the raw materials were fully converted. The reaction solution was concentrated. The residue was purified by silica gel column chromatography (dichloromethane/methanol = 10/1) to obtain the compound ((4-bromopyridin-2-yl)sulfonyl)tert-butyl carbamate **22e** (611 mg, 85% yield), which was a yellow oily substance. LCMS: [M+Na]⁺ = 359.0

### Step 5: ((4-bromopyridin-2-yl)sulfonyl)(methyl-d3)tert-butyl carbamate (22f)

The compound ((4-bromopyridin-2-yl)sulfonyl)tert-butyl carbamate **22e** (200 mg, 0.59 mmol, 1.0 eq) was dissolved in anhydrous N,N-dimethylformamide (5 mL), and potassium carbonate (123 mg, 0.88 mmol, 1.5 eq) and deuterated iodomethane (94 mg, 0.65 mmol, 1.1 eq) were added. The reaction solution reacted at 60°C for 2 h. The reaction was monitored by LCMS until the raw materials were fully converted. The reaction solution with water (10 mL) added was subjected to extraction with ethyl acetate (10 mL × 3), washed with water and saturated saline sequentially, dried over anhydrous sodium sulfate, and then concentrated. The residue was purified by silica gel column chromatography (petroleum ether/ethyl acetate = 10/1) to obtain **22f** (130 mg, 62% yield), which was a white solid. LCMS: [M+Na]⁺ = 376.0

### Step 6: (2R,3S,4S,5R)-3-(3,4-difluoro-2-methoxyphenyl)-4,5-dimethyl-5-(trifluoromethyl)tetrahydrofuran-2-carboxamide (22g)

The intermediate **22g** was obtained according to a method similar to that in Example 1 with ammonium chloride that replaced 4-aminobenzene-1,2-dicarboxamide in Example 1. LCMS: [M+H]⁺ = 357.1.

### Step 7: 2-methylpropan-2-yl ({[4-({[(2R,3S,4S,5R)-3-{3,4-difluoro-2-[(methyl-d3)oxy]phenyl}-4,5-dimethyl-5-(trifluoromethyl)tetrahydrofuran-2-yl]carbonyl}amino)pyridine-2-yl]dioxo-λ⁶-thio}(methyl-d3)amino)methanoate (22h)

The compound **22f** (70 mg, 0.20 mmol, 1.0 eq) was dissolved in dioxane (5 mL), and the compound **22g** (139 mg, 0.24 mmol, 1.2 eq), 4,5-bis(diphenylphosphino)-9,9-dimethylxanthene (23 mg, 0.04 mmol, 0.1 eq), cesium carbonate (128 mg, 0.39 mmol, 2.0 eq), and palladium acetate (3 mg, 0.02 mmol, 0.1 eq) were added. The reaction solution reacted at 105°C under nitrogen protection for 16 h. The reaction was monitored by LCMS until the raw materials were fully converted. The reaction solution was concentrated. The residue was purified by silica gel column chromatography (petroleum ether/ethyl acetate = 5/1) to obtain **22h** (65 mg, 51% yield), which was a yellow oily substance. LCMS: [M+H]⁺ = 630.2.

### Step 8: (2R,3S,4S,5R)-3-{3,4-difluoro-2-[(methyl-d3)oxy]phenyl}-N-(2-{dioxo[(methyl-d3)amino]-λ⁶-thio}pyridin-4-yl)-4,5-dimethyl-5-(trifluoromethyl)tetrahydrofuran-2-carboxamide

**22h** (50 mg, 0.08 mmol, 1.0 eq) was dissolved in dichloromethane (5 mL), and p-toluenesulfonic acid (13.72 mg, 0.08 mmol, 1.0 eq) was added. The reaction solution reacted at 25°C for 16 h. The reaction was monitored by LCMS until the raw materials were fully converted. The reaction mixture was concentrated. The residue was purified by preparative separation (0.05% NH₃) to obtain **Example 22** (10.0 mg, 24% yield). LCMS: [M+H]⁺ = 530.2.

### Example 23:

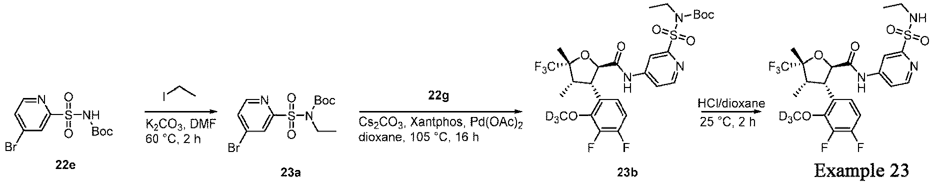

### Step 1: ((4-bromopyridin-2-yl)sulfonyl)(ethyl)tert-butyl carbamate (23a)

The compound ((4-bromopyridin-2-yl)sulfonyl)tert-butyl carbamate **22e** (200 mg, 0.59 mmol, 1.0 eq) was dissolved in anhydrous N,N-dimethylformamide (5 mL), and potassium carbonate (123 mg, 0.88 mmol, 1.5 eq) and iodoethane (101 mg, 0.65 mmol, 1.1 eq) were added. The reaction solution reacted at 60°C for 2 h. The reaction was monitored by LCMS until the raw materials were fully converted. The reaction solution with water (10 mL) added was subjected to extraction with ethyl acetate (10 mL × 3), washed with water and saturated saline sequentially, dried over anhydrous sodium sulfate, and then concentrated. The residue was purified by silica gel column chromatography (petroleum ether/ethyl acetate = 10/1) to obtain the compound ((4-bromopyridin-2-yl)sulfonyl)(ethyl)tert-butyl carbamate **23a** (130 mg, 60% yield), which was a yellow oily substance. LCMS: [M+Na]⁺ = 387.0

### Step 2: 2-methylpropan-2-yl (ethyl{[4-({[(2R,3S,4S,5R)-3-{3,4-difluoro-2-[(methyl-d3)oxy]phenyl }-4,5-dimethyl-5-(trifluoromethyl)tetrahydrofuran-2-yl]carbonyl}amino)pyridine-2-yl]dioxo-λ⁶-thio}amino)methanoate (23b)

The compound **23a** (130 mg, 0.35 mmol, 1.0 eq) was dissolved in dioxane (5 mL), and **22g** (197 mg, 0.39 mmol, 1.2 eq), 4,5-bis(diphenylphosphino)-9,9-dimethylxanthene (41 mg, 0.07 mmol, 0.2 eq), cesium carbonate (231 mg, 0.71 mmol, 2.0 eq), and palladium acetate (6 mg, 0.03 mmol, 0.1 eq) were added. The reaction solution reacted at 105°C under nitrogen protection for 16 h. The reaction was monitored by LCMS until the raw materials were fully converted. The reaction solution was concentrated. The residue was purified by silica gel column chromatography (petroleum ether/ethyl acetate = 4/1) to obtain **23b** (100 mg, 44% yield), which was a yellow oily substance. LCMS: [M+H]⁺ = 641.2.

### Step 3: (2R,3S,4S,5R)-3-{3,4-difluoro-2-[(methyl-d3)oxy]|phenyl}-N-{2-[(ethylamino)dioxo-λ⁶-thio]pyridin-4-yl}-4,5-dimethyl-5-(trifluoromethyl)tetrahydrofuran-2-carboxamide (Example 23)

The compound **23b** (100 mg, 0.16 mmol, 1.0 eq) was dissolved in hydrochloric acid/dioxane (4 M, 30 mL). The reaction solution reacted at 25°C for 2 h. The reaction was monitored by LCMS until the raw materials were fully converted. The reaction mixture was concentrated. The residue was purified by preparative separation (0.01% FA) to obtain **Example 23** (39.5 mg, 47% yield), which was a white solid. LCMS: [M+H]⁺ = 541.2.

### Example 24:

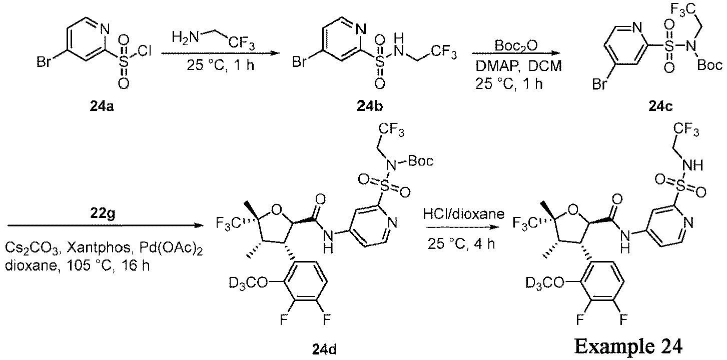

### Step 1: 4-bromo-N-(2,2,2-trifluoroethyl)pyridin-2-sulfonamide (24b)

The compound 2,2,2-trifluoroethan-1-amine (1.2 g, 11.7 mmol, 3.0 eq) was added to the compound 4-bromopyridine-2-sulfonyl chloride **24a** (1.0 g, 3.9 mmol, 1.0 eq). The reaction solution reacted at 25°C for 1 h. The reaction was monitored by LCMS until the raw materials were fully converted. The reaction solution was concentrated. The residue was purified by silica gel column chromatography (petroleum ether/ethyl acetate = 10/1) to obtain the compound 4-bromo-N-(2,2,2-trifluoroethyl)pyridin-2-sulfonamide **24b** (700 mg, 56% yield), which was a white solid. LCMS: [M+H]⁺ = 318.9.

### Step 2: ((4-bromopyridin-2-yl)sulfonyl)(2,2,2-trifluoroethyl)tert-butyl carbamate (24c)

The compound 4-bromo-N-(2,2,2-trifluoroethyl)pyridin-2-sulfonamide **24b** (700 mg, 2.2 mmol, 1.0 eq) was dissolved in dichloromethane (10 mL), and potassium carbonate (604 mg, 4.4 mmol, 2.0 eq), 4-dimethylaminopyridine (27 mg, 0.22 mmol, 0.1 eq), and di-tert-butyl dicarbonate (573 mg, 2.6 mmol, 1.2 eq) were added. The reaction solution reacted at 25°C for 1 h. The reaction was monitored by LCMS until the raw materials were fully converted. The reaction solution was concentrated. The residue was purified by silica gel column chromatography (petroleum ether/ethyl acetate = 10/1) to obtain the compound ((4-bromopyridin-2-yl)sulfonyl)(2,2,2-trifluoroethyl)tert-butyl carbamate **24c** (700 mg, 76% yield), which was a yellow oily substance. LCMS: [M+Na]⁺ = 441.0

### Step 3: 2-methylpropan-2-yl ({[4-({[(2R,3S,4S,5R)-3-{3,4-difluoro-2-[(methyl-d3)oxy]phenyl}-4,5-dimethyl-5-(trifluoromethyl)tetrahydrofuran-2-yl]carbonyl}amino)pyridine-2-yl]dioxo-λ⁶-thio}(2,2,2-trifluoroethyl)amino)methanoate (24d)

The compound **24c** (100 mg, 0.24 mmol, 1.0 eq) was dissolved in dioxane (5 mL), and the compound **22g** (154 mg, 0.26 mmol, 1.1 eq), 4,5-bis(diphenylphosphino)-9,9-dimethylxanthene (27 mg, 0.048 mmol, 0.2 eq), cesium carbonate (155 mg, 0.48 mmol, 2.0 eq), and palladium acetate (4 mg, 0.024 mmol, 0.1 eq) were added. The reaction solution reacted at 105°C under nitrogen protection for 16 h. The reaction was monitored by LCMS until the raw materials were fully converted. The reaction solution was concentrated. The residue was purified by silica gel column chromatography (dichloromethane/methanol = 10/1) to obtain the compound **24d** (80 mg, 48% yield), which was a yellow oily substance. LCMS: [M+H]⁺ = 695.2.

### Step 4: (2R,3S,4S,5R)-3-{3,4-difluoro-2-[(methyl-d3)oxy]phenyl}-N-(2-{dioxo[(2,2,2-trifluoroethyl)amino]-λ⁶-thio}pyridin-4-yl)-4,5-dimethyl-5-(trifluoromethyl)tetrahydrofuran-2-carboxamide (Example 24)

The compound **24d** (80 mg, 0.11 mmol, 1.0 eq) was dissolved in hydrochloric acid/dioxane (4 M, 5 mL). The reaction solution reacted at 25°C for 4 h. The reaction was monitored by LCMS until the raw materials were fully converted. The reaction mixture was concentrated. The residue was purified by preparative separation (0.1% FA) to obtain **Example 24** (15.4 mg, 22% yield). LCMS: [M+H]⁺ = 595.1.

### Example 28:

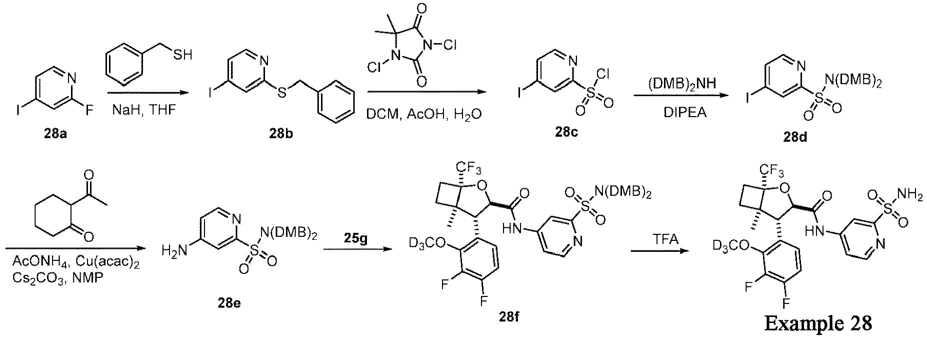

### Step 1: 2-benzylthio-4-iodopyridine (28b)

Benzyl mercaptan (2.9 g) and THF (50 mL) were added in a reaction flask and stirred uniformly, and NaH (848 mg) was added in batches under an ice bath. Then, the mixture was stirred at room temperature for 1 h. **28a** (5 g) was added under an ice bath. Then, the mixture was stirred at room temperature for 3 h. The reaction was monitored by LCMS until the raw materials were fully converted. The reaction solution with water (10 mL) added was subjected to extraction with ethyl acetate (10 mL × 3). The organic phases were combined, dried over anhydrous sodium sulfate, filtered, concentrated, and separated by column chromatography (EA/PE, 0%-10%), to obtain 6.6 g of light red liquid **28b** with a yield of 90.1%. LCMS: m/z (254 nm): 327.9 [M+H]⁺.

### Step 2: 4-iodopyridine-2-sulfonyl chloride (28c)

**28b** (3 g), DCM (30 mL), and DIPEA (3.8 g) were added in a reaction flask, and 1,3-dichloro-5,5-dimethylhydantoin (6.9 g) was added in batches under an ice bath. Then, the mixture was warmed to room temperature and stirred for 4 h. The reaction was monitored by LCMS until the reaction was substantially completed. The reaction solution with water (50 mL) added was subjected to extraction with dichloromethane (20 mL × 3). The organic phases were combined, dried over anhydrous sodium sulfate, filtered, concentrated, and separated by column chromatography (EA/PE, 0%-10%), to obtain 1.4 g of light yellow solid with a yield of 24.2%. LCMS: m/z (254 nm): 303.9 [M+H]⁺.

### Step 3: N,N-bis(2,4-dibenzyl)-4-iodopyridine-2-sulfonamide (28d)

**28c** (800 mg), N-methylpyrrolidone (7 mL), ammonium acetate (1.06 g), copper acetylacetonate (107 mg), Ligand (115 mg), and cesium carbonate (1.34 g) were added in a reaction flask, purged with nitrogen for three times, and stirred at 95°C for 16 h. The reaction solution was cooled down to room temperature. Then, water (20 mL) was added. Then, the reaction solution was subjected to extraction with ethyl acetate (20 mL × 3). The organic phases were combined, concentrated, and separated by column chromatography (EA/PE, 50%-100%), to obtain 460 mg of yellow oily substance with a yield of 70.8%. LCMS: m/z (254 nm): 521.0 [M+H]⁺.

### Step 4: 4-amino-N,N-bis[(2,4-dimethoxyphenyl)methyl]pyridine-2-sulfonamide (28e)

**28d** (800 mg), N-methylpyrrolidone (7 mL), ammonium acetate (1.06 g), copper acetylacetonate (107 mg), Ligand (115 mg), and cesium carbonate (1.34 g) were added in a reaction flask, purged with nitrogen for three times, and stirred at 95°C for 16 h. The reaction solution was cooled down to room temperature. Then, water (20 mL) was added. Then, the reaction solution was subjected to extraction with ethyl acetate (20 mL × 3). The organic phases were combined, concentrated, and separated by column chromatography (EA/PE, 50%-100%), to obtain 460 mg of yellow oily substance with a yield of 70.8%. LCMS: m/z (254 nm): 410.2 [M+H]⁺.

### Step 5: rac-(2R,3R,4S,5R)-N-[2-({bis[(2,4-dimethoxyphenyl)methyl]amino}dioxo-λ⁶-thio)pyridin-4-yl]-3-{3,4-difluoro-2-[(methyl-d3)oxy]phenyl}-3a-methyl-5a-(trifluoromethyl)hexahydrocyclobuta[1,2-b]furan-2-carboxamide (28f)

**28f** was obtained according to a method similar to that in **Example 1.** LCMS: MS m/z (254 nm): 825.3 [M+H]⁺.

### Step 6: rac-(2R,3R,4S,5R)-N-[2-(aminodioxo-λ⁶-thio)pyridin-4-yl]-3-{3,4-difluoro-2-[(methyl-d3)oxy]phenyl}-3a-methyl-5a-(trifluoromethyl)hexahydrocyclobuta[2,1-b]furan-2-carboxamide (Example 28)

**28f** (50 mg), DCM (2 mL), and trifluoroacetic acid (146 mg) were added in a reaction flask and stirred at room temperature for 12 h. The reaction was monitored by LCMS until the raw materials were fully converted. The reaction solution was concentrated and separated by silica gel plate chromatography (EA/PE, 40%) to obtain 23 mg of white solid with a yield of 74%. LC-MS: m/z (254 nm): 525.1 [M+H]⁺.

### Example 29:

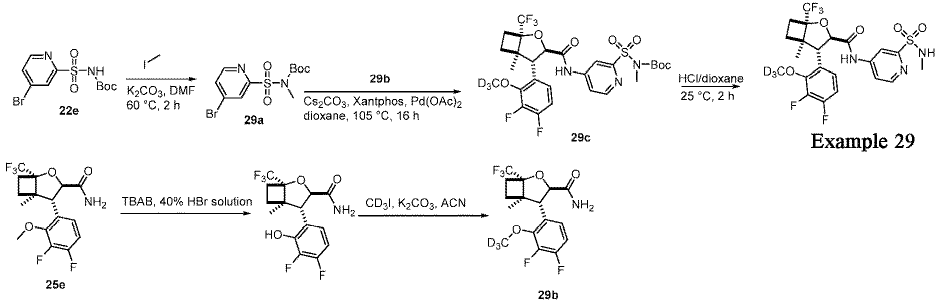

### Step 1: compound ((4-bromopyridin-2-yl)sulfonyl)(methyl)tert-butyl carbamate (29a)

The compound **22e** (100 mg, 0.30 mmol, 1.0 eq) was dissolved in anhydrous tetrahydrofuran (5 mL), and potassium carbonate (82 mg, 0.59 mmol, 2.0 eq) and iodomethane (50 mg, 0.35 mmol, 1.2 eq) were added. The reaction solution reacted at 60°C for 2 h. The reaction was monitored by LCMS until the raw materials were fully converted. The reaction solution with water (10 mL) added was subjected to extraction with ethyl acetate (10 mL × 3), washed with water and saturated saline sequentially, dried over anhydrous sodium sulfate, and then concentrated. The residue was purified by silica gel column chromatography (petroleum ether/ethyl acetate = 16/1) to obtain **29a** (100 mg, 96% yield), which was a white solid. LCMS: [M+Na]⁺ = 373.0.

### Step 2: rac-(3aR,3S,5aR)-3-{3,4-difluoro-2-[(methyl-d3)oxy]phenyl}-3a-methyl-5a-(trifluoromethyl)hexahydrocyclobuta[1,2-b]furan-2-carboxamide (29b)

The intermediate **29b** was prepared according to the synthesis method for the intermediate **1c**. LCMS: [M+H]⁺ = 369.1.

### Step 3: 2-methylpropan-2-yl rac-(2R,3R,4S,5R)-{[(4-{[(3-{3,4-difluoro-2-[(methyl-d3)oxy]phenyl}-3a-methyl-5a-(trifluoromethyl)hexahydrocyclobuta[2,1-b]furan-2-yl)carbonyl]amino }pyridine-2-yl)dioxo-λ⁶-thio](methyl)amino}methanoate (29c)

The compound **29a** (55 mg, 0.15 mmol, 1.1 eq) was dissolved in dioxane (5 mL), and the compound **29b** (52 mg, 0.14 mmol, 1.0 eq), 4,5-bis(diphenylphosphino)-9,9-dimethylxanthene (15 mg, 0.027 mmol, 0.2 eq), cesium carbonate (89 mg, 0.27 mmol, 2.0 eq), and palladium acetate (2 mg, 0.013 mmol, 0.1 eq) were added. The reaction solution reacted at 105°C under nitrogen protection for 16 h. The reaction was monitored by LCMS until the raw materials were fully converted. The reaction solution was concentrated. The residue was purified by silica gel column chromatography (dichloromethane/methanol = 10/1) to obtain the compound **29c** (70 mg, 80% yield), which was a yellow oily substance. LCMS: [M+H]⁺ = 639.2.

### Step 4: rac-(2R,3R,4S,5R)-3-{3,4-difluoro-2-[(methyl-d3)oxy]phenyl}-3a-methyl-N-{2-[(methylamino)dioxo-λ⁶-thio]pyridin-4-yl}-5a-(trifluoromethyl)hexahydrocyclobuta[2,1-b]furan-2-carboxamide (Example 29)

The compound **29c** (70 mg, 0.11 mmol, 1.0 eq) was dissolved in hydrochloric acid/dioxane (4 M, 10 mL). The reaction solution reacted at 25°C for 4 h. The reaction was monitored by LCMS until the raw materials were fully converted. The reaction mixture was concentrated. The residue was purified by preparative separation (0.01% FA) to obtain **Example 29** (4.2 mg, 7.1% yield). LCMS: [M+H]⁺ = 539.1.

### Example 30:

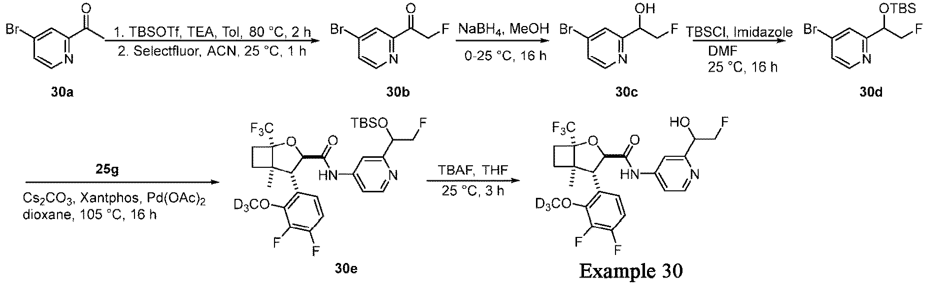

### Step 1: 1-(4-bromopyridin-2-yl)-2-fluoroethan-1-one (30b)

In a reaction flask, the compound 1-(4-bromopyridin-2-yl)ethan-1-one **30a** (300 mg, 1.50 mmol, 1.00 eq) was dissolved in toluene (3 mL), and tert-butyldimethylsilyl trifluoromethanesulfonate (595 mg, 2.25 mmol, 1.50 eq) and triethylamine (326 mg, 3.22 mmol, 2.15 eq) were added. The reaction solution was stirred at 80°C for 2 h. The reaction solution was concentrated, and acetonitrile (3 mL) and Selectfluor (537 mg, 1.51 mmol, 1.01 eq) were added. The reaction solution was stirred at 25°C for 1 h. The reaction was monitored by LCMS until the raw materials were fully converted. The reaction solution was concentrated. The residue was purified by silica gel column chromatography (petroleum ether/ethyl acetate = 50/1) to obtain the compound **30b** (176 mg, 54% yield), which was a white solid. LCMS: [M+H]⁺ = 218.0.

### Step 2: 1-(4-bromopyridin-2-yl)-2-fluoroethan-1-ol (30c)

In a reaction flask, the compound **30b** (176 mg, 0.81 mmol, 1.00 eq) was dissolved in methanol (5 mL), and sodium borohydride (60 mg, 1.60 mmol, 1.98 eq) was added at 0°C. The reaction solution was stirred at 25°C for 16 h. The reaction was monitored by LCMS until the raw materials were fully converted. The reaction solution was concentrated. The residue was purified by silica gel column chromatography (petroleum ether/ethyl acetate = 3/1) to obtain the compound **30c** (145 mg, 81% yield), which was a yellow oily substance. LCMS: [M+H]⁺ = 220.0.

### Step 3: 4-bromo-2-(1-((tert-butyldimethylsilyl)oxy)-2-fluoroethyl)pyridine (30d)

In a reaction flask, the compound **30c** (130 mg, 0.59 mmol, 1.0 eq) was dissolved in N,N-dimethylformamide (5 mL), and imidazole (149 mg, 2.18 mmol, 3.7 eq) and tert-butyldimethylchlorosilane (151 mg, 1.00 mmol, 1.7 eq) were added. The reaction solution was stirred at 25°C for 16 h. The reaction was monitored by LCMS until the raw materials were fully converted. The reaction solution was diluted with water (15 mL), subjected to extraction with ethyl acetate (10 mL × 3), washed with saturated saline, dried over anhydrous sodium sulfate, and then concentrated. The residue was purified by silica gel column chromatography (petroleum ether/ethyl acetate = 20/1) to obtain the compound **30d** (173 mg, 87% yield), which was a colorless oily substance. LCMS: [M+H]⁺ = 334.0.

### Step 4: rac-(2R,3R,4S,5R)-3-{3,4-difluoro-2-[(methyl-d3)oxy]phenyl}-N-[2-(1-fluoro-4,4,5,5-tetramethyl-3-oxa-4-silahex-2-yl)pyridin-4-yl]-3a-methyl-5a-(trifluoromethyl)hexahydrocyclobuta[2,1-b]furan-2-carboxamide (30e)

In a reaction flask, the compound **30d** (151 mg, 0.45 mmol, 1.5 eq) was dissolved in anhydrous 1,4-dioxane (5 mL), and cesium carbonate (196 mg, 0.60 mmol, 2.0 eq), 25g (110 mg, 0.30 mmol, 1.0 eq), 4,5-bis(diphenylphosphino)-9,9-dimethylxanthene (35 mg, 0.06 mmol, 0.2 eq), and palladium acetate (5 mg, 0.03 mmol, 0.1 eq) were added. The reaction solution reacted at 105°C in a nitrogen atmosphere for 16 h. The reaction was monitored by LCMS until the raw materials were fully converted. The reaction solution was diluted with water (15 mL), subjected to extraction with ethyl acetate (10 mL × 3), washed with saturated saline, dried over anhydrous sodium sulfate, and then concentrated. The residue was purified by silica gel column chromatography (petroleum ether/ethyl acetate = 2/1) to obtain the compound **30e** (193 mg, 98% yield), which was a colorless oily substance. LCMS: [M+H]⁺ = 622.3.

### Step 5: rac-(2R,3R,4S,5R)-3-{3,4-difluoro-2-[(methyl-d3)oxy]phenyl }-N-[2-(2-fluoro-1-hydroxyethyl)pyridin-4-yl]-3a-methyl-5a-(trifluoromethyl)hexahydrocyclobuta[2,1-b]furan-2-carboxamide (Example 30)

The compound **30e** (193 mg, 0.31 mmol, 1.0 eq) was dissolved in tetrahydrofuran (3 mL), and a tetrabutylammonium fluoride in tetrahydrofuran solution (0.4 mL) was added. The reaction solution reacted at 25°C for 3 h. The reaction was monitored by LCMS until the raw materials were fully converted. The reaction solution was diluted with water (10 mL), subjected to extraction with ethyl acetate (10 mL × 3), washed with saturated saline, dried over anhydrous sodium sulfate, and then concentrated. The residue was purified by silica gel column chromatography (petroleum ether/ethyl acetate = 1/1) and preparative separation (0.1% FA) to obtain the compound **Example 30** (68.5 mg, 41.3% yield). LCMS: [M+H]⁺ = 508.2.

### Example 31:

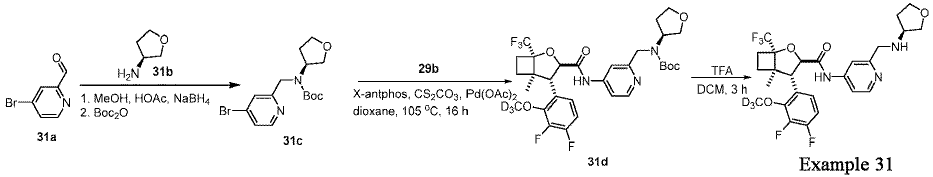

### Step 1: (S)-(4-bromopyridin-2-yl)methyl(tetrahydrofuran-3-yl)tert-butyl carbamate (31c)

In a single-necked flask, the compound 4-bromopyridine carbaldehyde **31a** (100 mg, 0.54 mmol, 1.0 eq) was dissolved in 1,2-dichloroethane (5 mL), and (S)-tetrahydrofuran-3-amine **31b** (61 mg, 0.70 mmol, 1.3 eq), acetic acid (32.38 mg, 0.54 mmol, 1.0 eq), and sodium triacetoxyborohydride (171 mg, 0.81 mmol, 1.5 eq) were added. The reaction solution was stirred at 25°C for 3 h. Then, di-tert-butyl dicarbonate (176 mg, 0.81 mmol, 1.5 eq) was added to the reaction solution. The reaction solution was stirred at 25°C for 0.5 h. The reaction was monitored by LCMS until the raw materials were fully converted. The reaction solution was concentrated. The residue was purified by silica gel column chromatography (petroleum ether/ethyl acetate = 4/1) to obtain the compound **31c** (170 mg, 88% yield), which was a yellow oily substance.

### Step 2: 2-methylpropan-2-yl rac-(4-(2R,3R,4S,5R)-{[(4-{[(3-{3,4-difluoro-2-[(methyl-d3)oxy]phenyl}-3a-methyl-5a-(trifluoromethyl)hexahydrocyclobuta[2,1-b]furan-2-yl)carbonyl]amino}pyridine-2-yl)methyl][(3S)-tetrahydrofuran-3-yl]amino}methanoate (31d)

In a single-necked flask, the compound **31c** (65 mg, 0.18 mmol, 1.0 eq) was dissolved in anhydrous 1,4-dioxane (5 mL), and cesium carbonate (107 mg, 0.33 mmol, 2.0 eq), the compound **29b** (60 mg, 0.16 mmol, 1.0 eq), 4,5-bis(diphenylphosphino)-9,9-dimethylxanthene (19 mg, 0.03 mmol, 0.2 eq), and palladium acetate (2.7 mg, 0.02 mmol, 0.1 eq) were added. The reaction solution reacted at 105°C in a nitrogen atmosphere for 16 h. The reaction was monitored by LCMS until the raw materials were fully converted. The reaction solution was diluted with water (15 mL), subjected to extraction with ethyl acetate (10 mL × 3), washed with saturated saline, dried over anhydrous sodium sulfate, and then concentrated. The residue was purified by silica gel column chromatography (petroleum ether/ethyl acetate = 20/1) to obtain the compound **31d** (30 mg, 29% yield), which was a yellow oily substance. LCMS: [M+H]⁺ = 645.2.

### Step 3: rac-(2R,3R,4S,5R)-3-{ 3,4-difluoro-2-[(methyl-d3)oxy]phenyl}-N-[2-({[(3S)-tetrahydrofuran-3-yl]amino}methyl)pyridin-4-yl]-3a-methyl-5a-(trifluoromethyl)hexahydrocyclobuta[2,1-b]furan-2-carboxamide (Example 31)

The compound **31d** (30 mg, 0.05 mmol, 1.0 eq) was dissolved in dichloromethane (2 mL), and trifluoroacetic acid (1 mL) was added. The reaction solution reacted at 25°C for 3 h. The reaction was monitored by LCMS until the raw materials were fully converted. The reaction mixture was concentrated. The residue was purified by preparative separation (0.1% FA) to obtain **Example 31** (5 mg, 18% yield). LCMS: [M+H]⁺ = 545.2.

### Example 32: rac-(2R,3R,4S,5R)-3-{3,4-difluoro-2-[(methyl-d3)oxy]phenyl}-3a-methyl-5a-(trifluoromethyl)-N-[2-(5-aza-2-oxahexa-6-yl)pyridin-4-yl]hexahydrocyclobuta[2,1-b]furan-2-carboxamide

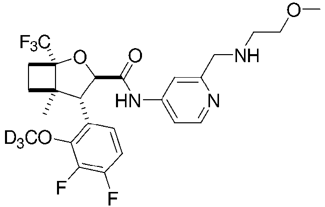

**Example 32** was prepared according to a method similar to that in Example 31. LCMS: [M+H]⁺ = 533.2.

### Example 36:

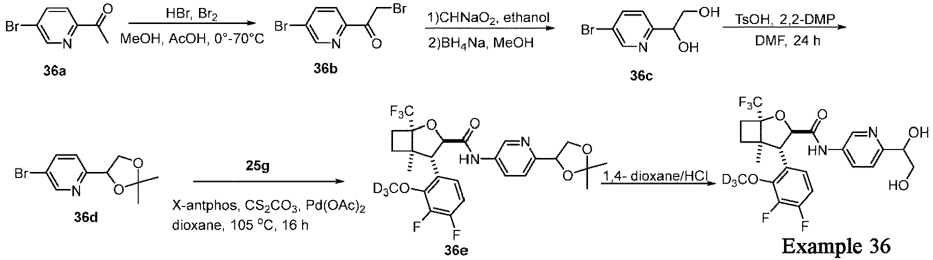

### Step 1: 2-bromo-1-(5-bromopyridin-2-yl)ethan-1-one (36b)

1-(5-bromopyridin-2-yl)ethanone **36a** (2.0 g, 0.01 mmol, 1.0 eq) was dissolved in methanol (10 mL) and acetic acid (15 mL) and cooled down to 0°C. A 30% hydrogen bromide in acetic acid solution (2.8 mL) was added. Bromine (1.6 g, 0.01 mmol, 1.0 eq) in acetic acid (5 mL) was added dropwise to warm the reaction mixture to room temperature, and then the reaction mixture was heated to 70°C and stirred for 1 h. The reaction was monitored by LCMS until the reaction was completed. The mixture was concentrated under reduced pressure. The residue was crystallized with isopropanol to obtain **36b** (1.0 g, 32% yield), which was a yellow solid. LCMS: [M+H]⁺ = 278.9.

### Step 2: 1-(5-bromopyridin-2-yl)ethan-1,2-diol (36c)

An ethanol solution (10 mL) of 2-bromo-1-(5-bromopyridin-2-yl)ethanone **36b** (1.0 g, 3.6 mmol, 1.0 eq) and sodium formate (1.0 g, 14.4 mmol, 4.0 eq) was heated to 50°C to react for 3 h. After the reaction, the reaction solution was cooled down to room temperature, and the solvent was evaporated under reduced pressure. The mixture was quenched with water (10 mL), and subjected to extraction with dichloromethane (50 mL × 3). The organic phases were combined, dried over sodium sulfate, and filtered. The filtrate was concentrated under reduced pressure. The residue was absorbed in methanol (10 mL) and cooled down to 0°C. Sodium borohydride (0.4 g, 10.8 mmol, 3.0 eq) was added in batches, and the mixture was stirred at 0°C for 1 h. Concentrated hydrochloric acid was added to adjust pH to 2, and the solvent was removed under reduced pressure. Extraction with dichloromethane (50 mL) and saturated aqueous sodium bicarbonate (10 mL) was carried out for three times. The organic phases were combined, and dried over sodium sulfate. The residue was purified by silica gel column chromatography (petroleum ether/ethyl acetate = 4/1) to obtain the compound **36c** (350 mg, 42% yield), which was a yellow solid. LCMS: [M+H]⁺ = 218.0.

### Step 3: 5-bromo-2-(2,2-dimethyl-1,3-dioxolan-4-yl)pyridine (36d)

1-(5-bromopyridin-2-yl)ethan-1,2-diol **36c** (100 mg, 0.46 mmol, 1.0 eq) was dissolved in N,N-dimethylformamide (5 mL), and 2,2-dimethoxypropane (48 mg, 0.46 mmol, 1.0 eq) and p-toluenesulfonic acid (7.9 mg, 0.05 mmol, 0.1 eq) were added. The mixture was stirred at 25°C for 24 h. The reaction was monitored by LCMS until the reaction was completed. The mixture was quenched with water (50 mL), and then subjected to extraction with dichloromethane (50 mL × 3). The organic phases were combined, dried over sodium sulfate, and filtered. The filtrate was concentrated under reduced pressure. The residue was purified by elution with ethyl acetate in petroleum ether in 0-50% to obtain 5-bromo-2-(2,2-dimethyl-1,3-dioxolan-4-yl)pyridine **36d** (60 mg, 48% yield), which was a yellow oily substance. LCMS: [M+H]⁺ = 258.0.

### Step 4: rac-(1R,3R,4S,5R)-3-{3,4-difluoro-2-[(methyl-d3)oxy]phenyl}-N-[6-(2,2-dimethyl-1,3-dioxol-4-yl)pyridin-3-yl]-3a-methyl-5a-(trifluoromethyl)hexahydrocyclobuta[2,1-b]furan-2-carboxamide (36e)

**25g** (50 mg, 0.14 mmol, 1.0 eq) was dissolved in 1,4-dioxane (5 mL), and 5-bromo-2-(2,2-dimethyl-1,3-dioxolan-4-yl)pyridine (53 mg, 0.21 mmol, 1.5 eq), 4,5-bis(diphenylphosphino)-9,9-dimethylxanthene (16 mg, 0.027 mmol, 0.2 eq), cesium carbonate (134 mg, 0.41 mmol, 3.0 eq), and palladium acetate (3.1 mg, 0.014 mmol, 0.1 eq) were added. The mixture was stirred at 105°C for 16 h. After the reaction, the mixture was quenched with water (50 mL), and then subjected to extraction with ethyl acetate (50 mL × 3). The organic phases were combined, dried over sodium sulfate, and filtered. The filtrate was concentrated under reduced pressure, and purified by silica gel column chromatography (petroleum ether/ethyl acetate = 3/1) to obtain **36e** (50 mg, 67% yield), which was a yellow oily substance. LCMS: [M+H]⁺ = 546.2.

### Step 5: rac-(1R,3R,4S,5R)-03-{3,4-difluoro-2-[(methyl-d3)oxy]phenyl}-N-[6-(1,2-dihydroxyethyl)pyridin-3-yl]-3a-methyl-5a-(trifluoromethyl)hexahydrocyclobuta[2,1-b]furan-2-carboxamide (Example 36)

**36e** (50 mg, 0.0184 mmol, 1.0 eq) was dissolved in hydrochloric acid/1,4-dioxane (5 mL). The mixture was stirred at 25°C for 1 h. The mixture was concentrated under reduced pressure, and purified by preparative separation (0.1% NH₃), to obtain the compound **Example 36** (4.2 mg, 9% yield). LCMS: [M+H]⁺ = 506.2.

### Example 40: rac-(2R,3R,4S,5R)-N-[6-(aminodioxo-λ⁶-thio)pyridin-3-yl]-3-{3,4-difluoro-2-[(methyl-d3)oxy]phenyl }-3a-methyl-5a-(trifluoromethyl)hexahydrocyclobuta[2,1-b]furan-2-carboxamide

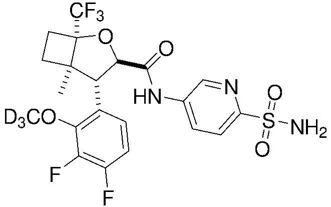

Example 40 was prepared according to a method similar to that in Example 28. LCMS: [M+H]⁺ = 524.2.

### Example 41:

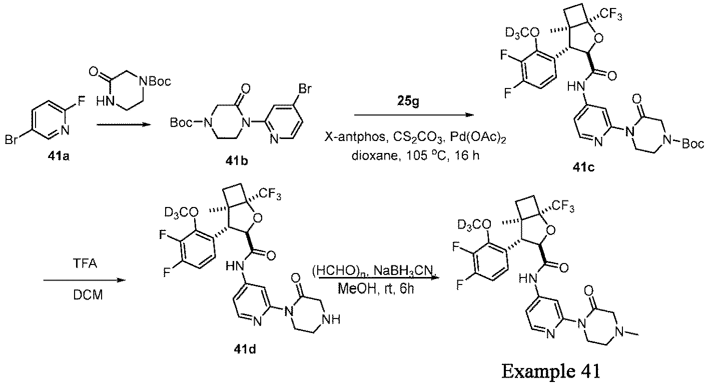

### Step 1: tert-butyl 4-(5-bromopyridin-2-yl)-3-oxopiperidin-1-carboxylate (41b)

1-Boc-3-oxopiperazine (1.37 g, 6.82 mmol, 1.2 eq) was dissolved in DMF (15 mL) and cooled down to 0°C, and sodium hydride (341 mg, 8.52 mmol, 1.5 eq) was added. The mixture was stirred for 30 min. 2-fluoro-5-bromopyridine 100a (1.0 g, 5.68 mmol, 1.0 eq) was added. Then, the mixture was heated to 60°C and stirred for 16 h. The mixture with water added was subjected to extraction with ethyl acetate (50 mL × 3). The organic phases were combined, washed with saline, dried over sodium sulfate, filtered, spin-dried, and purified by silica gel column chromatography (petroleum ether/ethyl acetate = 3/1), to obtain tert-butyl 4-(5-bromopyridin-2-yl)-3-oxopiperidin-1-carboxylate **41b** (560 mg, 27.7% yield), which was a white solid. LCMS: [M+H]⁺ = 355.9, 357.9.

### Step 2: rac-tert-butyl 4-(4-((1R,3R,4S,5R)-4-(3,4-difluoro-2-methoxyphenyl-d3)-5-methyl-1-(trifluoromethyl)-2-oxabicyclo[3.2.0]heptane-3-carboxamido)pyridin-2-yl)-3-oxopiperazin-1-carboxylate (41c)

The compound **25g** (50 mg, 0.14 mmol, 1.0 eq) was dissolved in dioxane (5 mL), and **41b** (65 mg, 0.18 mmol, 1.3 eq), cesium carbonate (133 mg, 0.41 mmol, 3.0 eq), 4,5-bis(diphenylphosphino)-9,9-dimethylxanthene (11 mg, 0.03 mmol, 0.2 eq), and palladium acetate (3 mg, 0.01 mmol, 0.1 eq) were added. The reaction solution reacted at 105°C in a nitrogen atmosphere for 16 h. The reaction was monitored by LCMS until the raw materials were fully converted. The reaction mixture was concentrated. The residue was purified by silica gel column chromatography (petroleum ether/ethyl acetate = 10/1) to obtain the compound **41c** (50 mg, 57% yield), which was a colorless oily substance. LCMS: [M+H]⁺ = 644.3.

### Step 3: rac-(1R,3R,4S,5R)-4-(3,4-difluoro-2-methoxyphenyl-d3)-5-methyl-N-(2-(2-oxopiperazin-1-yl)pyridin-4-yl)-1-(trifluoromethyl)-2-oxabicyclo[3.2.0]heptane-3-carboxamide (41d)

The compound **41c** (50 mg, 0.078 mmol, 1.0 eq) was dissolved in trifluoroacetic acid (5 mL). The reaction solution reacted at 25°C for 2 h. The reaction was monitored by LCMS until the raw materials were fully converted. The reaction mixture was concentrated to obtain the compound **41d** (40 mg, 95% yield), which was a white solid. LCMS: [M+H]⁺ = 544.3.

### Step 4: rac-(1R,3R,4S,5R)-4-(3,4-difluoro-2-methoxyphenyl-d3)-5-methyl-N-(2-(4-methyl-2-oxopiperazin-1-yl)pyridin-4-yl)-1-(trifluoromethyl)-2-oxabicyclo[3.2.0]heptane-3-carboxamide (Example 41)

The compound **41d** (40 mg, 0.07 mmol, 1.0 eq) and paraformaldehyde (4 mg, 0.14 mmol, 2.0 eq) were dissolved in methanol (5 mL). The reaction solution reacted at 25°C for 30 min. Sodium cyanoborohydride (8 mg, 0.14 mmol, 2.0 eq) was added. The reaction solution reacted at 40°C for 1 h. The reaction was monitored by LCMS until the raw materials were fully converted. The reaction mixture was concentrated. The residue was purified by preparative separation (0.1% FA) to obtain **Example 41** (6.7 mg, 13% yield). LCMS: [M+H]⁺ = 558.2.

### Example 45:

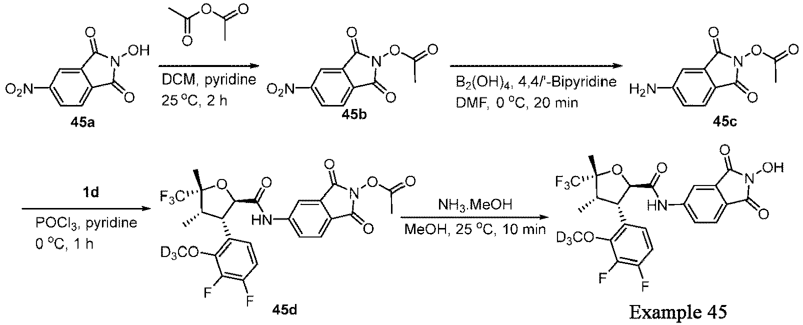

### Step 1: 5-nitro-1,3-dioxoisoindolin-2-yl acetate (45b)

In a single-necked flask, the compound 2-hydroxy-5-nitroisoindolin-1,3-dione **45a** (300 mg, 1.44 mmol, 1.0 eq) was dissolved in dichloromethane (5 mL), and the compound acetic anhydride (221 mg, 2.17 mmol, 1.5 eq) and pyridine (228 mg, 2.88 mmol, 2.0 eq) were added. The reaction solution was stirred at 0°C for 2 h. The reaction was monitored by LCMS until the raw materials were fully converted. The reaction solution was concentrated. The residue was purified by silica gel column chromatography (petroleum ether/ethyl acetate = 4/1) to obtain the compound **45b** (300 mg, 83% yield), which was a white solid. LCMS: [M+H]⁺ = 251.0.

### Step 2: 5-amino-1,3-dioxoisoindolin-2-yl acetate (45c)

In a single-necked flask, the compound **45b** (300 mg, 1.20 mmol, 1.0 eq) was dissolved in N,N-dimethylformamide (5 mL), and tetrahydroxydiboron (430 mg, 4.80 mmol, 4.0 eq) and 4,4'-dipyridyl (19 mg, 0.12 mmol, 0.1 eq) were added. The reaction solution reacted at 0°C for 20 min. The reaction was monitored by LCMS until most of the raw materials were fully converted. Ethyl acetate (20 mL) and water (20 mL) were added sequentially to the reaction mixture. The organic phase was washed once with saturated saline (20 mL), dried over anhydrous sodium sulfate, and filtered. The filtrate was concentrated. The residue was separated and purified by column chromatography (petroleum ether/ethyl acetate = 1/1) to obtain the compound 45c (80 mg, 30% yield), which was a yellow oily substance. LCMS: [M+H]⁺ = 221.0.

### Step 3: rac-(2R,3S,4S,5R)-N-(2-acetoxy-1,3-dioxo-2,3-dihydro-1H-isoindol-5-yl)-3-{3,4-difluoro-2-[(methyl-d3)oxy]phenyl}-4,5-dimethyl-5-(trifluoromethyl)tetrahydrofuran-2-carboxamide (45d)

The compound 1d (100 mg, 0.28 mmol, 1.0 eq) was dissolved in anhydrous pyridine (3 mL), and the compound 45c (62 mg, 0.28 mmol, 1.0 eq) and phosphorus oxychloride (173 mg, 1.13 mmol, 4.0 eq) were added. The reaction solution reacted at 0°C for 1 h. The reaction was monitored by LCMS until the raw materials were fully converted. The reaction mixture was diluted with water (10 mL), subjected to extraction with ethyl acetate (10 mL × 3), washed with saturated saline, and filtered. The filtrate was concentrated. The residue was separated and purified by column chromatography (petroleum ether/ethyl acetate = 4/1) to obtain the compound 45d (100 mg, 64% yield), which was a white solid. LCMS: [M+H]⁺ = 560.2.

### Step 4: (2R,3S,4S,5R)-3-{3,4-difluoro-2-[(methyl-d3)oxy]phenyl}-N-(2-hydroxy-1,3-dioxo-2,3-dihydro-1H-isoindol-5-yl)-4,5-dimethyl-5-(trifluoromethyl)tetrahydrofuran-2-carboxamide (Example 45)

The compound 45d (100 mg, 0.18 mmol, 1.0 eq) was dissolved in methanol (3 mL), and an ammonia in methanol solution (0.5 mL) was added. The reaction solution reacted at 25°C for 10 min. The reaction was monitored by LCMS until the raw materials were fully converted. The reaction mixture was concentrated. The residue was purified by preparative separation (0.1% FA) to obtain Example 45 (28.2 mg, 29% yield). LCMS: [M-H]⁻ = 516.1.

### Control compound 1:

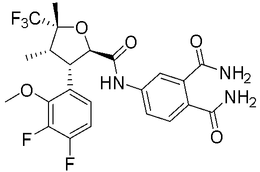

The **control compound 1** was prepared with the intermediate **1a** that replaced **1d** in Example 1. ESI-MS m/z calc. 515.1, found 516.1 (M+1)⁺. ¹H NMR (400 MHz, DMSO-*d*₆) δ 10.42 (s, 1H), 7.76-7.60 (m, 4H), 7.49 (d, *J =* 8.2 Hz, 1H), 7.30 (d, *J =* 20.8 Hz, 2H), 7.22-7.09 (m, 2H), 5.07 (d, *J =* 10.4 Hz, 1H), 4.24 (dd, *J =* 10.3, 7.6 Hz, 1H), 3.95 (d, *J =* 2.1 Hz, 2H), 2.76 (t, *J* = 7.5 Hz, 1H), 1.60 (s, 3H), 0.73 (d, *J =* 7.4 Hz, 3H).

**Bioactivity test:** In vitro inhibitory effects of the compound against Naᵥ1.8: In vitro inhibitory effect IC₅₀ of the test compound against Naᵥ1.8.

A CHO cell line stably expressing the Naᵥ1.8 sodium channel was used for experimental assays with the following gene information: Sodium channel, voltage-gated, type 8, alpha (SCN10A), cDNA strictly similar to GenBank accession number: NM_006514.

Before the patch clamp assay, the cells were detached with 0.25%-Trypsin-EDTA, and 6.5×10³ cells were spread onto cover slips and cultured in a 24-well plate (final volume: 500 µL) for 18 h. Then, the assay was carried out.

Fluids for electrophysiological recording included: extracellular fluid: K-007-1, 140 mM NaCl, 3.5 mM KCl, 1 mM MgCl₂•6H₂O, 2 mM CaCl₂•2H₂O, 10 mM D-Glucose, 10 mM HEPES, 1.25 mM NaH₂PO₄•2H₂O, and NaOH for adjustment to pH = 7.4; and intracellular fluid: Nav-001-2, 50 mM CsCl, 10 mM NaCl, 10 mM HEPES, 60 mM CsF, 20 mM EGTA, and CsOH for adjustment to pH = 7.2. The extracellular fluid was stored for 2 weeks. The intracellular fluid was prepared and dispensed as 1 mL per tube, and frozen in a refrigerator at -20°C. Freshly thawed intracellular fluid was used for daily experiments. All intracellular fluids have to be used up within three months. Beyond three months, the old intracellular fluid has to be discarded, and the intracellular fluid needs to be prepared again.

Patch clamp assay: Whole-cell patch-clamp recording Naᵥ1.8 sodium currents under voltage stimulation was carried out as follows: When a whole-cell seal was formed, the cell was clamped at a voltage of -120 mV and maintained for 30 ms. The cell was depolarized from the clamping voltage to 0 mV and maintained for 50 ms. Then, the voltage was restored to -50 mV (for a specific voltage, refer to a half-inactivation voltage from an IV test) and maintained for 5 s. Next, the cell membrane potential was restored to -120 mV and maintained for 20 ms. Then, the cell was depolarized to 0 mV and maintained for 50 ms. Finally, the voltage was restored to the clamping voltage -120 mV and maintained for 30 ms. Data was collected at 20 ms intervals. The effect of the drug on a peak sodium current was observed. Experimental data was collected by an EPC 10 amplifier (HEKA) and stored in PatchMaster (HEKA) software.

A capillary glass tube was pulled into a recording electrode by using a microelectrode puller. The electrode filled with the intracellular fluid was loaded into an electrode holder. A microelectrode manipulator was manipulated under an inverted microscope to immerse the electrode into the extracellular fluid, and an electrode resistance (Rpip) was recorded. The electrode was brought into contact with the cell surface, and suction was applied under negative pressure to form a high-resistance seal (GΩ). In this case, fast capacitance compensation was carried out, and negative pressure was then applied to break the cell membrane through suction to form a whole-cell recording mode. Then, slow capacitance compensation was carried out, and experimental parameters such as a membrane capacitance (Cm) and a series resistance (Rs) were recorded. No leakage compensation was carried out.

The drug was administered after the Naᵥ1.8 current obtained by whole-cell recording. The drug acted at each concentration for 5 min (or until the current was stable), and then the drug was tested at a next concentration. Each test compound was tested for multiple concentrations. The cover slips with the cells spread were placed in a recording bath under an inverted microscope. A blank control extracellular fluid and a working solution of the to-be-tested compound were perfused by gravity through the recording bath into the cells from low concentration to high concentration. Fluid exchange was carried out by using a peristaltic pump during the recording. A current detected for each cell in the compound-free extracellular fluid served as its own control. For each concentration, at least three cells were tested independently for three times. All the electrophysiological tests were carried out at room temperature.

Data analysis: First, the current after the action of drug at each concentration and the blank control current were normalized ( $\frac{Peak tail {current}_{component}}{Peak tail {current}_{control}}$). Then, the inhibition ( $1 - \frac{Peak tail {current}_{component}}{Peak tail {current}_{control}}$) corresponding to each drug concentration was calculated, and the mean (Mean), standard deviation (SD), and standard error (SE) were calculated for the inhibition of each concentration.
Y = Bottom + (Top - Bottom)/(1 + 10^((LogIC₅₀ - X) × HillSlope))

The IC₅₀ value of each compound was calculated by using the foregoing equation, and nonlinear fitting was carried out for the dose-dependent effect, where IC₅₀ is the half-maximal inhibitory concentration. The calculation of IC₅₀ and curve fitting were carried out using GraphPad Prism software.

**Table 2: Inhibitory activity of the compound of the present invention on Naᵥ1.8**

| Example | IC₅₀ (nM) |
|---|---|
| **1** | < 0.5 |
| **54** | < 0.5 |
| **55** | < 0.5 |

The inhibitory effect against Naᵥ1.8 was tested for other compounds in the present invention by using the same method. It indicates that the compound of the present invention has a good inhibitory effect against Naᵥ1.8.

### Experiment on liver microsome stability of the compound of the present invention:

Step 1: Preparation of working solutions of test compounds and control compounds: Add 5 µL of stock solution (10 mM DMSO solution) to 495 µL of acetonitrile solution (that is, 100 µM, 99% acetonitrile). Testosterone, diclofenac, and propafenone were used as controls in this experiment.

Step 2: Preparation of NADPH coenzyme working solution: β-nicotinamide adenine dinucleotide phosphate, reduced, tetrasodium salt; (Supplier: BONTAC, Product No: BT04). Weigh an appropriate amount of NADPH powder, and dilute the NADPH powder in 10 mM MgCl₂ solution.

Step 3: Preparation of liver microsomes: Prepare liver microsomes with 100 mM potassium phosphate buffer into 0.56 mg/mL working solution.

Step 4: Preparation of stop solution: Use cold (4°C) acetonitrile with 250 nM tolbutamide and 250 nM labetalol added as internal standards as a stop solution.

Step 5: Experimental operation:
a. Add 445 µL of liver microsome working solution (0.56 mg/mL) to pre-warmed T60 and NCF60 plates, followed by uniform stirring. Add 54 µL of liver microsome working solution and 6 µL of NADPH coenzyme working solution to a T0 blank plate, and add 180 µL of stop solution for use.
b. Add 5 µL of working solution of a test compound to the T60 and NCF60 plates containing the liver microsome working solution.
c. Add 50 µL of phosphate buffer to the NCF60 plate.
d. Transfer 54 µL of solution from the T60 plate to the T0 plate as a sample at minute 0, and add 44 µL of NADPH coenzyme working solution.

**Table 3: Final concentrations of components in the reaction system**

| Component | Concentration |
|---|---|
| Microsome | 0.5 mg/mL |
| Test compound | 1 µM |
| Control compound | 1 µM |
| Acetonitrile | 99% |
| DMSO | 1% |
| NADPH | 1 mM |

e. Transfer 60 µL of sample from the T60 plate to the stop plate separately at minute 5, minute 15, minute 30, minute 45, and minute 60.
f. Transfer 60 µL of sample from the NCF60 plate to the stop plate at minute 60.
g. Mix all the samples fully on the plate for 10 min, and centrifuge the samples by using a centrifuge at 4°C and 4000 rpm for 20 min. Transfer 80 µL of supernatant and dilute it with 240 µL of ultrapure water, followed by LC-MS/MS analysis.

Step 6: Data Analysis: Calculate T1/2 and CLint(mic) (µL/min/mg) through first-order kinetic equations. ${C}_{t} = {C}_{0} \cdot {e}^{- {k}_{e} \cdot t}$ when ${C}_{t} = \frac{1}{2} {C}_{0} ,$ ${T}_{1 / 2} = \frac{Ln 2}{{k}_{e}} = \frac{0.693}{{k}_{e}}$ ${CL}_{int \left(mic\right)} = \frac{0.693}{In vitro {T}_{1 / 2}} \cdot \frac{1}{mg / mL microsomal protein in reaction system}$ ${CL}_{int \left(liver\right)} = {CL}_{int \left(mic\right)} \cdot \frac{mg microsomes}{g liver} \cdot \frac{g liver}{kg body weight}$

Results of the experiment on liver microsome metabolic stability of monkeys, rats, and humans are shown in Table 4. It can be learned from the table that Example 1 of the present invention provides better metabolic stability and may offer better pharmacokinetics, safety, and efficacy than the control compound 1.

**Table 4: Results of the experiment on liver microsome metabolic stability of monkeys, rats, and humans**

| | Monkey liver microsome | Rat liver microsome | Human liver microsome |
|---|---|---|---|
| | Half-life t_{1/2} (min) | Half-life t_{1/2} (min) | Half-life t_{1/2} (min) |
| Control compound 1 | 45.6 | 122 | 61.4 |
| Example 1 | > 145 | > 145 | > 145 |
| Example 54 | > 145 | > 145 | > 145 |
| Example 55 | > 145 | > 145 | > 145 |

### Pharmacokinetics of the compound of the present invention in rats:

Solvents for test compounds: DMSO:Solutol HS 15:Tween 80:Saline = 5:10:0.2:84.8 (v/v/v/v). Preparation method: Weigh an appropriate amount of compound, and add DMSO, Solutol HS 15, Tween 80, and physiological saline sequentially, to obtain a drug in a dosage form in a final concentration of 0.5 mg/mL for administration via gavage.

Male SD rats (from Vital River Laboratory Animal Technology Co., Ltd.), three rats (180-250 g) per group, fasted for at least 12 hours (free to drink water), and then took the drug via gavage at a volume of 10 mL/kg. Blood was collected from jugular sinus by about 0.2 mL separately before administration and at 0.25 h, 0.5 h, 1 h, 2 h, 4 h, 8 h, 10 h, and 24 h after administration. The 0.2 mL collected whole blood was placed in a pre-cooled EDTA-K2 anticoagulant tube and centrifuged (1500-1600 g) at 4°C for 10 min. The plasma was separated and then stored at -90°C to -60°C for biological sample analysis.

Pharmacokinetics parameters of rats are shown in Table 5. Compared with the control compound 1, the compound of the present invention exhibits a higher peak plasma concentration and a higher plasma exposure amount, indicating that the compound provided in the present invention offers better pharmacokinetic performance, and has more excellent potential for pain treatment.

**Table 5: Pharmacokinetics parameters of rats**

| | Peak time tₘₐₓ (h) | Peak plasma concentration Cₘₐₓ (ng/mL) | Plasma exposure amount AUC (ng*h/mL) | Half-life t_{1/2} (h) | Residence time MRT_{inf} (h) |
|---|---|---|---|---|---|
| Control compound 1 | 0.5 | 111 | 431 | 3.4 | 4.82 |
| Example 1 | 1.0 | 136 | 1080 | 3.37 | 5.78 |
| Example 54 | 3.33 | 213 | 1986 | 5.16 | 7.66 |
| Example 55 . | 4.00 | 114 | 1017 | 4.91 | 7.37 |

## Claims

1. A compound shown in formula I 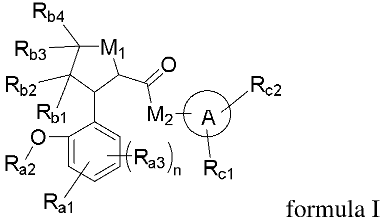
or a pharmaceutically acceptable salt or a stereoisomer thereof, wherein
Rₐ₁ and Rₐ₃ are each independently selected from hydrogen, halogen, hydroxyl, C₁-C₅ alkyl, C₁-C₅ alkoxy, C₃-C₆ cycloalkyl, C₃-C₆ cycloalkoxy, or -N(R_{c3}R_{c4})ₘ, or any adjacent two of Rₐ₁ and Rₐ₃ are linked together to form a 5- to 7-membered ring, the 5- to 7-membered ring is a saturated or unsaturated carbocyclic or heterocyclic ring; the heterocyclic ring optionally contains one or more N, O, or S(=O)ₘ heteroatoms;
Rₐ₂ is selected from C₁-C₆ alkyl substituted with one or more deuterium or fluorine;
R_{b1}, R_{b2}, R_{b3}, and R_{b4} are each independently selected from hydrogen, halogen, hydroxyl, C₁-C₅ alkyl, or C₁-C₅ alkoxy; or any two of R_{b1}, R_{b2}, R_{b3}, and R_{b4} are linked together to form a 3- to 7-membered ring;
M₁ and M₂ are each independently selected from C, O, S(=O)ₘ, or N-Rₙ, where Rₐ is optionally selected from hydrogen or C₁-C₅ alkyl;
a ring A is A1 or A2: 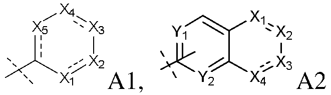
- - - optionally represents a single bond or a double bond;
X₁, X₂, X₃, X₄, and X₅ are each independently selected from a bond, C, N, O, C=O, or S(=O)ₘ;
Y₁ and Y₂ are the same or different, and are each optionally selected from a C atom or a N atom;
R_{c1} and R_{c2} are each independently selected from hydrogen, halogen, cyano, hydroxyl, amino, C₁-C₅ alkyl, C₁-C₅ alkoxy, -S(=O)ₘ-R_{c3}R_{c4}, -C(=O)-NR_{c3}R_{c4}, -C(=S)-NR_{c3}R_{c4}, - N(R_{c3}R_{c4})ₘ, -P(=O)ₘ-R_{c3}R_{c4}, -C(=N)-NR_{c3}R_{c4}, -S(=O)₂NR_{c3}R_{c4}, -C₁-C₅ alkyl NR_{c3}R_{c4}, or - S(=O)(=NR_{c3})R_{c4};
R_{c3} and R_{c4} are each independently selected from hydrogen, hydroxyl, amino, C₁-C₅ alkyl, C₁-C₅ alkoxy, or a 3- to 7-membered ring, or R_{c3} and R_{c4} are linked together to form a 3- to 7-membered ring;
m and n are each optionally 0, 1, or 2; and
the alkyl, alkoxy, cycloalkyl, cycloalkoxy, 5- to 7-membered ring, or 3- to 7-membered ring is optionally substituted with one or more deuterium, halogen, hydroxyl, cyano, oxo, C₁-C₅ alkyl, C₁-C₅ alkoxy, or amino.

2. The compound shown in formula I according to claim 1, wherein Rₐ₁ and Rₐ₃ are each independently selected from fluorine, chlorine, methyl, ethyl, methoxy, ethoxy, propoxy, trifluoromethoxy, difluoromethoxy, or amino.

3. The compound shown in formula I according to claim 1, wherein Rₐ₂ is optionally selected from methyl substituted with one, two, or three deuterium or fluorine.

4. The compound shown in formula I according to claim 1, wherein any adjacent two of Rₐ₁ and Rₐ₃ are linked together to form a 5- or 6-membered carbocyclic or heterocyclic ring.

5. The compound shown in formula I according to claim 1, wherein R_{b1}, R_{b2}, R_{b3}, and R_{b4} are each independently selected from hydrogen, methyl, trifluoromethyl, ethyl, methoxy, or ethoxy.

6. The compound shown in formula I according to claim 1, wherein any two of R_{b1}, R_{b2}, R_{b3}, and R_{b4} are linked together to form a 3-membered ring, a 4-membered ring, or a 5-membered ring.

7. The compound shown in formula I according to claim 1, wherein the ring A has the following structures:

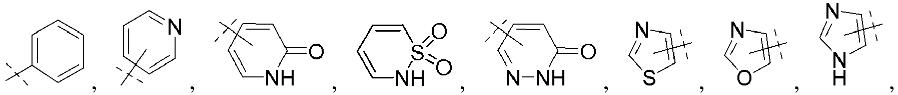

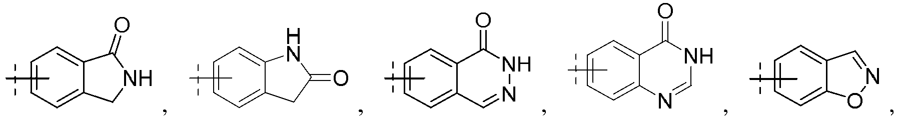

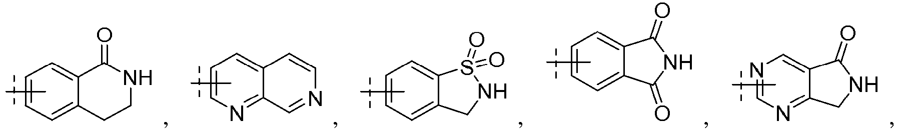

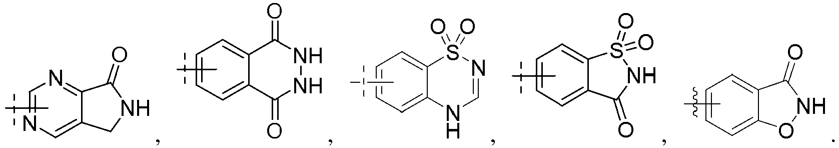

8. The compound shown in formula I according to claim 1, wherein the formula I has a structure shown in formula I-A or formula I-B:

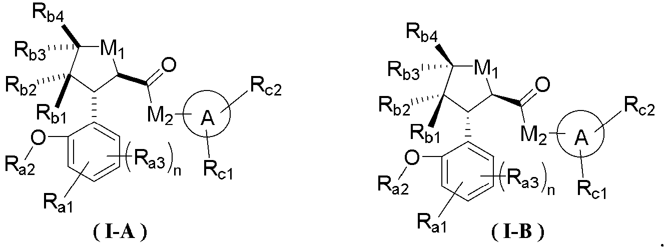

9. The compound shown in formula I according to claim 1 or 8, having the following structures:

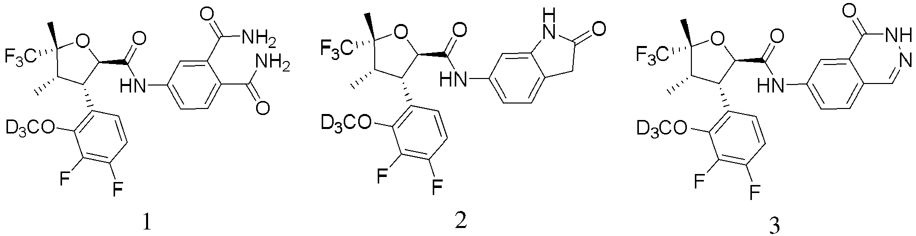

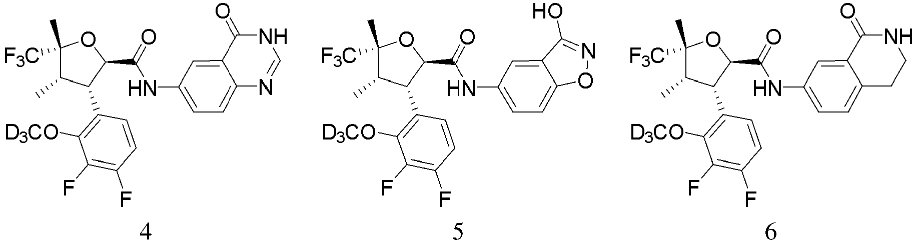

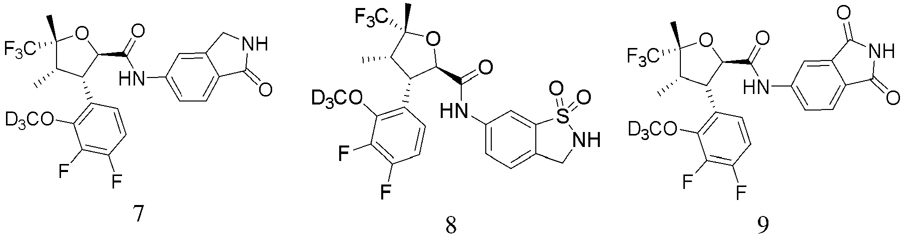

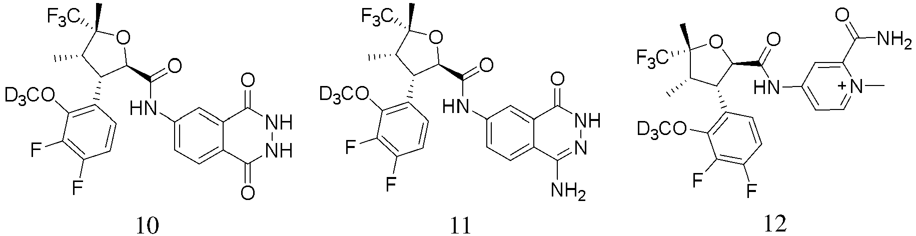

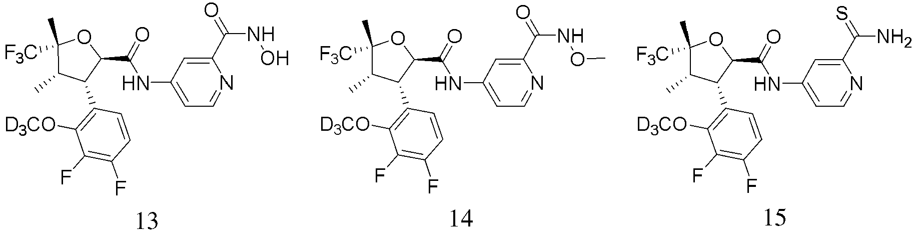

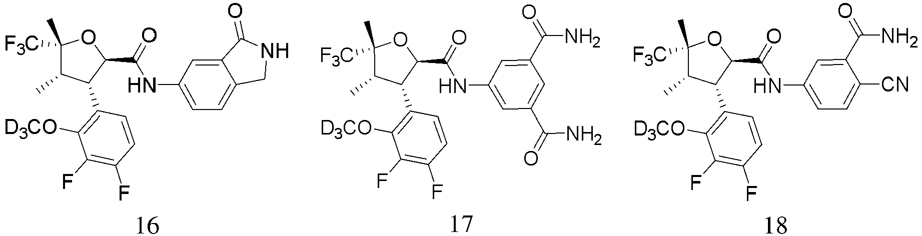

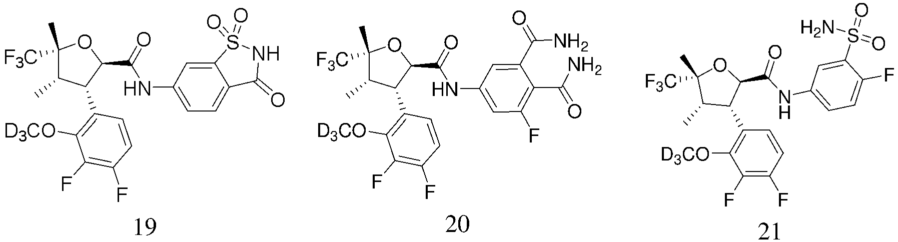

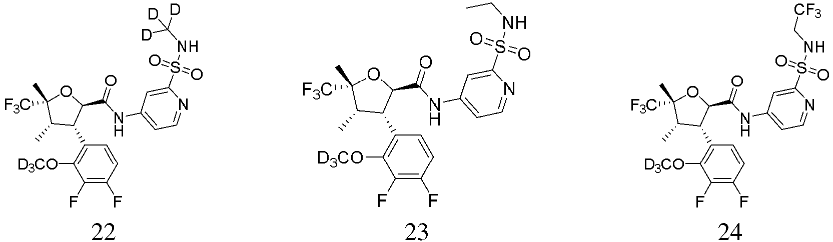

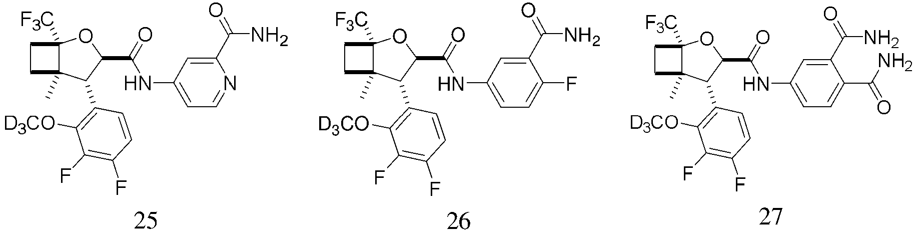

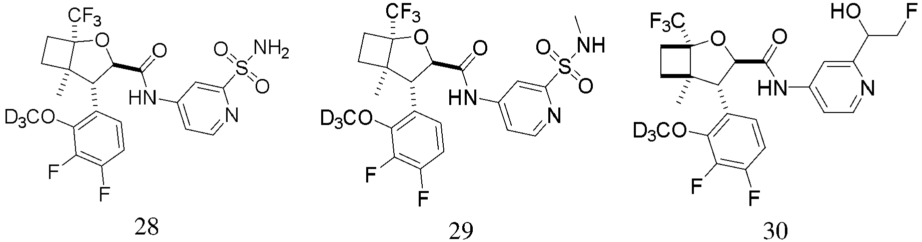

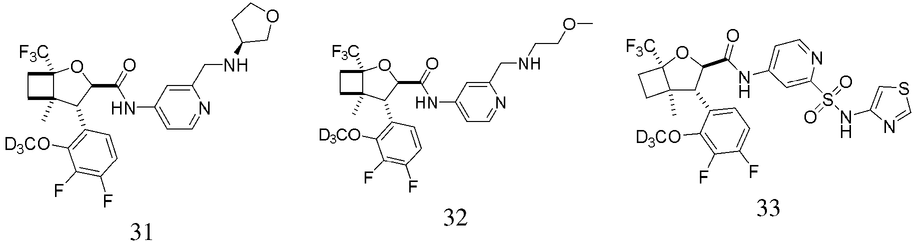

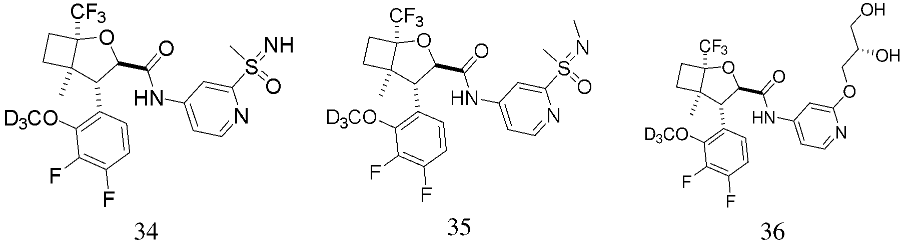

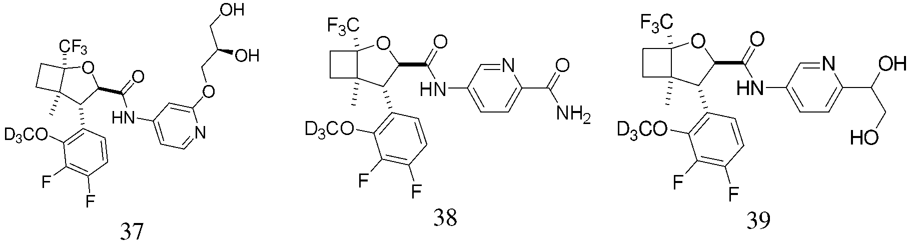

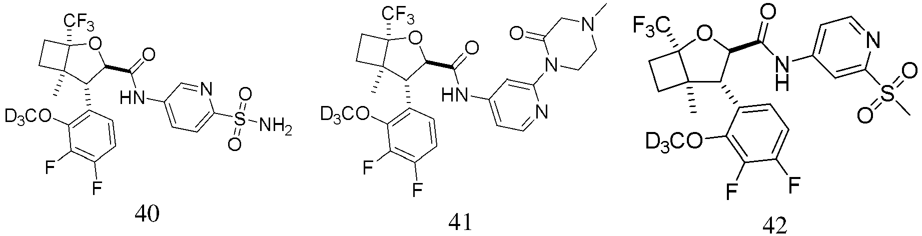

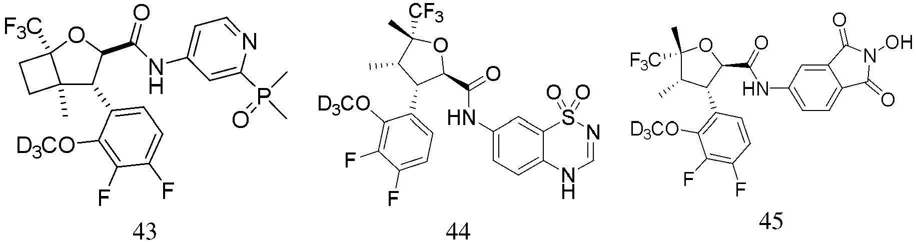

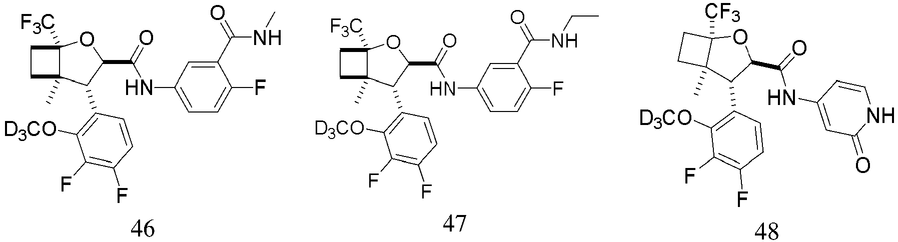

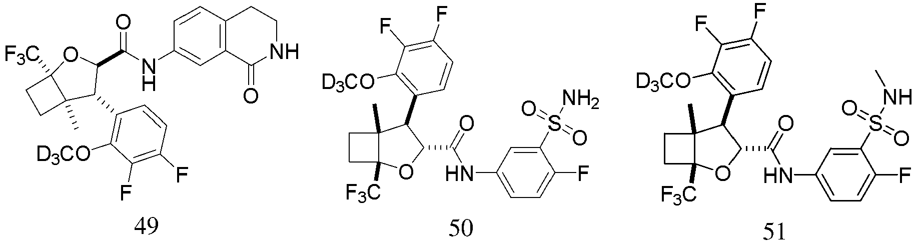

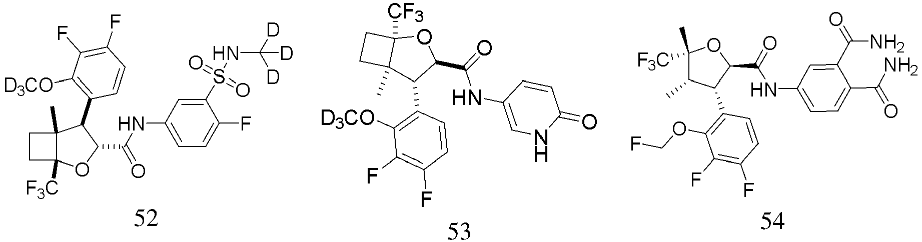

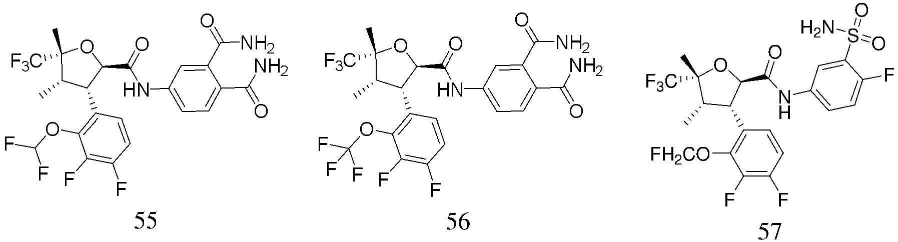

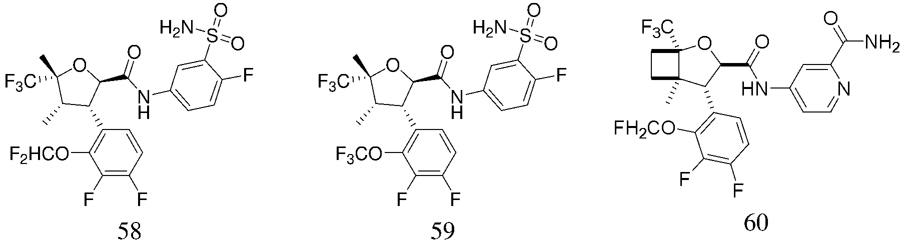

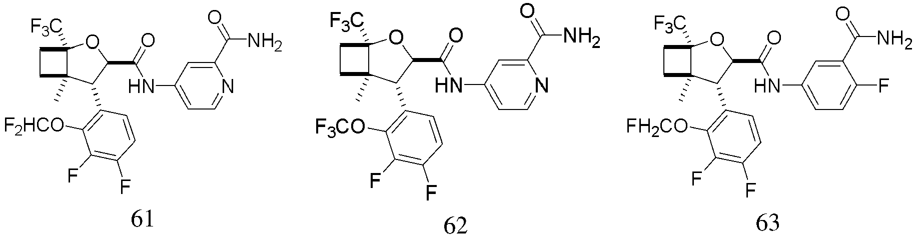

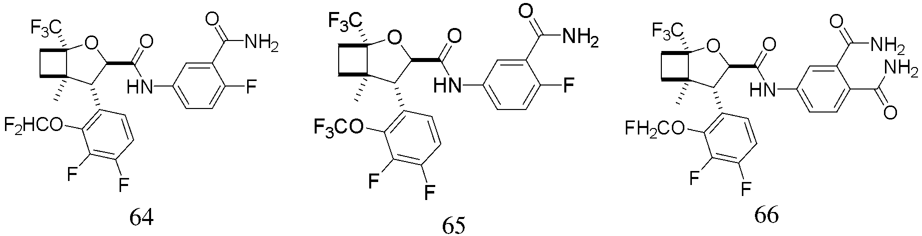

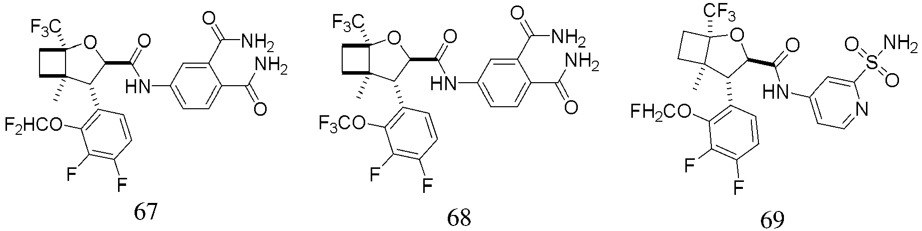

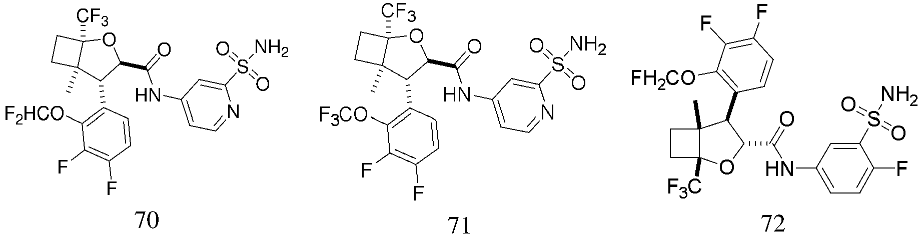

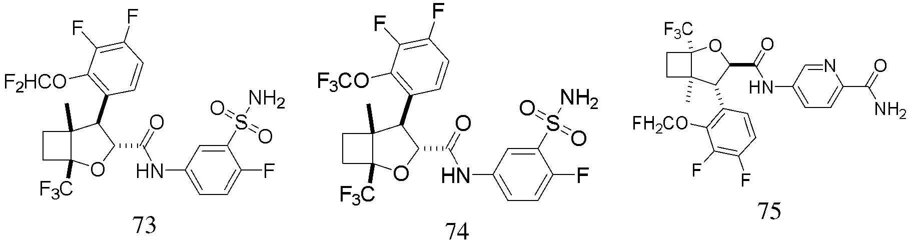

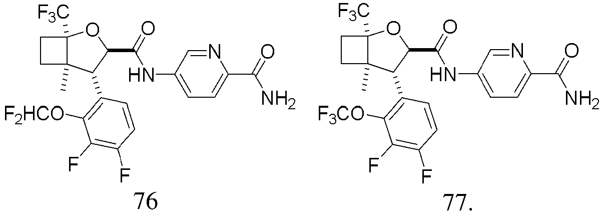

10. A pharmaceutical composition, wherein the composition contains the compound shown in formula I or a pharmaceutically acceptable salt or a stereoisomer thereof, and a pharmaceutically acceptable excipient.

11. Use of the pharmaceutical composition according to claim 10 or the compound shown in formula I according to claim 1 or a pharmaceutically acceptable salt or a stereoisomer thereof in preparation of a drug for treatment, prevention, or relief of a disease related to voltage-gated sodium channels.

12. The use according to claim 11, wherein the disease is pain, multiple sclerosis, Charcot-Marie-Tooth disease, incontinence, pathologic cough, or arrhythmia.
